# EUROPEAN PATENT SPECIFICATION

(11) **EP 1 185 493 B1**
(45) Date of publication and mention of the grant of the patent: **20.07.2005**
(21) Application number: 00928562.8
(22) Date of filing: 27.04.2000
(51) Int. Cl.: C07C 50/10

(54) **QUINONES FOR TREATMENT OF DISEASES**
CHINONVERBINDUNGEN ZUR BEHANDLUNG VON KRANKHEITEN
NOVEL QUINONES UTILISEES DANS LE TRAITEMENTS DE MALADIES

(30) Priority: 30.04.1999 US 131842 P
(43) Date of publication of application: 13.03.2002
(73) Proprietor: SLIL Biomedical Corporation, Madison, Wisconsin 53711-6233 (US)
(72) Inventor: BLOKHIN, Andrei, V., Madison, WI 53719 (US); FRYDMAN, Benjamin, Madison, WI 53719 (US); MARTON, Laurence, J., Madison, WI 53711 (US); NEDER, Karen, M., Madison, WI 53703 (US); SUN, Jerry, Shunneng, Madison, WI 53705 (US)
(74) Representative: Roques, Sarah Elizabeth
(86) International application number: PCT/US2000/011538
(87) International publication number: WO 2000/066528

(56) References cited:
- EP-A- 0 255 679
- WO-A-97/08162
- WO-A-97/31611
- WO-A-97/31936
- US-A- 4 273 706
- US-A- 5 763 625
- TAKUWA, AKIO ET AL: "Structural influences on the isomerization of 4-benzyl- and 4-allyl-1,2-naphthoquinones to quinone methides and their stereochemistry" J. CHEM. SOC., PERKIN TRANS. 1 (1986), (9), 1627-31, XP002142390
- DATABASE CHEMABS [Online] CHEMICAL ABSTRACTS SERVICE, COLUMBUS, OHIO, US; PERSHIN, G. N. ET AL: "Bonaphthon, a new antiviral chemotherapeutic agent" retrieved from STN Database accession no. 82:118979 XP002142391 & FARMAKOL. TOKSIKOL. (MOSCOW) (1975), 38(1), 69-73,
- NELSON, WENDEL L. ET AL: "3,4-Catechol derivative of propranolol, a minor dihydroxylated metabolite" J. MED. CHEM. (1984), 27(7), 857-61, XP002142392
- BULLOCK, F. J. ET AL: "Antiprotozoal quinones. II. Synthesis of 4-amino-1,2- naphthoquinones and related compounds as potential antimalarials" J. MED. CHEM. (1970), 13(1), 97-103, XP002142393
- DUNN, W. J., III ET AL: "Structure-activity analyzed by pattern recognition: the asymmetric case" J. MED. CHEM. (1980), 23(6), 595-9, XP002142394
- TAKUWA, AKIO ET AL: "Addition of alcohol to 1,2-naphthoquinone promoted by metal ions. Facile synthesis of 4-alkoxy-1,2-naphthoquinones" BULL. CHEM. SOC. JPN. (1986), 59(9), 2959-61, XP002142395
- DATABASE CHEMABS [Online] CHEMICAL ABSTRACTS SERVICE, COLUMBUS, OHIO, US; DOLAN, M. EILEEN ET AL: "Effects of 1,2-naphthoquinones on human tumor cell growth and lack of cross-resistance with other anticancer agents" retrieved from STN Database accession no. 129:156459 XP002142396 & ANTI-CANCER DRUGS (1998), 9(5), 437-448,
- COTE, PHILIP N. ET AL: "Glucopyranosides derived from 2-hydroxy-1,4-naphthoquinones" CARBOHYD. RES., vol. 26, no. 1, 1973, pages 247-251, XP000925262
- PLANCHON, SARAH M. ET AL: "Bcl-2 protects against.beta.-lapachone-mediated caspase 3 activation and apoptosis in human myeloid leukemia (HL-60) cells" ONCOL. REP., vol. 6, no. 3, 1999, pages 485-492, XP000957471

## Description

### TECHNICAL FIELD

This invention relates to novel quinones and also relates to various medicinal and industrial uses of these compounds.

### BACKGROUND OF THE INVENTION

The quinones are a large and varied group of natural products found in all major groups of organisms. Quinones are a group of aromatic dioxo compounds derived from benzene or multiple-ring hydrocarbons such as naphthalene, anthracene, etc. They are classified as benzoquinones, naphthoquinones, anthraquinones, etc., on the basis of the ring system. The C=O groups are generally *ortho* or *para,* and form a conjugated system with at least two C=C double bonds; hence the compounds are colored, yellow, orange or red. Quinones with long isoprenoid side chains, such as plastoquinone, ubiquinone and phytoquinone are involved in the basic life processes of photosynthesis and respiration. Quinones are biosynthesized from acetate/malonate via shikimic acid. A few quinones are used as laxatives and worming agents, and others are used a pigments in cosmetics, histology and aquarrell paints. Quinones have a variety of medicinal and industrial uses.

Many efficient antineoplastic drugs are either quinones (anthracycline derivatives, mitoxantrone, actinomycin), quinonoid derivatives (quinolones, genistein, bactracyclin), or drugs such as etoposide that can easily be converted to quinones by *in vivo* oxidation. Gantchev et al. (1997) *Biochem. Biophys. Res. Comm.* 237:24-27. The literature on quinone-DNA interactions is replete with references to quinones having the potential to undergo redox cycling with the formation of highly reactive oxygen species that are thought to relate to their cytotoxicity. O'Brien (1991) *Chem. Biol. Interactions* 80:1-41. It has also been shown that many quinones are efficient modifiers of the enzymatic activity of topoisomerase II, an enzyme essential for cell division.

Quinones are now widely used as anti-cancer, anti-bacterial and anti-malarial drugs, as well as fungicides. The antitumor activities of the quinones were revealed more than two decades ago when the National Cancer Institute published a report in which fifteen-hundred synthetic and natural quinones were screened for their anticancer activities. Driscoll et al. (1974) *Cancer Chemot. Reports* 4:1-362. Anti-cancer quinones include β-Lapachone, a plant product, which inhibits DNA topoisomerase II and induces cell death with characteristics of apoptosis in human prostate and promyelocytic leukemia cancer cell lines. Human breast and ovary carcinoma showed sensitivity of the cytotoxic effect of β-lapachone without signs of apoptosis. Li et al. (1995) *Cancer Res.* 55:3712-5; and Planchon et al. (1995) *Cancer Res.* 55:3706-11. 1,2-Naphthoquinone (3,4-b)dihydrofuran inhibits neoplastic cell growth and proliferation of several cancers, such as prostate, breast, colon, brain and lung, including multi-drug resistant types. WO 97/31936. Furano-naphthoquinone derivatives and other naphthoquinones and naphth-[2,3-d]-imidazole-4,9-dione compounds are also useful in treating malignant tumors such as those affecting the blood, breast, central nervous system, cervix, colon, kidney, lung, prostate and skin. WO 97/30022 and JP Patent No. 9235280. Anthraquinone derivatives with telomerase inhibitory activity are also useful in treating leukemia, lung cancer, myeloma, lymphoma, prostate, colon, head and neck, melanoma, hepatocellular carcinoma, bladder, ovarian, breast and gastric cancers. WO 98/25884 and WO 98/25885. Ansamycin benzoquinones are useful in the treatment of primitive neuroectodermal tumors, prostate cancer, melanoma and metastatic Ewing's sarcoma. WO 94/08578.

Quinones also have a number of other medicinal uses. Terpenoid-type quinones are also useful as treatments for diabetes. U.S. Patent No. 5,674,900. Additional quinones can be used to treat cirrhosis and other liver disorders. U.S. Patent Nos. 5,210,239 and 5,385,942. Hydroquinone amines and quinone amines are also useful for treating a number of conditions, including spinal trauma and head injury. U.S. Patent No. 5,120,843. Degenerative central nervous system diseases, as well as vascular diseases, are treatable with quinones such as Idebenone [2,3-dimethoxy-5-methyl-6-(10-hydroxydecyl)-1,4-benzoquinone] and Rifamycin S. Mordente et al. (1998) *Chem. Res. Toxicol.* 11:54-63; Rao et al. (1997) *Free Radic. Biol. Med*. 22:439-46; Cortelli et al. (1997) *J. Neurol. Sci.* 148:25-31; and Mahadik et al. (1996) *Prostaglandins Leukot. Essent*. *Fatty Acids* 55:45-54. A vitamin K analog, 6-cyclo-octylamino-5,8-quinoline quinone shows efficacy for treatment of leprosy and tuberculosis. U.S. Patent No. 4,963,565. Hydroquinone is used to treat skin pigmentation disorders. Clarys et al. (1998) *J. Dermatol.* 25:412-4. Mitomycin C-related drug indoloquinone EO9 has demonstrated cell killing against HL-60 human leukemia cells, H661 human lung cancer cells, rat Walker tumor cells and human HT29 colon carcinoma cells. Begleiter et al. (1997) *Oncol. Res.* 9:371-82: and Bailey et al. (1997) *Br. J. Cancer* 76:1596-603. Quinones such as aloin, a C-glycoside derivative of anthraquinone, accelerate ethanol oxidation and may be useful in treating acute alcohol intoxication. Chung et al. (1996) *Biochem. Pharmacol.* 52:1461-8 and Nanji et al. (1996) *Toxicol. Appl. Pharmacol*. 140:101-7. Quinones capsaicin and resiniferatoxin blocked activation of nuclear transcription factor NF-κB, which is required for viral replication, immune regulation and induction of various inflammatory and growth-regulatory genes. Singh et al. (1996) *J. Immunol.* 157:4412-20. Antiretroviral and antiprotozoan naphthoquinones are described in U.S. Patent Nos. 5,780,514 and 5,783,598. Anthraquinones are also useful as laxatives. Ashraf et al. (1994) *Aliment. Pharmacol. Ther.* 8:329-36; and Muller-Lissner (1993) *Pharmacol*. 47 (Suppl. 1): 138-45.

A subset of quinones designated lapachones has been shown to have activity against neoplastic cells, as described in U.S. Patent Nos. 5,969,163, 5,824,700, and 5,763,625. Antiviral activity (in combination with xanthine) or reverse transcriptase inhibitory activity for β-lapachone is suggested in U.S. Pat. Nos. 5,641,773 and 4,898,870, while antifungal and trypanosidal activity of β-lapachone is suggested in U.S. Pat. Nos. 5,985,331 and 5,912,241.

Quinones can be administered alone or in conjunction with other agents, such as 1,2-dithiole-3-thione. Begleiter et al. (1997). Hydroxyquinone can be used in conjunction with glycol or glyceryl esters of retinoic acid to treat skin disorders. WO 9702030. Combinational chemotherapy of carboquone, a benzoquinine derivative, and *cis*-Platinum, diminishes the side effects of the former. Saito (1988) *Gan To Kagaku Ryoho* 15:549-54.

Quinones also have various additional uses. A few quinones are used as laxatives and worming agents, and others are used a pigments in cosmetics, histology and aquarrell paints. Quinones include 2,5-cyclohexadiene-1,4-dione, which is useful as an oxidizing agent; in photography (U.S. Patent No. 5,080,998); in manufacturing dyes and hydroquinone; in tanning hides; in strengthening animal fibers; and as a reagent.

In rapidly dividing cells such as tumor cells, cytotoxicity due to quinone administration has been attributed to DNA modification. However the molecular basis for the initiation of quinone cytotoxicity in resting or non-dividing cells has been attributed to the alkylation of essential protein thiol or amine groups and/or the oxidation of essential protein thiols by activated oxygen species and/or GSSG, glutathione disulfide. Oxidative stress arises when the quinone is reduced by reductases to a semiquinone radical which reduces oxygen to superoxide radicals and reforms the quinone. This futile redox cycling and oxygen activation forms cytotoxic levels of hydrogen peroxide and GSSG is retained by the cell and causes cytotoxic mixed protein disulfide formation. O'Brien (1991) *Chem. Biol. Interact.* 80:1-41.

Conjugation of quinones and glutathione (GSH) are sometimes associated with the process of detoxification. Jeong et al. (1996) *Mol. Pharmacol*. 50:592-8. For example, certain o-quinones contribute to the neurodegenerative processes underlying Parkinson's disease and schizophrenia. Glutathione transferase (GST) M2-2, which conjugates glutathione and o-quinones, prevents these processes. Baez et al. (1997) *Biochem. J*. 324:25-8. However, in many cases, conjugation with GSH actually leads to quinone bioactivation and toxicity. For example, the nephrotoxicity of hydroquinone and bromobenzene is mediated via quinone-glutathione conjugates. Jeong et al. (1996) *Mol. Pharmacol.* 50:592-8. The formation af GSH conjugates is also involved in the bioactivation of vicinal dihalopropane 1,2-dibromo-3-chloropropane. Hinson et al. (1995) *Can. J. Physiol. Pharm*. 73:1407-13. Additional examples of GSH conjugation potentiating the toxicity of quinones are described in Fowler et al. (1991) *Hum. Exp. Toxicol.* 10:451-9; Mertens et al. (1991) *Toxicol. Appl. Pharmacol*. 110:45-60; Puckett-Vaughn et al. (1993) *Life Sci.* 52:1239-47; Dekant (1993) *Toxicol. Lett*. 67:151-160; Monks et al. (1994) *Chem. Res. Toxicol.* 7:495-502; Monks (1995) *Drug Metab. Rev.* 27:93-106; and Eyer (1994) *Environ. Health Persp.* 102 (Suppl. 6):123-32.

Because of the wide variety of biological processes in which quinones play a critical role, it would be advantageous to develop novel quinones for various uses, including disease treatment.

### SUMMARY OF THE INVENTION

The invention provides novel quinone compounds and methods for use of the quinone compounds in treating diseases.

The invention comprises compounds of the formula 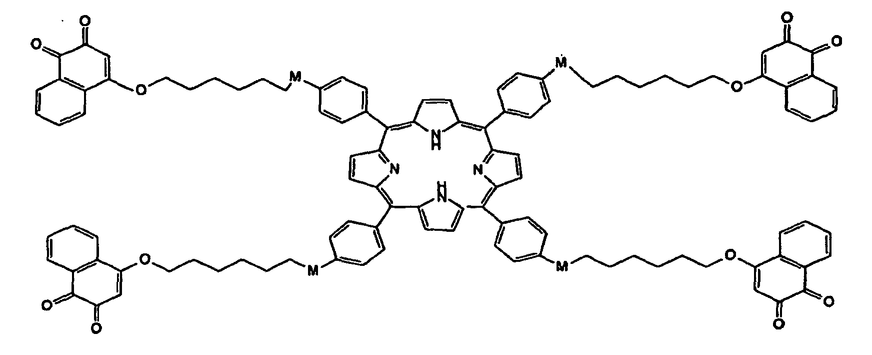 wherein M is selected from the group consisting of -O-, -C(=O)-O-, -O-(C=O)- - C(=O)-N-, and -N-(C=O)-. The invention also comprises the above compounds in combination with a pharmaceutically acceptable carrier. The invention also comprises use of the above compounds to treat an indication characterised by the proliferation of disease cells in an individual, comprising administering to the individual a therapeutic amount of one or more of the above compounds, optionally together with another therapeutically effective compound or compounds.

The invention also includes all salts, stereoisomers and tautomers of the foregoing compounds, unless explicitly indicated otherwise.

In another embodiment, the invention comprises any one or more of the foregoing compounds, optionally in combination with another therapeutic compound, combined with a pharmaceutically acceptable excipient or carrier.

The invention also provides methods of treating an indication comprising the step of administering to the individual an effective amount of a composition comprising a novel quinone. In one embodiment, the invention comprises a method of treating an indication characterised by the proliferation of disease cells in an individual comprising administering to the individual a therapeutic amount of any of the foregoing compounds. In one method, the indication is cancer. In various embodiments the cancer affects cells of the bladder, blood, brain, breast, colon, digestive tract, lung, ovaries, pancreas, prostate gland, or skin. In other embodiments, the indication can also include, but is not limited to, Alzheimer's disease, epilepsy, multiple sclerosis, problems associated with tissue grafts and organ transplants, psoriasis, restenosis, stomach ulcers, or tissue overgrowth after surgery. In other embodiments, the indication is an infection or infestation of parasites, bacteria, fungi or insects.

### BRIEF DESCRIPTION OF THE DRAWINGS

Figure 1 depicts Scheme 1, illustrating the synthetic preparation of certain compounds.

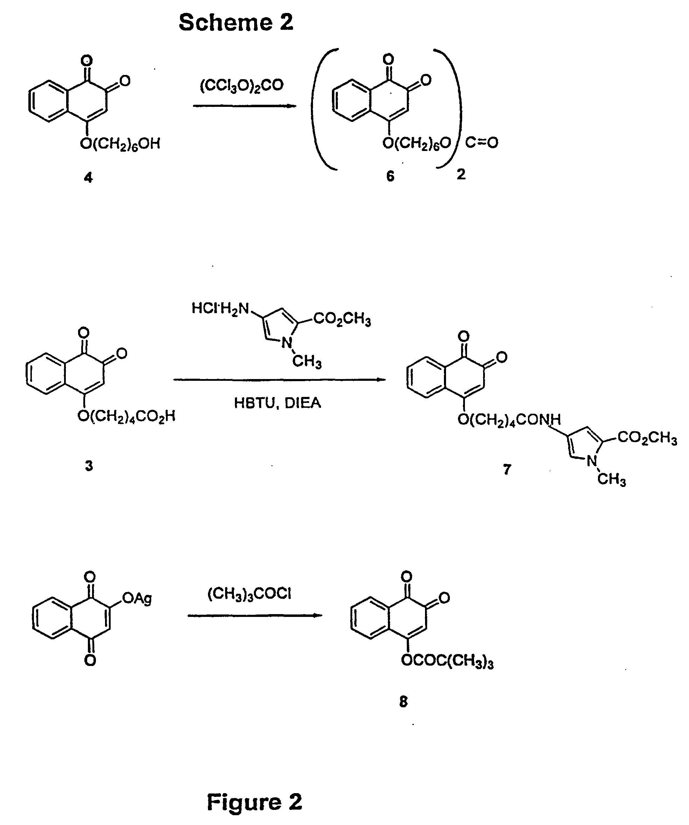

Figure 2 depicts Scheme 2, illustrating the synthetic preparation of additional compounds.

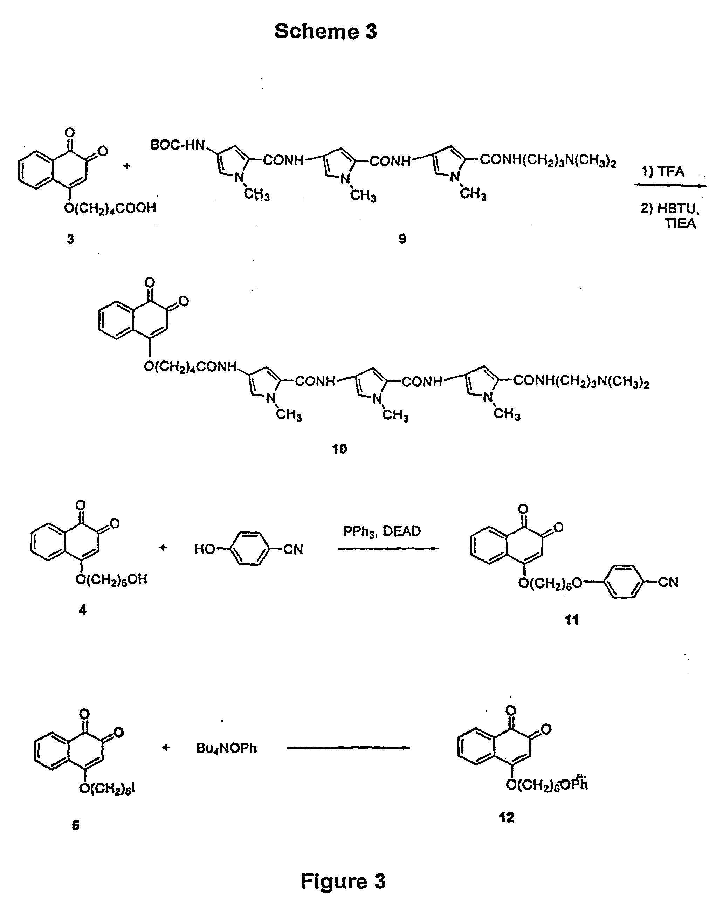

Figure 3 depicts Scheme 3, illustrating the synthetic preparation of additional compounds.

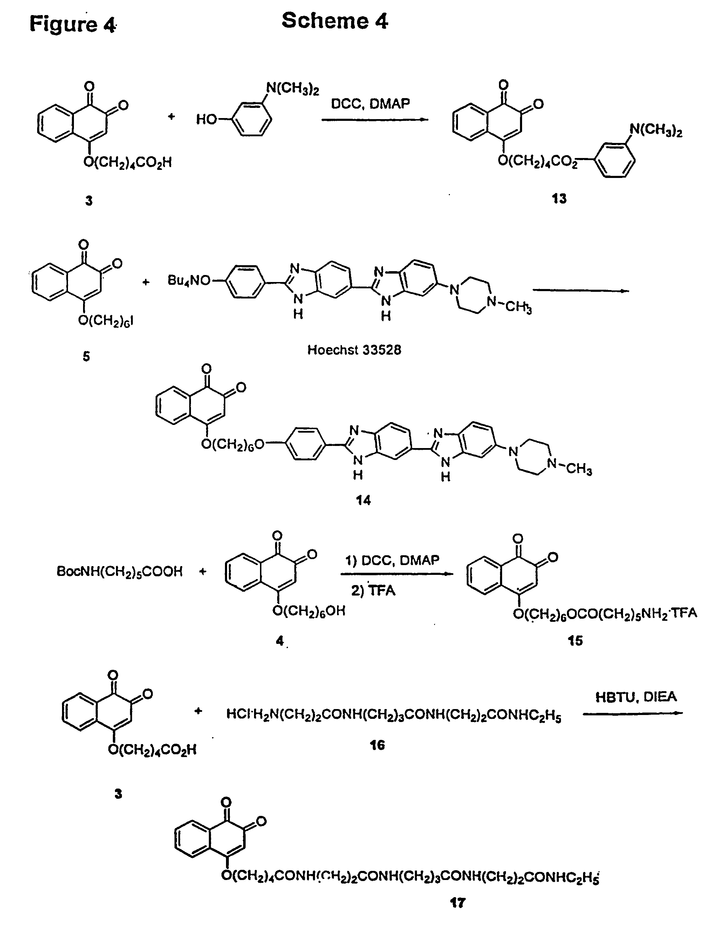

Figure 4 depicts Scheme 4, illustrating the synthetic preparation of additional compounds.

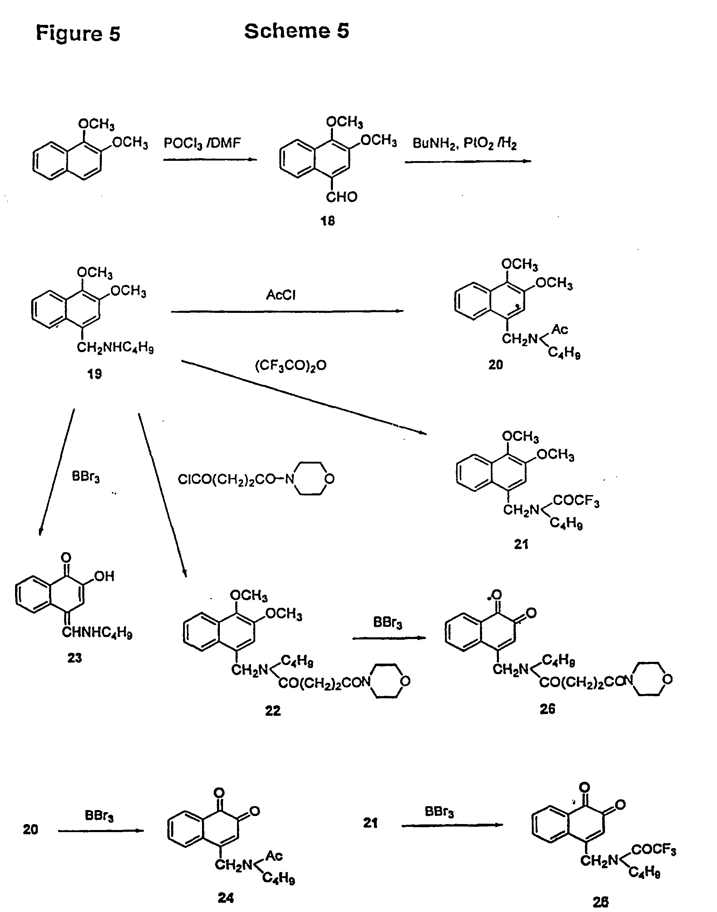

Figure 5 depicts Scheme 5, illustrating the synthetic preparation of additional compounds.

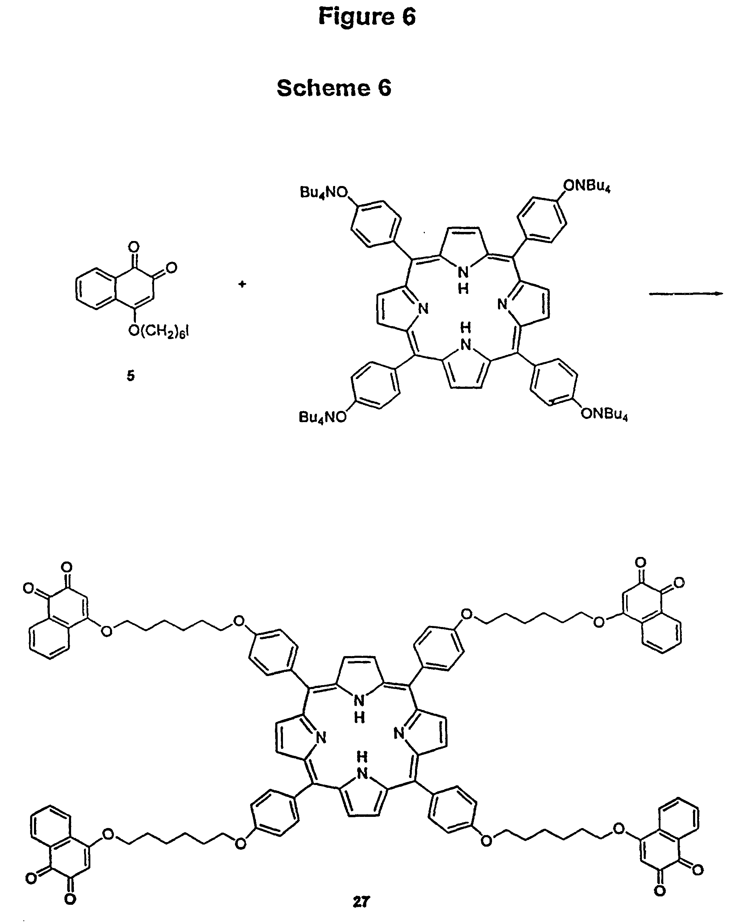

Figure 6 depicts Scheme 6, illustrating the synthetic preparation of additional compounds.

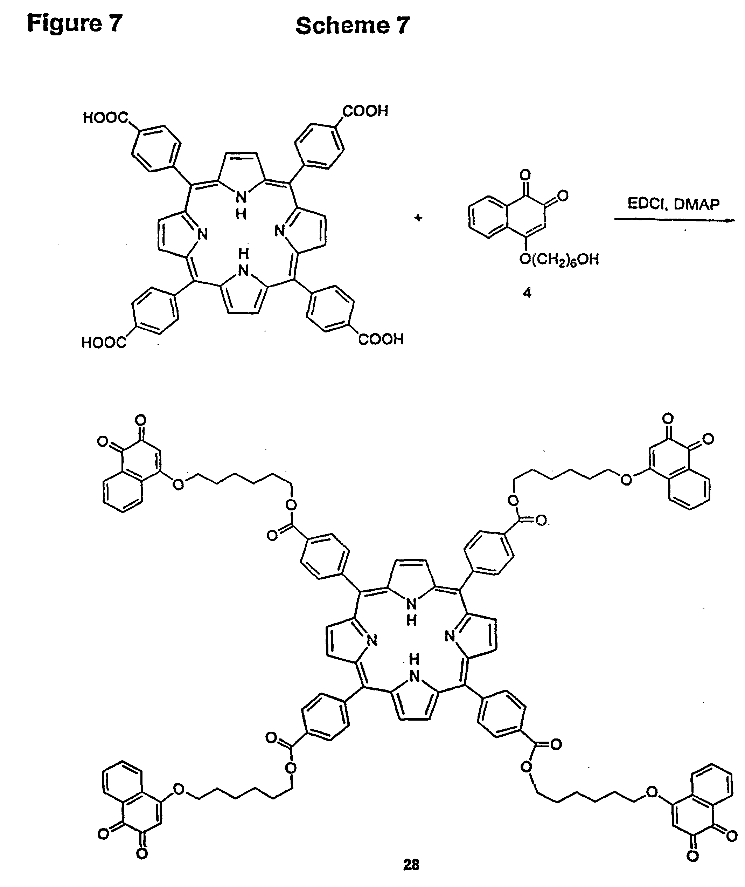

Figure 7 depicts Scheme 7, illustrating the synthetic preparation of compounds of the invention.

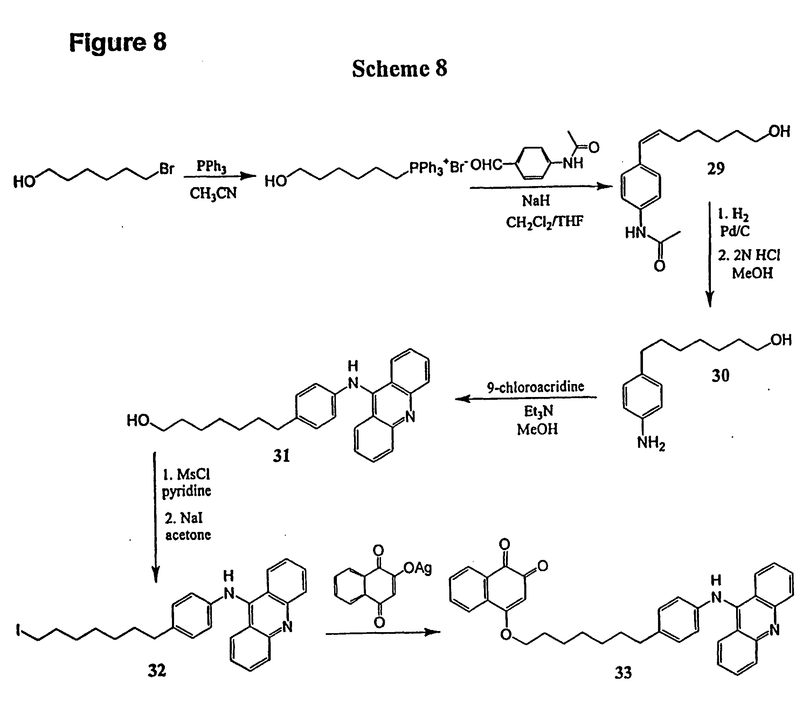

Figure 8 depicts Scheme 8, illustrating the synthetic preparation of additional compounds.

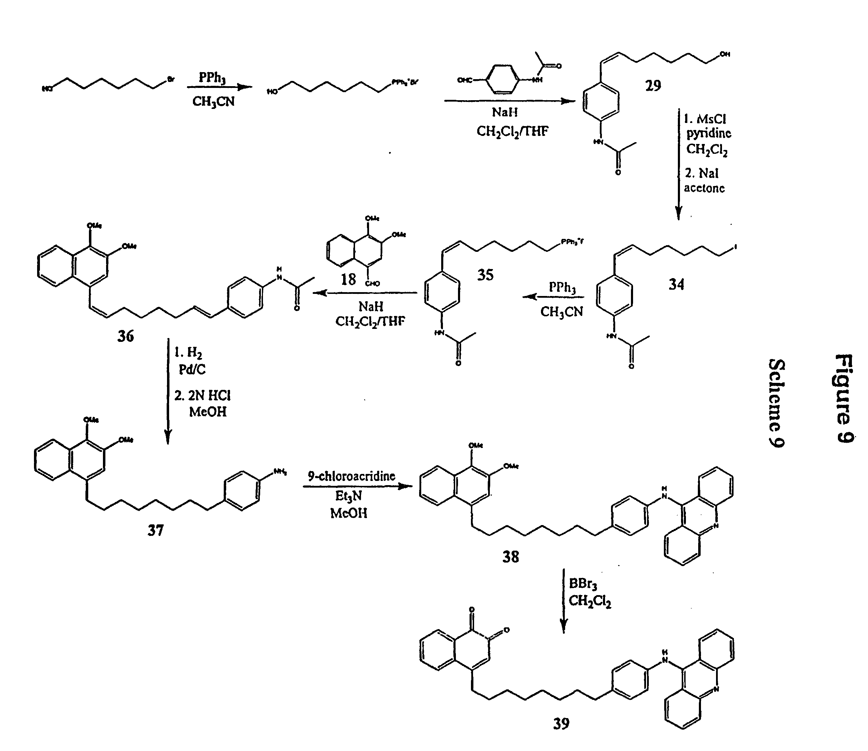

Figure 9 depicts Scheme 9, illustrating the synthetic preparation of additional compounds.

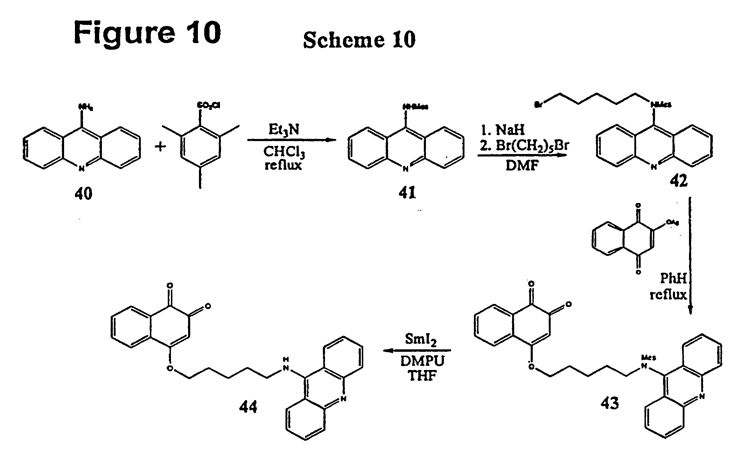

Figure 10 depicts Scheme 10, illustrating the synthetic preparation of additional compounds.

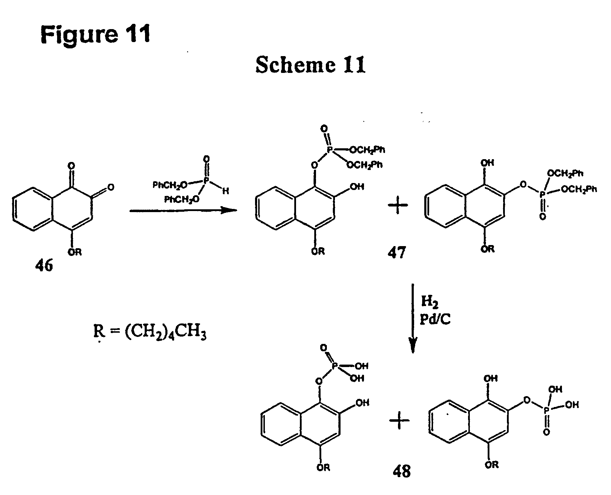

Figure 11 depicts Scheme 11, illustrating the synthetic preparation of additional compounds.

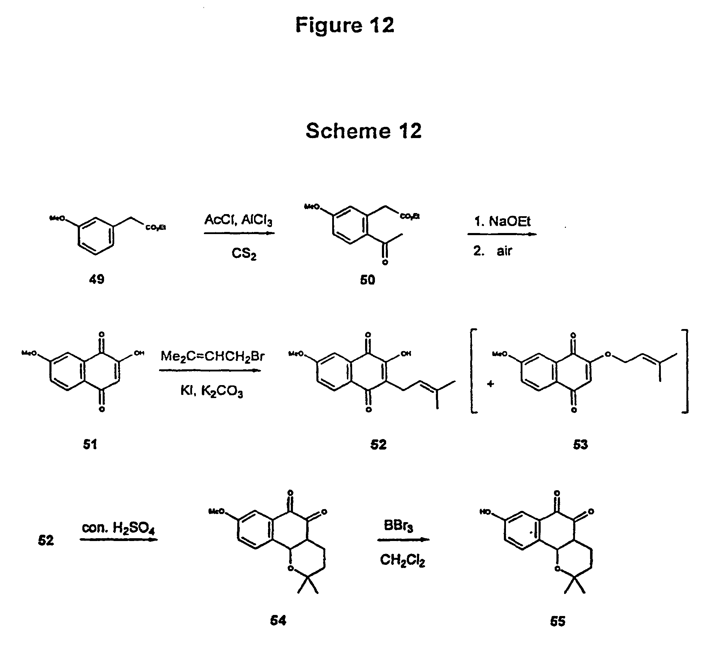

Figure 12 depicts Scheme 12, illustrating the synthetic preparation of additional compounds.

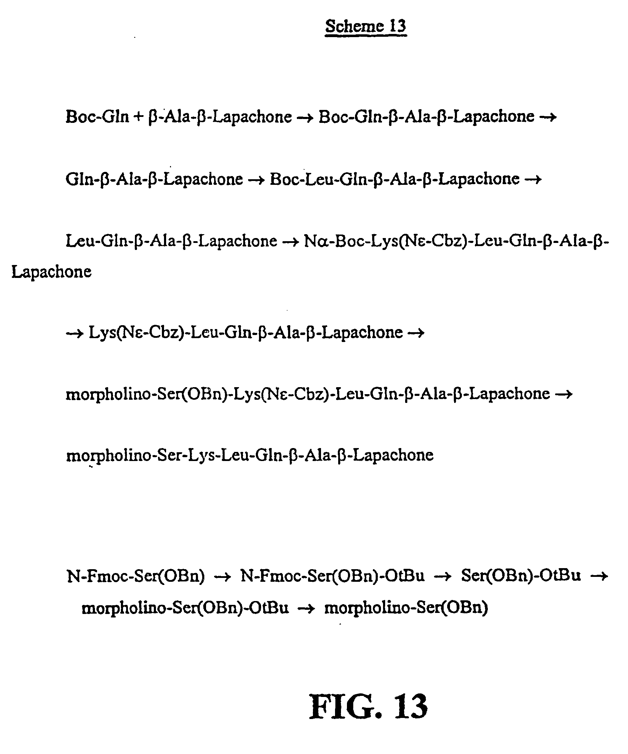

Figure 13 depicts Scheme 13, illustrating synthetic preparation of peptides conjugated to certain quinone compounds.

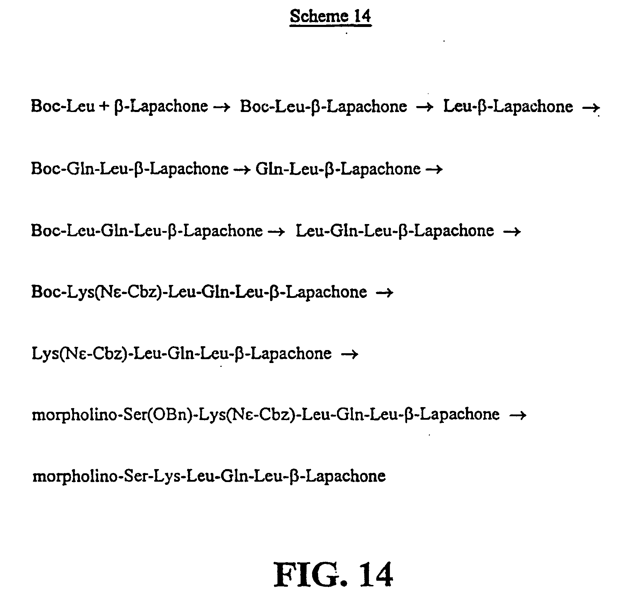

Figure 14 depicts Scheme 14, illustrating additional synthetic preparation of peptides conjugated to certain quinone compounds.
Figure 15 depicts additional synthetic preparation of peptides conjugated to certain quinone compounds, including attachment of a linker group between the quinone and the peptide.
Figure 16 depicts the attachment of doxorubicin to a peptide, including attachment of a linker group between doxorubicin and the peptide.
Figure 17 depicts additional synthetic preparation of peptides conjugated to certain quinone compounds, including attachment of a linker group between the quinone and the peptide.

### MODES FOR CARRYING OUT THE INVENTION

The present invention encompasses novel quinones and methods of their use. Such methods include treating indications in an individual comprising the step of administering to the individual an effective amount of a novel quinone. The indications include cancer. In various embodiments, the cancer affects cells of the bladder, blood, brain, breast, colon, digestive tract, lung, ovaries, pancreas, prostate gland, or skin. In other embodiments, the indication can also include, but is not limited to, Alzheimer's disease, epilepsy, multiple sclerosis, problems associated with tissue grafts and organ transplants, psoriasis, restenosis, stomach ulcers, or tissue overgrowth after surgery. In other embodiments, the indication is an infection or infestation of parasites, bacteria, fungi or insects.

### Definitions

By a "quinone" is meant any of a group of aromatic dioxo compounds derived from benzene or multiple-ring hydrocarbons such as naphthalene, anthracene, etc. They are classified as benzoquinones, naphthoquinones, anthraquinones, etc., on the basis of the ring system. The C=O groups are generally *ortho* or *para,* and form a conjugated system with at least two C=C double bonds; hence the compounds are colored, yellow, orange or red. This type of chromophore is found in many natural and synthetic pigments. Exemplary quinones include 2,5-cyclohexadiene-1,4-dione, which is useful as an oxidizing agent, in photography, in manufacturing dyes and hydroquinone, in tanning hides, in strengthening animal fibers, and as a reagent; and various 1,2-naphthoquinones, which have medicinal uses. Frydman et al. (1997) *Cancer Res.* 57:620-627. By "hydroquinone" is meant the reduced form of any quinone; for example, the reduced form of 1,4-benzoquinone is 1,4-dihydroxybenzene (p-dihydroxybenzene).

An "indication" includes any symptom or the like which points out a suitable remedy or treatment or which shows the presence of a disease. As used herein, an "indication" also includes a "disease" itself, where a disease is a condition of an organ, part, structure or system of the body in which there is incorrect function resulting from the effect(s) of heredity, infection, diet and/or environment. The indication can be characterized by proliferation of diseased cells, such as cancer. By "cancer" is meant the abnormal presence of cells which exhibit relatively autonomous growth, so that they exhibit an aberrant growth phenotype characterized by a significant loss of cell proliferation control. Cancerous cells can be benign or malignant. In various embodiments, the cancer affects cells of the bladder, blood, brain, breast, colon, digestive tract, lung, ovaries, pancreas, prostate gland, or skin. In other embodiments, the indication can also include, but is not limited to, Alzheimer's disease, epilepsy, multiple sclerosis, problems associated with tissue grafts and organ transplants, psoriasis, restenosis, stomach ulcers, or tissue overgrowth after surgery. In other embodiments, the indication is an infection or infestation of parasites, bacteria, fungi or insects.

By "DNA toposiomerase II" is meant is the scaffold protein capable of cleaving double-stranded DNA, passing an uncut portion of the DNA between the cut ends, and resealing the cut. DNA topoisomerase II ("topo II") is critical in DNA replication, because it can unknot tangles of DNA that would otherwise form as the long parental strands unwind and daughter strands are synthesized. During cleavage by topo II, the free 5' phosphates on the DNA strands become covalently linked to tyrosine side chains of the enzyme. Staining of metaphase chromosomes with fluorescent antibodies raised against highly purified topo II demonstrates that this enzyme is associated with the chromosome scaffold. Even in interphase chromosomes, which are not as condensed as metaphase chromosomes, the DNA remains associated with topo II and hence with the chromosome scaffold. During interphase, proteins, including topo II, are bound to fixed sites in mammalian DNA that are 30-90 kb apart. The binding sites for topo II are called scaffold-associated regions (SARs), which occur between but not within transcription units. DNA topoisomerase II is reviewed and discussed in, for example, Austin et al. (1998) *Bioessays* 20:215-26; Larsen et al. (1996) *Prog. Cell Cycle Res.* 2:229-39; Chaly et al. (1996) *Chromosome Res.* 4:457-66; Kimura et al. (1994) *J. Biol. Chem.* 269:1173-6; and Roca et al. (1993) *J. Biol. Chem.* 268:14250-5.

An "individual" is a vertebrate, preferably a mammal, more preferably a human. Mammals include, but are not limited to, farm animals, sport animals, rodents, primates, and pets.

An "effective amount" or "therapeutic amount" is an amount sufficient to effect beneficial or desired clinical results. An effective amount can be administered in one or more administrations. For purposes of this invention, an effective amount of a quinone is an amount that is sufficient to palliate, ameliorate, stabilize, reverse, slow or delay the progression of the disease state. A therapeutic amount of a quinone of the present invention is an amount sufficient to inhibit proliferation of diseased cells. A quinone is considered to be an effective agent if it is effective against at least one disease or in at least one application, even if it is not effective against another disease or in another application.

As used herein, "treatment" is an approach for obtaining beneficial or desired clinical results. For purposes of this invention, beneficial or desired clinical results include, but are not limited to, alleviation of symptoms, diminishment of extent of disease, stabilization (i.e., not worsening) of state of disease, prevention of spread (i.e., metastasis) of disease, delay or slowing of disease progression, amelioration or palliation of the disease state, improvement in quality of enjoyment of life, and remission (whether partial or total), whether detectable or undetectable. "Treatment" can also mean prolonging survival as compared to expected survival if not receiving treatment.

"Palliating" a disease means that the extent and/or undesirable clinical manifestations of a disease state are lessened and/or time course of the progression is slowed or lengthened, as compared to not administering quinones of the present invention.

The invention includes all salts of the compounds described herein. Particularly preferred are pharmaceutically acceptable salts. Pharmaceutically acceptable salts are those salts which retain the biological activity of the free acids or bases and which are not biologically or otherwise undesirable. The desired salt may be prepared by methods known to those of skill in the art by treating an amine-containing quinone with an acid, or by treating an acid-containing quinone with a base. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Examples of bases include, but are not limited to, sodium hydroxide and potassium hydroxide (which yield sodium and potassium salts, respectively), triethylamine, and t-butylamine.

The invention also includes all stereoisomers of the compounds, including diastereomers and enantiomers, as well as mixtures of stereoisomers, including, but not limited to, racemic mixtures. Unless stereochemistry is explicitly indicated in a structure, the structure is intended to embrace all possible stereoisomers of the compound depicted.

The term "alkyl" refers to saturated aliphatic groups including straight-chain, branched-chain, cyclic groups, and combinations thereof, having the number of carbon atoms specified, or if no number is specified, having up to 12 carbon atoms. Examples of alkyl groups include, but are not limited to, groups such as methyl, ethyl, n-propyl, isopropyl, n-butyl, isobutyl, sec-butyl, t-butyl, n-pentyl, neopentyl, cyclopropyl, cyclobutyl, cyclopentyl, cyclohexyl, and adamantyl. Cyclic groups can consist of one ring, including, but not limited to, groups such as cycloheptyl, or multiple fused rings, including, but not limited to, groups such as adamantyl or norbomyl. Alkyl groups may be unsubstituted, or may be substituted with one or more substituents including, but not limited to, groups such as halogen (fluoro, chloro, bromo, and iodo), alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. Examples of substituted alkyl groups include, but are not limited to, -CF₃, -CF₂-CF₃, and other perfluoro and perhalo groups.

The term "alkenyl" refers to unsaturated aliphatic groups including straight-chain, branched-chain, cyclic groups, and combinations thereof, having the number of carbon atoms specified, or if no number is specified, having up to 12 carbon atoms, which contain at least one double bond (-C=C-). Examples of alkenyl groups include, but are not limited to, -CH₂-CH=CH-CH₃ and -CH₂-CH₂-cyclohexenyl, there the ethyl group can be attached to the cyclohexenyl moiety at any available carbon valence. The term "alkynyl" refers to unsaturated aliphatic groups including straight-chain, branched-chain, cyclic groups, and combinations thereof, having the number of carbon atoms specified, or if no number is specified, having up to 12 carbon atoms, which contain at least one triple bond (-C≡C-). "Hydrocarbon chain" or "hydrocarbyl" refers to any combination of straight-chain, branched-chain, or cyclic alkyl, alkenyl, or alkynyl groups, and any combination thereof. "Substituted alkenyl," "substituted alkynyl," and "substituted hydrocarbon chain" or "substituted hydrocarbyl" refer to the respective group substituted with one or more substituents, including, but not limited to, groups such as halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group.

"Aryl" or "Ar" refers to an aromatic carbocyclic group having a single ring (including, but not limited to, groups such as phenyl) or multiple condensed rings (including, but not limited to, groups such as naphthyl or anthryl), and includes both unsubstituted and substituted aryl groups. Substituted aryls can be substituted with one or more substituents, including, but not limited to, groups such as alkyl, alkenyl, alkynyl, hydrocarbon chains, halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group.

"Heteroalkyl," "heteroalkenyl," and "heteroalkynyl" refer to alkyl, alkenyl, and alkynyl groups, respectively, that contain the number of carbon atoms specified (or if no number is specified, having up to 12 carbon atoms) which contain one or more heteroatoms as part of the main, branched, or cyclic chains in the group. Heteroatoms include, but are not limited to, N, S, O, and P; N and O are preferred. Heteroalkyl, heteroalkenyl, and heteroalkynyl groups may be attached to the remainder of the molecule either at a heteroatom (if a valence is available) or at a carbon atom. Examples of heteroalkyl groups include, but are not limited to, groups such as -O-CH₃, -CH₂-O-CH₃, -CH₂-CH₂-O-CH₃, -S-CH₂-CH₂-CH₃, -CH₂-CH(CH₃)-S-CH₃, -CH₂-CH₂-NH-CH₂-CH₂-,1-ethyl-6-propylpiperidino, 2-ethylthiophenyl, and morpholino. Examples of heteroalkenyl groups include, but are not limited to, groups such as -CH=CH-NH-CH(CH₃)-CH₂-. "Heteroaryl" or "HetAr" refers to an aromatic carbocyclic group having a single ring (including, but not limited to, examples such as pyridyl, thiophene, or furyl) or multiple condensed rings (including, but not limited to, examples such as imidazolyl, indolizinyl or benzothienyl) and having at least one hetero atom, including, but not limited to, heteroatoms such as N, O, P, or S, within the ring. Heteroalkyl, heteroalkenyl, heteroalkynyl and heteroaryl groups can be unsubstituted or substituted with one or more substituents, including, but not limited to, groups such as alkyl, alkenyl, alkynyl, benzyl, hydrocarbon chains, halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. Examples of such substituted heteroalkyl groups include, but are not limited to, piperazine, substituted at a nitrogen or carbon by a phenyl or benzyl group, and attached to the remainder of the molecule by any available valence on a carbon or nitrogen, -NH-SO₂-phenyl, -NH-(C=O)O-alkyl, -NH-(C=O)O-alkyl-aryl, and -NH-(C=O)-alkyl. The heteroatom(s) as well as the carbon atoms of the group can be substituted. The heteroatom(s) can also be in oxidized form. Unless otherwise specified, heteroalkyl, heteroalkenyl, heteroalkynyl, and heteroaryl groups have between one and five heteroatoms and between one and twenty carbon atoms.

The term "alkylaryl" refers to an alkyl group having the number of carbon atoms designated, appended to one, two, or three aryl groups.

The term "alkoxy" as used herein refers to an alkyl, alkenyl, alkynyl, or hydrocarbon chain linked to an oxygen atom and having the number of carbon atoms specified, or if no number is specified, having up to 12 carbon atoms. Examples of alkoxy groups include, but are not limited to, groups such as methoxy, ethoxy, and t-butoxy.

The terms "halo" and "halogen" as used herein refer to Cl, Br, F or I substituents.

"Protecting group" refers to a chemical group that exhibits the following characteristics: 1) reacts selectively with the desired functionality in good yield to give a protected substrate that is stable to the projected reactions for which protection is desired; 2) is selectively removable from the protected substrate to yield the desired functionality; and 3) is removable in good yield by reagents compatible with the other functional group(s) present or generated in such projected reactions. Examples of suitable protecting groups can be found in Greene et al. (1991) Protective Groups in Organic Synthesis, 2nd Ed. (John Wiley & Sons, Inc., New York). Preferred amino protecting groups include, but are not limited to, benzyloxycarbonyl (CBz), t-butyloxycarbonyl (Boc), t-butyldimethylsilyl (TBDIMS), 9-fluorenylmethyloxycarbonyl (Fmoc), or suitable photolabile protecting groups such as 6-nitroveratryloxy carbonyl (Nvoc), nitropiperonyl, pyrenylmethoxycarbonyl, nitrobenzyl, dimethyl dimethoxybenzil, 5-bromo-7-nitroindolinyl, and the like. Preferred hydroxyl protecting groups include Fmoc, benzyl, t-butyl, TBDIMS, photolabile protecting groups (such as nitroveratryl oxymethyl ether (Nvom)), Mom (methoxy methyl ether), and Mem (methoxy ethoxy methyl ether). Particularly preferred protecting groups include NPEOC (4-nitrophenethyloxycarbonyl) and NPEOM (4-nitrophenethyloxymethyloxycarbonyl). Amino acid protecting groups are well-known in the field of peptide synthesis, and include groups such as those disclosed in Stewart, J.M. and Young, J.D., Solid Phase Peptide Synthesis. 2nd Ed., Pierce Chemical Company: Rockford, IL, 1984; Atherton, E. and Sheppard, R.C., Solid Phase Peptide Synthesis: A Practical Approach, IRL Press: New York, 1989; Jones, J., The Chemical Synthesis of Peptides (International Series of Monographs on Chemistry, No. 23), Clarendon Press: Oxford, 1991; Bodanszky, M., The Practice of Peptide Synthesis, Springer-Verlag: New York, 1984; Bodanszky, M., Peptide Chemistry: A Practical Textbook. 2nd Ed., Springer-Verlag: New York, 1993; Bodanszky, M., Principles of Peptide Synthesis. 2nd Ed., Springer-Verlag: New York, 1993; Synthetic Peptides: A User's Guide (Grant, G.A., Ed.), W.H. Freeman: New York, 1992; and Barany, G. and Merrifield, R.B., "Solid Phase Peptide Synthesis", Chapter 1 (pp. 1-284) of The Peptides, Vol. 2, Academic Press: New York, 1979. Additional publications include the 97/98 Novabiochem Catalog and Peptide Synthesis Handbook and the Novabiochem Combinatorial Chemistry Catalog (Calbiochem-Novabiochem, San Diego, CA), and the user's manuals and synthesis bulletins for Perkin-Elmer Applied Biosystems (Foster City, CA) peptide synthesizers. Purification methods appropriate for peptides are discussed in the references cited above, and in High-Performance Liquid Chromatography of Peptides and Proteins: Separation, Analysis and Conformation (Mant, C.T. and Hodges, R.S.. Eds.), CRC Press: Boca Raton, FL, 1991. Materials for use in peptide synthesis, such as protected amino acids, synthesis reagents, solvents, and resin supports, are available commercially from a number of suppliers, including Calbiochem-Novabiochem, San Diego, CA; Advanced Chemtech, Louisville, KY; Bachem Bioscience, Inc., King of Prussia, PA; Sigma Chemical Company, St. Louis, MO; Richelieu Biotechnologies, Inc., Montreal, Quebec, Canada; Peninsula Laboratories, Inc., Belmont, CA; Perkin-Elmer Applied Biosystems, Inc., Foster City, CA; and Peptides International, Louisville, KY.

An "amino-capping group" or "amino-terminal capping group" or "N-terminal capping group" is a group that covalently links to an amino group. Examples of amino-capping groups include, but are not limited to, 4-morpholinocarbonyl, acetyl, and trifluoroacetyl.

### Novel quinones

The present invention encompasses novel quinones. While not wishing to be bound by any particular theory explaining quinone toxicity, the inventors suggest that the novel quinones can be designed based on the suspected DNA topoisomerase II-poisoning activity of quinones. Alternatively, quinone toxicity may be related to the compound's potential to undergo redox cycling with the formation of highly reactive oxygen species. O'Brien (1991) *Chem. Biol. Interactions* 80:1-41. In the next step, the quinone is tested *in vitro* for efficacy in inhibiting proliferation of diseased cells (such as tumor cells). If it is efficable, the quinone is then tested in animals, such as nude mice with tumor xenografts. Simultaneously, toxicity of the compound should be determined. If the quinone is found to efficable and safe, testing can then proceed to human trials.

### In vitro testing of novel quinones

Novel quinones of the present invention can be tested *in vitro* by any means known in the art. The quinones can be tested, for example, for toxicity against a chosen cell line, such as a tumor cell line.

### In vivo testing of novel quinones

Following a showing of efficacy of the novel quinones *in vitro,* these compounds can be tested *in vivo.* Typical tests include, but are not limited to, examinations of the effects of compound administration on animals, such as nude mice with tumor xenografts.

### Methods of administrating quinones

The novel quinone compounds of the present invention can be administered to an individual via any route known in the art, including, but not limited to, those disclosed herein. Preferably administration of the novel quinones is intravenous. Other methods of administration include but are not limited to, oral, intrarterial, intratumoral, intramuscular, subcutaneous, intraperitoneal, gastrointestinal, and directly to a specific or affected organ. The novel quinone compounds described herein are administratable in the form of tablets, pills, powder mixtures, capsules, injectables, solutions, suppositories, emulsions, dispersions, food premixes, and in other suitable forms. Additional methods of administration are known in the art. The pharmaceutical dosage form which contains the compounds described herein is conveniently admixed with a non-toxic pharmaceutical organic carrier or a non-toxic pharmaceutical inorganic carrier. Typical pharmaceutically-acceptable carriers include, for example, mannitol, urea, dextrans, lactose, potato and maize starches, magnesium stearate, talc, vegetable oils, polyalkylene glycols, ethyl cellulose, poly(vinylpyrrolidone), calcium carbonate, ethyl oleate, isopropyl myristate, benzyl benzoate, sodium carbonate, gelatin, potassium carbonate, silicic acid, and other conventionally employed acceptable carriers. The pharmaceutical dosage form can also contain non-toxic auxiliary substances such as emulsifying, preserving, or wetting agents, and the like. A suitable carrier is one which does not cause an intolerable side effect, but which allows the novel quinone compounds to retain its pharmacological activity in the body. Formulations for parenteral and nonparenteral drug delivery are known in the art and are set forth in *Remington's Pharmaceutical Sciences,* 18th Edition, Mack Publishing (1990). Solid forms, such as tablets, capsules and powders, can be fabricated using conventional tableting and capsule-filling machinery, which is well known in the art. Solid dosage forms can contain any number of additional non-active ingredients known to the art, including excipients, lubricants, dessicants, binders, colorants, disintegrating agents, dry flow modifiers, preservatives, and the like. Liquid forms for ingestion can be formulated using known liquid carriers, including aqueous and non-aqueous carriers, suspensions, oil-in-water and/or water-in-oil emulsions, and the like. Liquid formulations can also contain any number of additional non-active ingredients, including colorants, fragrance, flavorings, viscosity modifiers, preservatives, stabilizers, and the like. For parenteral administration, novel quinone compounds can be administered as injectable dosages of a solution or suspension of the compound in a physiologically acceptable diluent or sterile liquid carrier such as water or oil, with or without additional surfactants or adjuvants. An illustrative list of carrier oils would include animal and vegetable oils (peanut oil, soy bean oil), petroleum-derived oils (mineral oil), and synthetic oils. In general, for injectable unit doses, water, saline, aqueous dextrose and related sugar solutions, and ethanol and glycol solutions such as propylene glycol or polyethylene glycol are preferred liquid carriers. The pharmaceutical unit dosage chosen is preferably fabricated and administered to provide a final concentration of drug at the point of contact with the cancer cell of from 1 µM to 10 mM. More preferred is a concentration of from 1 to 100 µM. As with all pharmaceuticals, the optimal effective concentration of novel quinone compounds will need to be determined empirically and will depend on the type and severity of the disease, route of administration, disease progression and health and mass or body area of the patient. Such determinations are within the skill of one in the art.

### EXAMPLES

### EXAMPLE 1

### Synthetic Preparation of Quinone Compounds

Preparation of quinones is described below and depicted in the Figures.

New chemistry was developed in order to construct drugs where the 1,2-naphthoquinone moiety is bound to a DNA minor groove binder unit or a DNA intercalator. While not wishing to limit the invention to any particular theory of operation, it is believed that the 1,2-naphthoquinone derivatives "poison" topoisomerase II and transform this essential DNA replication enzyme into a nuclease-type enzyme that cleaves DNA. It is postulated that this modification of topoisomerase II by the 1,2-naphthoquinones is very likely due to the alkylation of the thiol residues of the enzyme by the quinones (Michael additions).

Scheme 1 outlines derivatization reactions leading to 1,2-naphthoquinone intermediates. The silver salt of 2-hydroxy-1,4-naphthoquinone was alkylated with the tert-butyl or benzyl esters of 5-bromo-pentanoic acid to give either **1** or **2.** The benzyl ester **2** was transformed into the acid **3** by hydrogenolysis. The silver salt was also alkylated with 6-bromohexanol to give **4,** or with 1,6-diiodohexane to give **5**. The alcohol 4 treated with triphosgene gives **6** (Scheme 2). The acid **3** can be derivatized by reaction with 3-amino-1-methyl-5-methyloxycarbonylpyrrole (Baird and Dervan (1996) *J. Am. Chem. Soc.* 118:6141) in the presence of o-benzotriazol-1-yl-N,N,N',N'-tetramethyluronium hexafluorophosphate (HBTU) and diisopropylethyl amine (DIEA) to give the amide **7**. The silver salt of 2-hydroxy-1,4-naphthoquinone reacted with pivalyl chloride to give **8** (Scheme 2). Acid **3** was condensed with the polypyrrole amide **9** (Baird and Dervan (1996) *J. Am. Chem. Soc.* 118:6141) after cleavage of the protecting t-butyl group with TFA. The resulting product **10** is a molecule where the 1,2-naphthoquinone moiety is covalently bound to a DNA minor groove binder (Scheme 3). Alcohol **4** was condensed using the Mitsonobu reaction (triphenylphosphine, diethyl acetylenedicarboxylate) with 4-hydroxybenzonitrile to give **11.** Iodide **5** was reacted with the tetrabutyl ammonium salt of phenol to give **12.**

The acid **3** was esterified with 3-dimethylaminophenol using dicyclohexylcarbodiimide (DCC) and 4-dimethylamino pyridine (DMAP) and gave **13.** By reaction of 5 and the tetrabutylammonium salt of Hoechst 33528 it was possible to obtain **14,** where the quinone is covalently bound to the DNA minor groove binder. By esterification of **4** with 6-aminohexanoic acid (used as its BOC derivative and deprotected with TFA) in the presence of DCC and DMAP, it was possible to obtain **15** as its trifluoroacetate (Scheme 4). By condensation of the acid **3** with the N-ethyl diamide **16,** the polyamide quinone **17** was prepared (Scheme 4).

A new class of 4-aminoalkyl substituted 1,2-naphthoquinones was obtained following the outline depicted in Scheme 5. A Vilsmeier reaction on 1,2-dimethoxynaphthalene gave the formyl derivative **18.** It was converted by reductive amination with n-butylamine into **19.** Treatment of **19** with acetyl chloride gave **20,** while treatment with trifluoroacetic anhydride gave **21** (Scheme 5). Acylation of **19** with morpholino succinyl chloride gave **22.** Cleavage of the 1,2-dimethoxy groups of **19** with boron tribromide gave the quinone **23** which was found to exist in the p-quinonemethine form. Cleavage of the dimethoxy residues of **20** and **21** led to the expected quinones **24** and **25.** Cleavage of the methoxy residues of **22** gave the quinone **26** (Scheme 5).

The 1,2-naphthoquinone residue was also covalently bound to a porphyrin backbone, since porphyrins are known to concentrate in cancer tissues. By reaction of the iodide **5** with the tetrabutylammonium salt of meso-p-hydroxyphenylporphyrin, the porphyrin quinone **27** was obtained (Scheme 6).

By esterification of 4,4',4'',4'''-(21H, 23H-porphine-5,10,15,20-tetrayl)tetrakis(benzoic acid) with the quinone alcohol 4 in the presence of EDCI (1,(3-dimethyl aminopropyl)-3-ethylcarbodiimide) and DMAP it was possible to prepare the quinone-porphyrin **28** (Scheme 7).

### Synthesis of 1,2-naphthoquinones bound to DNA intercalators

It is known that 4-aminoacridine derivatives intercalate in the DNA helix. Therefore syntheses of 1,2-naphthoquinone residues bound to 4-aminoacridine derivatives were designed (Scheme 8). The salt (6-hydroxyhexyl)triphenylphosphonium bromide was prepared by the reaction of 6-bromohexanol with triphenylphosphine in refluxing acetonitrile. Wittig reaction of (6-hydroxyhexyl)triphenylphosphonium bromide with 4-acetamidobenzaldehyde produced alkene **29** as a mixture of E and Z isomers. Reduction of the double bond (H₂, Pd/C) and acidic hydrolysis (2N HCl, MeOH) afforded 4-(7-hydroxyheptyl)-aniline **30.** Aniline **30** was reacted with 9-chloroacridine in MeOH in the presence of triethylamine to give alcohol **31.** Alcohol **31** was converted to iodide **32** by reaction with methanesulfonyl chloride in pyridine, followed by reaction with sodium iodide in acetone. Reaction of iodide **32** with the silver salt of 2-hydroxy-1,4-naphthoquinone afforded quinone **33** as a mixture of ortho- and para-quinone isomers. The ortho- and para-quinone isomers could be separated and purified by column chromatography.

A second approach to these types of compounds is shown in Scheme 9. The isomer mixture **34** was converted to the iodide **35** by reaction with methanesulfonyl chloride in CH₂Cl₂ in the presence of pyridine, followed by a displacement with sodium iodide in acetone. Reaction of **35** with triphenylphosphine in refluxing acetonitrile afforded the phosphonium salt. A Wittig reaction between the phosphonium salt and naphthaldehyde **18** produced diene **36** (as a mixture of double bond isomers). Reduction with H₂ over Pd/C followed by hydrolysis (2N HCl, MeOH) gave aniline **37.** Aniline **37** was reacted with 9-chloroacridine in MeOH in the presence of triethylamine to give **38.** Cleavage of the methyl ethers with boron tribromide gave quinone **39.**

A third synthetic approach to a 1,2-naphthoquinone moiety bound to an aminoacridine intercalator is depicted in Scheme 10. Aminoacridine was protected with mesitylenesulfonyl chloride to give **41,** which was then alkylated with 1,5-dibromopentane to **42.** The latter is brought into reaction with the silver salt of 2-hydroxy-1,4-naphthoquinone and the quinone-acridine **43** was thus obtained. Cleavage of the amide group using samarium iodide gave **44,** the expected compound.

### Synthesis of 1,2-naphthoquinol phosphates

In order to obtain 1,2-naphthoquinone derivatives that behave as "pro-drugs" the synthesis of quinol phosphates that can be hydrolyzed by cell phosphatases to liberate the parent quinones was carried out. Scheme 11 outlines the synthesis of the quinol phosphates. The parent 1,2-naphthoquinone **46** was brought into reaction with dibenzylphosphite to give a mixture of the two possible regioisomers **47.** By cleavage of the benzyl residues with hydrogen in the presence of 10% Pd on charcoal the mixture of the two possible quinol phosphates **48** was obtained. They were used as such in the biological studies.

### Synthesis of 8-hydroxy-β-lapachone 55

Scheme 12 outlines the synthesis of **55,** a phenol derivative of β-lapachone that could be used as a building block for the construction of peptide derivatives of β-lapachone. The synthesis starts with the commercially available ester **49,** that is acetylated using a Friedel-Crafts reaction to give **50.** Cyclization of **50** in the presence of base and air gave the p-quinone **51.** Alkylation of **51** with dimethyl allyl bromide gave a mixture of the C-alkyl derivative **52** and the O-alkyl derivative **53.** They were separated and on treatment of **52** with concentrated sulfuric acid, the 8-methoxy-β-lapachone **54** was obtained. Cleavage of the methoxy group with boron tribromide gave the expected σ-naphthoquinone **55.**

### Synthesis of 1,2-naphthoquinone bisulfite adducts

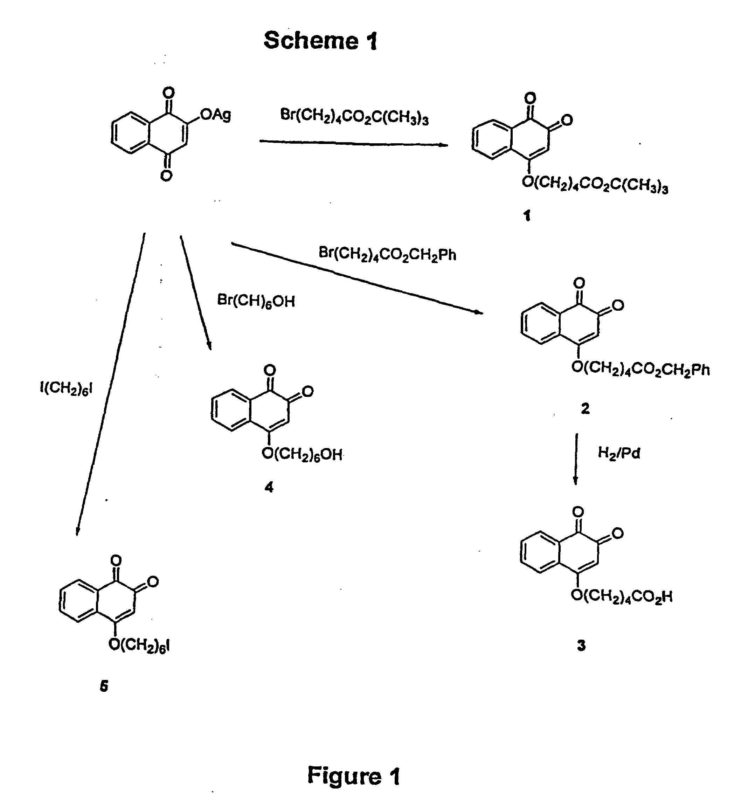

Bisulfite adducts of 1,2-naphthoquinones were prepared as "pro-drugs." They are stable in aqueous solutions at pH below 7 but liberate the quinone core at pH above **7.** Since biological media are usually above pH 7, the bisulfite adducts led to a slow release of the quinones after administration in an aqueous medium. A list of selected bisulfite adducts is given in Fig. 1. General preparation procedures are given in Experimental.

### Synthesis of 1,2-naphthoquinone peptides

1,2-Naphthoquinone conjugates of tetra and hexapeptides were prepared to obtain "prodrug" derivatives that can be cleaved by prostatic PSA. The guidelines followed for the synthesis of the peptides were based on the published results of Isaacs and coworkers (Denmeade et al. Cancer Res. 1997, 57, 4924), where they define the substrate specificity of PSA (prostate specific antigen). The synthesis of a quinone tetrapeptide is outlined in Scheme 13 for the 3-β-alanyloxy-β-lapachone (SL-11006) conjugate. SL-11006 (Quin) was coupled to Boc-Gln with DCC in the presence of 1-hydroxybenzotriazole to give Boc-Gln-Quin. Removal of the Boc group from Boc-Gln-Quin with TFA in CH₂Cl₂ gave TFA·Gln-Quin. Boc-Leu was coupled to TFA·Gln-Quin with DCC in the presence of 1-hydroxybenzotriazole to give Boc-Leu-Gln-Quin. Removal of the Boc group from Boc-Leu-Gm-Quin with TFA in CH₂Cl₂ gave TFA·Leu-Gln-Quin. Boc-Lys(Nε-Cbz) was coupled to TFA·Leu-Gln-Quin with DCC in the presence of 1-hydroxybenzotriazole to give Boc-Lys(Nε-Cl-Cbz)-Leu-Gln-Quin. Removal of the Boc group from Boc-Lys(Nε-Cbz)-Leu-Gln-Quin with TFA in CHCl₃ gave TFA·Lys(Nε-Cbz)-Leu-Gln-Quin. Morpholino-Ser(OBn) was coupled to TFA-Lys(Nε-Cbz)-Leu-Gln-Quin with DCC in the presence of 1-hydroxybenzotriazole to give morpholino-Ser(OBn)-Lys(Nε-Cbz)-Leu-Gln-Quin. The side chain protecting groups were removed by hydrogenolysis to yield morpholino-Ser-Lys-Leu-Gln-Quin. During the hydrogenolysis, the quinone was reduced to the hydroquinone, which reoxidized to the quinone on exposure to air.

Morpholino-Ser(OBn) was prepared from N-Fmoc-Ser(OBn). Esterification of N-Fmoc-Ser(OBn) with isobutylene in the presence of a catalytic amount of H₂SO₄ afforded N-Fmoc-Ser(OBn)-OtBu. The Fmoc group was removed with piperidine in CH₂Cl₂ to produce Ser(OBn)-OtBu. Reaction of Ser(OBn)-OtBu with 4-morpholinecarbonyl chloride in pyridine yielded morpholino-Ser(OBn)-OtBu. Morpholino-Ser(OBn)-OtBu was hydrolyzed with TFA in CH₂Cl₂ to yield morpholine-Ser(OBn).

The synthesis of a tetrapeptide conjugate of 3-leucyloxy-β-lapachone is outlined in Scheme 14.

### EXPERIMENTAL

***tert*****-Butyl δ-[(1,2-dihydro-1,2-dioxonaphth-4-yl)oxy]valerate (1).** A mixture of *tert*-butyl 5-bromovalerate (1 g, 4.2 mmol) and the silver salt of 2-hydroxy-1,4-naphthoquinone (0.8 g, 3.84 mmol) in benzene (10 mL), was stirred for 24 h at 50° C. The reaction mixture was filtered through celite and the solvent was removed *in vacuo.* The residue was purified by flash chromatography (5% methanol in chloroform) to give a yellow solid (384 mg, 30%). ¹H NMR (CDCl₃) 8.12 (d, J=7.7 Hz, 1H), 7.8° (d, J=7.7 Hz, 1H), 7.70 (t, J= 6.1 Hz, 1H), 7.59 (t, J=6.4 Hz, 1H), 5.95 (s, 1H), 4.17 (t, J=5.9 Hz, 2H), 2.35 (t, J=7.2 Hz, 2H), 1.90-2.05 (m, 2H), 1.78-1.90 (m, 2H), 1.47 (s, 9H).
**Benzyl 5-[(1,2-dihydro-1,2-dioxonaphth -4-yl)oxy]valerate (2).** A mixture of benzyl 5-bromovalerate (2.27 g, 8.4 mmol) and the silver salt of 2-hydroxy-1,4-naphthoquinone (1.63 g, 5.81 mmol) in benzene (8 mL) was stirred for 48 h at 55° C and filtered through celite. The filtrate was diluted with diethyl ether, extracted with a 20% aqueous solution of NaHSO₃ then basified to pH 10-11 with Na₂CO₃, and extracted with CH₂Cl₂. Yellow solid (1.334 g, 63%). ¹H NMR (CDCl₃) 8.12 (d, J=7.5 Hz, 1H), 7.85 (d, J=7.7 Hz, 1H), 7.68 (t, J=7.5, 1H), 7.58 (t, J=7.7 Hz, 1H), 7.25-7.50 (m, 5H), 5.93 (s, 1H), 5.14 (s, 2H), 4.15 (t, J=5.7 Hz, 2H), 2.50 (t, J=7.0 Hz, 2H), 1.8-2.2 (m, 4H).
**5-[(1,2-Dioxo-1,2-dihydronaphth-4-yl)oxy]valeric Acid (3).** Benzyl ester **2** (1.90 g, 5.22 mmol) was hydrogenated at 30 psi with Pd (400 mg) in ethyl acetate (120 mL) for 6 h. The catalyst was removed by filtration through celite, the solvent was evaporated *in vacuo* and the residue was oxidized with Ag₂O (1.45 g, 6.25 mmol) in Et₂O by stirring for 10 h. Following filtration and evaporation of the solvent the product was crystallized from benzene to afford 0.53 g of pure material. The mother liquor was purified by flash chromatography (CH₂Cl₂/MeOH 15:1), the product dissolved in CH₂Cl₂, extracted with aqueous NaHCO₃ solution, acidified to pH 1 with 3% HCl and extracted back with CH₂Cl₂ to give additional 0.25 g of pure material (total yield 55%), mp 134-136°C; ¹H NMR (CDCl₃) 8.12 (d, J=7.0 Hz, 1H), 7.87 (d, J=7.6 Hz, 1H), 7.70 (t, J=7.5 Hz, 1H), 7.59 (t, J=7.4 Hz, 1H), 7.27 (s, 1H), 4.18 (t, J=5.9 Hz, 2H), 2.51 (t, J=7.0 Hz, 2H), 1.75-2.15 (m, 4H).
**1,2-Dihydro-4-(6-hydroxyhexyloxy)-1,2-dioxo-naphthalene (4).** A mixture of 6-bromohexanol-1 (4.5 g, 24.85 mmol) and the silver salt of 2-hydroxy-1,4-naphthoquinone (6.46 g, 23.01 mmol) in benzene (24 mL) was stirred for 48 h at 60° C. The reaction mixture was worked up as described for **2** and crystallized from hexane to afford a yellow solid (3.18 g, 50%). mp 96-98°C, ¹H NMR (CDCl₃) 8.12 (d, J=7.5 Hz, 1H), 7.87 (d, J=7.7 Hz, 1H), 7.70 (t, J=7.5 Hz, 1H), 7.58 (t, J=7.5 Hz, 1H), 5.95 (s, 1H), 4.15 (t, J=6.3 Hz, 2H), 3.69 (t, J=6.2 Hz, 2H), 1.92-1.97 (m, 2H), 1.3-1.8 (m, 7H)
**1,2-Dihydro-4-(6-iodohexyloxy) -1,2-dioxonaphthalene (5).** A mixture of 1,6-diiodohexane (10.14 g, 30 mmol) and the silver salt of 2-hydroxy-1,4-naphthoquinone (2.81 g, 10 mmol) in benzene (60 mL) was stirred for 12 h at room temperature. The reacton mixture was filtered through Celite, concentrated *in vacuo,* and purified by flash chromatography (hexane/ EtOAc 4:1) to give a yellow solid (2,19 g, 57%); mp 85-87°C; ¹H NMR (CDCl₃) 8.12 (dd, J=6.5, 1.0 Hz, 1H), 7.86 (dd, J=6.9, 0.9 Hz, 1H), 7.70 (dt, J=7.6, 1.5 Hz, 1H), 7.58 (dt, J=7.5, 1.3 Hz, 1H), 5.95 (s, 1H), 4.15 (t, J=6.3, 2H), 3.22 (t, J=6.9 Hz, 2H), 1.80-2.05 (m, 4H), 1.45-2.10 (m, 4H).
**bis [6-[(1,2- Dihydro-1,2-dioxonaphth-4-yl)oxy]hexyl]carbonate (6).** Pyridine (0.12 ml, 1.5 mmol) was added to a stirred solution of the alcohol 4 (200 mg, 0.73 mmol) and bis(trichloromethyl)carbonate (40 mg, 0.134 mmol) in CH₂Cl₂ (5 mL) at 0°C. The cooling bath was removed, the reaction mixture was diluted with CH₂Cl₂, washed with 3% HCl, brine, dried (Na₂SO₄) and purified by column chromatography (benzene/EtOAc 4:1, 2:1). The product was triturated with Et₂O to afford a yellow solid (127 mg, 30%), mp 78-82°C (decomp.). MS (LSIMS, 3-NBA) 576 (M⁺+2), 401, 175; ¹H NMR (CDCl₃) 8.09 (dd, J=6.0, 1.6 Hz, 1H), 7.85 (dd, J=7.8, 1.2 Hz, 1H), 7.71 (t, J=6.9 Hz, 1H), 7.58 (t, J=6.2 Hz, 1H), 5.94 (s, I H), 4.17 (t, J=6.0 Hz, 2H), 4.15 (t, J=5.6 Hz, 2H), 1.85-2.10 (m, 2H), 1.65-1.85 (m, 2H), 1.40-1.65 (m, 4H).
**N-(1-Methyl-5-methyloxycarbonylpyrrol-3-yl)-5-[(1,2-dihydro-1,2-dioxonaphth -4-yl)oxy]valeramide (7).** A solution of an acid 3 (334 mg, 1.22 mmol) in DMF (1.67 mL) was treated with HBTU (462 mg, 1.22 mmol) followed by DIEA (452 mg, 3.5 mmol) and stirred for 5 min. 3-Amino-1-methyl-5-methyloxycarbonylpyrrol hydrochloride (232 mg, 1.22 mmol) and DIEA (378 mg, 3 mmol) were added to the reaction mixture. The latter was stirred for 2 h, diluted with Et₂O, the precipitate was removed, dissolved in CHCl₃, washed with 3% HCl, H₂O, aqueous NaHCO₃, H₂O again, dried (MgSO₄) and purified by chromatography on alumina column (CHCl₃/MeOH 80:1, 50:1). The product was triturated with Et₂O/CHCl₃ to obtain a yellow-red solid (200 mg, 40%); mp 122-123° C (decomp.): MS (LSIMS. 3-NBA) 410 (M⁺), 237 (M⁺-173). ¹H NMR (CDCl₃) 8.08 (d, J=7.5 Hz, 1H), 7.86 (d, J=7.3 Hz, 1H), 7.68 (t, J=7.5 Hz, 1H), 7.57 (t, J=7.5 Hz, 1H), 7.38 (d, J= 1.8 Hz, 1H), 7.34 (s, 1H), 6.65 (d, J= 2 Hz, 1H), 5.95 (s, 1H), 4.19 (t, J=5.53 Hz, 2H), 3.88 (s, 3H), 3.80 (s, 3H), 2.46 (t, J=6.6 Hz, 2H), 1.90-2.15 (m, 4H).
**4-(*****tert*****-Butylcarbonyloxy)-1,2- dihydro-1,2-dioxonaphthalene (8).** A mixture of the silver salt of 2-hydroxy-1,4-naphthoquinone (842 mg, 3 mmol), and pivaloyl chloride (434 mg, 3.6 mmol) in benzene (5 mL) was stirred for 8 h at room temperature. The reaction mixture was filtered through Celite, the precipitate washed with EtOAc, and the combined organic solutions were concentrated *in vacuo* and purified by flash chromatography (EtOAc/hexane 1:10, 1:5). The product was recrystallized from hexane to afford a yellow solid (190 mg, 25%); mp 125-126°C; ¹H NMR (CDCl₃) 8.15(dd, J= 7.7, 1.1 Hz, 1H), 7.71(dt, J=7.7, 1.5 Hz, 1H), 7.59 (dt, J=7.5, 1.2 Hz, 1H), 7.57 (dd, J= 7.6, 1.1 Hz, 1H), 6.48 (s, I H), 1.44 (s, 9H).
**N-[3-(Dimethylamino)propyl][3-[[3-[[3-[4-[(1,2-dihydro-1,2-dioxonaphth-4-yl)oxy]butylcarbonylamino]-1-methylpyrrol-5-yl]carbonylamino]-1-methylpyrrol-5-yl]carbonylamino]-1-methylpyrrol-5-yl]carboxamide (10)** was prepared from acid **3** (61 mg, 0.222 mmol) and Boc-protected pyrrolylamine **9** (84 mg, 0.148 mmol) using the procedure described for **7**. After the reaction was completed, the reaction mixture was diluted with Et₂O, the precipitate was removed, triturated with hot EtOAc and crystallized from a CHCl₃/ Et₂O mixture. The product was a yellow solid (30 mg, 28%); mp 159-162° C (decomp.); ¹H NMR (DMSO-d₆) 9.90 (s, 1H), 9.89 (s, 1H), 9.86 (s, 1H), 8.08 (bs, 1H), 7. 97 (d, J= 7.7 Hz, 1H), 7.87 (d, J=7.2 Hz, 1H), 7.81 (t, J=7.9 Hz, 1H), 7.68 (t, J=7.2, 1H), 7.24 (s, 1H), 7.19 (s, 1H), 7.04 (s, 1H), 6.89 (s, 1H), 6.84 (s, 1H), 6.06 (s, 1H), 4.25 (t, J= 5.8 Hz, 1H), 3.85 (s, 3H), 3.84 (s, 3H), 3.80 (s, 3H),3.12-3.30 (m, 2H), 2.25-2.45 (m, 4H), 2.19 (s, 6H), 1.72-2.00 (m, 4H), 1.60-1.70 (m, 2H).). MS (LSIMS, 3-NBA) 725.2 (M⁺+1).
**1,2-Dihydro-4-[6-[(4-cyanophenyl)oxy]hexyloxy] -1,2-dioxonaphthalene (11).** A mixture of 4-hydroxybenzonitrile (87 mg, 0.73 mmol), naphthoquinone **4** (200 mg, 0.73 mmol), PPh₃ (191 mg, 0.73 mmol) in dioxane (10 mL) was cooled to 10°C and treated with DEAD (140 mg, 0.80 mmol). The reaction mixture was stirred for 10 h, , concentrated *in vacuo* and purified by chromatography (5% EtOAc in benzene) to afford **11** as a yellow solid (171 mg, 53%), ¹H NMR (CDCl₃) 8.13 (dd, J=7.3, 1.4 Hz, 1H), 8.86 (dd, J=7.7, 1.1 Hz, 1H), 7.67 (dt, J=7.5, 1.5Hz 1H), 7.60 (dt, J=7.5, 1.5 Hz, 1H), 7.57 (d, J=8.8 Hz, 2H), 6.93 (d, J=8.9 Hz, 2H),5.96 (s, 1H), 4.17 (t, J=6.4 Hz, 2H), 4.03 (t, J=6.3 Hz, 2H), 1.80-2.05 (m, 4H), 1.58-1.68 (m, 4H).
**1,2-Dihydro-4-[6-(phenyloxy)hexyloxy] -1,2-dioxonaphthalene (12).** Phenol (28 mg, 0.3 mmol) was treated with tetrabutylammonium hydroxide (0.3 mL of 1.0 M solution in methanol) and the reaction mixture was concentrated to dryness *in vacuo.* Iodonaphtoquinone **5** (115 mg, 0.3 mmol) in DMF (3 mL) was added to the tetrabutylammonium salt, stirred for 48 h and quenched with H₂O (10 mL). The product was extracted with CHCl₃, the extract was washed with H₂O, then brine, dried (Na₂SO₄), and purified by chromatography (5% EtOAc in benzene) to give 12 as a yellow solid (45 mg, 43%) ¹H NMR (CDCl₃) 8.13 (d, J=7.4 Hz, 1H), 7.86 (d, J=7.4 Hz, 1H), 7.67 (t, J=7.6 Hz, 1H), 7.61 (t, J=7.5 Hz, 1H),7.15-7.40 (m, 2H), 6.85-7.10 (m, 3H), 5.96 (s, 1H), 4.17 (t, J=6.5 Hz, 2H), 3.99 (t, J=6.2 Hz), 1.70-2.10 (m, 4H), 1.35-1.70 (m, 4H).
**3-Dimethylaminophenyl 5-[(1,2-dihydro-1,2-dioxonaphth-4-yl)oxy]valerate (13).** A mixture of acid **3** (137 mg, 0.5 mmol), 3-dimethylaminophenol (82 mg, 0.6 mmol), DCC (103 mg, 0.5 mmol), and DMAP (12 mg, 0.01 mmol) in THF (2 mL) was stirred for 2 h.
The reaction mixture was concentrated *in vacuo,* the residue dissolved in benzene, washed with H₂O and dried (Na₂SO₄). Column chromatography (10% EtOAc) in benzene gave 13 as a yellow solid (70 mg, 36%), ¹H NMR (CDCl₃) 8.13, (d, J=7.3 Hz, 1H), 7.90 (d, J=7.4 Hz, 1H), 7.69 (t, J=6.1 Hz, 1H), 7.58 (t, J=7.6 Hz, 1H), 7.22 (dd, J=8.1, 8.1 Hz, 1H), 6.30-6.70 (m, 2H), 5.96 (s, 1H), 4.21 (t, J=5.6 Hz, 2H), 2.69 (t, J=6.5 Hz, 2H), 1.90-2.15 (m, 4H).
**2'-[4-[6-(1,2-Dihydro-1,2-dioxo-naphth-4-yl)oxyhexyl]oxyphenyl]-5-(4-methylpiperazin-1-yl)-2,5'-bi-1H-benzimidazole (14).** Hoechst 33258 (3.0 g, 5 mmol) was dissolved in a hot mixture of isopropanol-water (24 mL/ 12 mL) and neutralized with ammonium hydroxide (3 mL). The precipitate was filtered, triturated with Et₂O and dried *in vacuo* to obtain the free base of bisbenzimidazole. A 1.0 M solution of Bu₄NOH in MeOH (0.6 mL, 0.6 mmol) was added to the solution of bisbenzimidazole (1.635 g, 3.85 mmol) in MeOH (30 mL), stirred for 15 min and concentrated to dryness *in vacuo*. Iodonaphthoquinone 5 (1.485 g, 3.87 mmol) in DMF (30 mL) was added to the tetrabutyl ammonium salt and the mixture was stirred for 48 h. The reaction mixture was suspended in H₂O, the crude product was filtered, washed with H₂O, dried and purified by flash chromatography (MeOH/CHCl₃ 1:9, 1:5) to afford **14** as a yellow solid (790 mg, 30%). ¹H NMR (CDCl₃/MeOH-d₄) 8.21 (s, 1H), 8.09 (d, J=7.6 Hz, 1H), 8.05 (d, J=8.7 Hz, 2H), 7.85-7.95 (m, 2H), 7.48-7.75 (m, 4H), 7.14 (bs, 1H), 7.10-6.98 (m 3H), 4.21 (t, J=6.3 Hz, 2H), 4.08 (t, J=6.2 Hz, 2H), 2.65-2.75 (m, 4H), 2.40 (s, 3H), 1.80-2.15 (m, 4H), 1.60-1.75 (m, 4H). MS (LSIMS, 3-NBA) 725.2 (M⁺+1).
**Trifluoroacetate of 6-[(1,2-dihydro-1,2-dioxonaphth-4-yl)oxy]hexyl 6-aminohexanoate (15).** [6-(*tert*-Butyloxycarbonyl)amino]hexanoic acid (139 mg, 0.6 mmol) was added into solution of DCC (113 mg, 0.55 mmol) and DMAP (64 mg, 0.52 mmol) in CH₂Cl₂ (10 mL) at 0°C and stirred for 15 min, when naphthoquinone 4 (137 mg, 0.5 mmol) was added. The reaction mixture was stirred for 12 h at room temperature, diluted with CH₂Cl₂, extracted 3 times with an aqueous solution of KHSO₄, then with a NaHCO₃ solution followed by brine, dried (MgSO₄), and finally it was concentrated to dryness *in vacuo* and triturated with Et₂O. The residue was dissolved in CH₂Cl₂ (3 mL), TFA (0.5 mL) was added to the solution and the mixture stirred at 0°C for 1h. All volatiles were removed *in vacuo* and the residue was triturated in Et₂O to give **15** (100 mg, 40%). as a dark yellow oil. ¹H NMR (CDCl₃) 8.90 (d, J=7.6 Hz, 1H), 7.99 (bs, 3H), 7.87 (d, J=7.8 Hz, 1H), 7.71 (t, J=7.6 Hz, 1H), 7.59 (t, J=7.5 Hz, 1H), 5.96 (s, 1H), 4.17 (t, J=6.3 Hz, 2H), 4.09 (t, J=6.2 Hz, 2H), 2.90-3.15 (m, 2H), 2.29 (t, J=7.1 Hz, 2H), 1.90-2.10 (m, 2H), 1.30-1.85 (m, 12H).
**1,2-Dihydro-1,2-dioxo-4-[4-[2-[3-[2-(Ethylaminocarbonyl)ethylaminocarbonyl]propyl= aminocarbonyl]ethylaminocarbonyl]butyloxy]naphthalene (17).** Acid **3** (137 mg, 0.5 mmol) was dissolved in DMF (1 mL), treated with HBTU (190 mg, 0.5 mmol) followed by DIEA (260 µL, 1.5 mmol) and stirred for 10 min. N-Ethyl[2-[3-(2-aminoethylcarbonylamino) propylcarbonylamino]ethyl]carboxamide hydrochloride **16** (154 mg, 0.5 mmol) and DIEA (260 µL, 1.5 mmol) were added to the reaction mixture, the latter was stirred for 2 h, and the reaction mixture was diluted with Et₂O. The product was filtered and triturated with CHCl₃ to afford a yellow solid (100 mg, 38%), mp 145-170°C (decomp.) ¹H NMR (CDCl₃, MeOH-d₄) 8.10 (dd, J=7.6, 1.4 Hz, 1H), 7.92 (dd, J=7.8, 1.2 Hz, 1H), 7.72 (dt, J=7.7, 1.2 Hz, 1H), 7.62 (dt, 7.6, 1.3Hz, 1H), 7.30-7.50 (m, 2H), 7.15 (bs, 1H), 5.97 (s, 1H), 4.20 (t, J=5.8 Hz, 2H), 3.35-3.50 (m, 4H), 3.10-3.30 (m, 4H), 3.32-3.42 (m, 4H), 2.30 (t, J=6.9 Hz, 2H), 2.19 (t, J=7.4 Hz, 2H), 1.75-2.05 (m, 4H), 1.78 (t, J=7.2, 2H), 1.13 (t, J=7.3, 3H). MS (FAB, NaI) 551.2 (M+Na), 529 (M⁺+1).
**3,4-Dimethoxy-1-naphthaldehyde (18).** A mixture of 1,2-dimethoxynaphthalene (0.74 g, 4 mmol) and DMF (0.8 mL, 10 mmol) in dichlorobenzene (0.8 mL) was stirred with POCl₃ at 100°C for 2h. The reaction mixture was cooled to 0°C, quenched with a cold aqueous solution of NaOAc, diluted with H₂O and extracted with benzene. The extracts were dried (MgSO₄), concentrated and *in vacuo* and dichlorobenzene was removed by kugelrohr distillation at 110°C/0.5 mm Hg.. Column chromatography (20%EtOAc in hexane) gave the product **18** (596 mg, 68%), which was used in the following step without further purification. ¹H NMR (CDCl₃) 10.42 (s, 1H), 9.00-9.15 (m, 1H), 8.15-8.30 (m, 1H), 7.61 (s, 1H), 7.50-7.65 (m, 2H), 4.12 (s, 3H), 4.07 (s, 3H).
**4-Butylaminomethyl-1,2-dimethoxy-naphthalene (19).** A suspension of PtO₂ (40 mg) in EtOH (2 mL) was stirred with H₂ at 25 psi for 30 min. Naphthaldehyde **18** (596 mg, 2.8 mmol) was dissolved in EtOH and added into the suspension followed by the addition of butylamine (219 mg, 3 mmol). The reaction mixture was hydrogenated for 6 h at 50 psi. The catalyst was filtered through Celite, washed with acetone and the filtrate was concentrated to dryness to give **19** as an oil (665 mg, 87%). The product was utilized in the following step without further purification. ¹H NMR (CDCl₃) 8.16 (d, J=7.5 Hz, 1H), 7.99 (d, J=7.6 Hz, 2H), 7.40-7.60 (m, 2H), 7.35 (s, 1H), 4.19 (s, 2H), 4.00 (s, 3H), 3.98 (s, 3H), 2.76 (t, J=7.0 Hz, 2H), 1.64 (bs, 1H), 1.45-1.60 (m. 2H), 1.30-1.45 (m, 2H), 0.93 (t, J=7.2 Hz, 3H).
**4-(N-Acetyl-N-butylaminomethyl)-1,2-dimethoxy-naphthalene (20).** Triethylamine (350 µL, 2.5 mmol) was added to a solution of aminonaphthalene **19** (250 mg, 0.9 mmol) and AcCl (90µL, 1.27 mmol) in CH₂Cl₂ (5 mL) at 0 °C. The cooling bath was removed after 10 min, the reaction mixture was stirred for 1h at room temperature, diluted fivefold with CH₂Cl₂, washed with an aqueous solution of NaHCO₃ followed by 3% HCl, brine and dried (MgSO₄). The crude product (315 mg, 100%) obtained after evaporation of the solvent was used in the following step without further purification. ¹H NMR (CDCl₃) 8.19, 8.15 (2d, J=7.6, 8.4 Hz, 1H), 8.97, 7.80 (2d, J=7.9, 8.2 Hz, 1H), 7.35-7.58 (m, 2H), 7.16, 7.04 (2s, 1H), 5.05, 4.95 (2s, 2H), 4.01, 3.99 (2s, 3H), 3.99, 3.96 (2s, 3H), 3.47, 3.13 (2t, J=7.4, 7.8 Hz, 2H), 2.20, 2.09 (2s, 3H), 1.15-1.70 (m, 4H), 0.91, 0.87 (2t, J=7.2, 7.3 Hz, 3H).
**4-(N-Butyl-N-trifluoroacetylaminomethyl)-1,2-dimethoxy-naphthalene (21).** Naphthalene **19** (200 mg, 0.73 mmol) was acylated with trifluoroacetyl anhydride (210 mg, 1 mmol) in the presence of TEA (0.2 mL, 1.5 mmol) by raising the temperature during 3 h from -40° to 0°C. The reaction mixture was diluted with CH₂Cl₂, washed with aqueous NaHCO₃, 3% HCl, brine and finally dried (MgSO₄). The crude product (266 mg, 99%) was used in the following step without further purification. ¹H NMR (CDCl₃) 8.17-8.25 (m, 1H), 7.82 (t, J=7.7 Hz, 1H), 7.40-7.55 (m, 2H), 7.16, 7.03 (2s, 1H), 5.11, 5.08 (2s, 2H), 4.01, 4.03 (2s, 3H), 3.98, 3.96 (2s, 3H), 3.40, 3.25 (2t, J=7.5, 7.4 Hz, 2H), 1.45-2.75 (m, 2H), 1.10-1.45 (m, 2H), 4.89 (t, J=7.4, 3H).
**4-[N-Butyl-N-[3-(4-morpholinocarbonyl)ethylcarbonyl]aminomethyl]-1,2-dimethoxynaphthalene (22).** 3-(N-Morpholinocarbonyl)propionic acid (139 mg, 0.74 mmol) in CH₂Cl₂ (5 mL) was heated to reflux with thionyl chloride (440 mg, 3.7 mmol) for 1h and all volatiles were evaporated *in vacuo.* The residue was dissolved in anhydrous CH₂Cl₂ (3 mL), cooled to 0°C and naphthalene **19** (100 mg, 0.37 mmol), followed by DMAP (45 mg, 0.37 mmol) and TEA (140µL, 1 mmol) were added into the reaction mixture. After stirring for 1h at room temperature the reaction was quenched with wet EtOAc (10 mL), washed with 3% HCl, aqueous NaHCO₃, brine, and dried (Na₂SO₄). Purification by chromatography (15%EtOAc in hexane) gave **22** (160 mg, 98%). The product was used directly in the next step. ¹H NMR (CDCl₃) 8.19, 8.17 (2d, J=7.7, 7.8 Hz, 1H), 7.92, 7.85 (2d, J=8.2, 8.05 Hz, 1H), 7.38-7.56 (m, 4H), 7.25, 7.17 (2s, 1H), 5.05, 5.03 (2s, 2H), 4.03, 4.00 (2s, 3H), 3.99, 3.98 (2s, 3H), 3.25-3.82 (m, 14H), 1.15-1.82 (m, 4H), 0.88. 0.85 (2t, J=7.1, 6.7 Hz, 3H).
**Demethylation of dimethoxynaphthalenes with boron tribromide. 4-(Butylamino= methylene)-1,4-dihydro-2-hydroxy-1-oxo-naphthalene (23).** A solution of dimethoxynaphthalene **19** (30 mg, 0.11 mmol) in CH₂Cl₂ (2 mL) was treated with a 1M solution of BBr₃ in CH₂Cl₂ (1.1 mL) at -78°C and stirred at this temperature for 2h. The reaction mixture was placed in a freezer at -10°C for 3 h, quenched with Et₂O (1 mL) by stirring for 15 min at room temperature and neutralized with aqueous solution of NaHCO₃. The product was extracted with EtOAc, dried (MgSO₄), and the solvent was removed in *vacuo.* The residue was dissolved in Et₂O, stirred for 10 h in an open flask and purified by chromatography (5% MeOH in CHCl₃). Trituration with Et₂O yielded the product 23 (8 mg, 30%). ¹H NMR (CDCl₃) 9.05 (bs, 1H), 8.31 (d, J=8.1 Hz, 1H), 7.65-7.85 (m, 1H), 7.05-7.65 (m, 3H), 3.20-3.60 (m, 2H), 1.50-1.85 (m. 2H), 2.25-1.50 (m, 2H), 0.80-1.10 (m, 3H). HRMS (EI) 243.1250. Calcd for C₁₅H₁₇NO₂ 243.1259.
**4-(N-Acetyl-N-butylaminomethyl)- 1,2-dihydro-1,2-dioxonaphthalene (24)** was prepared from dimethoxynaphthalene **20** using the procedure described for **23.** The product (60%) was purified by chromatography (1.5% MeOH in CHCl₃) followed by trituration with Et₂O. ¹H NMR (CDCl₃) 8.1 (dd, J=7.53, 1.2 Hz, 1H), 7.67 (dd, J=7.7, 1.1 Hz, 1H), 7.50-7.62 (m, 2H), 6.21 (s, 1H), 4.68 (s, 2H), 3.35 (t, J=8.0 Hz, 2H), 2.25 (s, 3H), 1.50-1.75 (m, 2H), 1.15-1.50 (m, 2H), 0.96 (t, J=5.8, 3H). HRMS (EI) 285.1383. Calcd for C₁₇H₁₉NO₃ 285.1365.
**4-(N-Butylaminomethyl-N-trifluorocetyl)-1,2-dihydro-1,2-dioxonaphthalene (25)** was obtained from dimethoxynaphthalene **21** using the procedure described for **23.** The product (37%) was purified by chromatography (3% MeOH in CHCl₃) followed by trituration in Et₂O. ¹H NMR (CDCl₃) 8.29 (d, J=7.13 Hz, 1H), 7.40-7.85 (m, 3H), 6.19 (s, 1H), 4.73 (s, 2H), 3.35-3.70 (m, 2H), 1.50-1.80 (m, 2H), 1.35-1.80 (m, 2H), .96 (t, J=7.2 Hz, 3H). HRMS (EI) 339.1106. Calcd for C₁₇H₁₆F₃NO₃ 339.1082.
**4-[[N-Butyl-N-(4-morpholino-4-oxobutyryl)amino]methyl]-1,2-dihydro-1,2-dioxonaphthalene (26)** was obtained from dimethoxynaphthalene **22** using the procedure described for **23.** The product (10%) was purified by chromatography (25%-40% EtOAc in hexane) followed by trituration in Et₂O. ¹H NMR (CDCl₃) 8.19 (d, J=7.4 Hz, 1H), 7.70 (t, J=6.4 Hz, 1H), 7.59 (d, J=6.5 Hz, 1H), 7.50 (t, J=7.9 Hz, 1H), 6.33 (s, 1H), 4.65 (s, 2H), 3.35-3.80 (m, 14 H), 1.65-1.85 (m, 2H), 1.25-1.50 (m, 2H), 0.96 (t, J=7.2 Hz, 3H).
**meso-Tetra[4-[6-[(1,2-dihydro-1,2-dioxonaphth -4-yl)oxy]hexyloxy]phenyl]porphine (27).** A 1 M solution of Bu₄NOH in MeOH (0.212 mL,) was added to a stirred solution of meso-tetra(4-hydroxyphenyl)porphine (36 mg, 0.53 mmol) in MeOH (5 mL), stirring was kept for 10 min and the mixture concentrated to dryness *in vacuo.* Naphthoquinone **5** (81 mg, 0.21 mmol) in DMF (2 mL) was added to the porphyrin, the solution stirred for 48 h and diluted with H₂O (20 mL). The product was extracted with CHCl₃, washed with brine, the solvent was evaporated and the residue was triturated with Et₂O. Purification by flash chromatography (2-3% MeOH in CHCl₃) followed by recrystallization from CHCl₃/Et₂O (1:3) afforded the product as a dark red solid (19.6 mg, 21%). ¹H NMR (CDCl₃) 8.86 (s, 8H), 8.01-8.15 (m, 12H), 7.9 (d, J=7.8 Hz, 4H), 7.68 (t, J=6.3 Hz, 4H), 7.55 (t, J=7.5 Hz, 4H), 7.27 (d, J=7.8 Hz, 8H), 5.98 (s, 4H), 4.15-4.30 (m, 16 H), 1.80-2.10 (m, 16H), 1.65-1.80 (m, 16H). Anal. Calcd for C₁₀₈H₉₄N₄O₁₆x1.5 H₂O: C, 74.87, H, 5.43; N, 3.23. Found: C, 74.62; H, 5.57; N, 3.11.
**meso-Tetra[4-[6-[(1,2-dihydro-1,2-dioxanaphth-4-yl)oxybexyl]oxycarbonyl]phenyl]porphyrin (28).** EDCI (518 mg, 2.7 mmol) was added at 0°C to a mixture of meso-tetra(4-carboxyphenyl)porphyrin (500 mg, 0.63 mmol), alcohol 4 (831 mg, 3 mmol), and DMAP (159 mg, 1.3 mmol) in CH₂Cl₂ (10 mL). The solution was stirred for 2 h, the cooling bath was removed and the reaction mixture was left at room temperature overnight. It was diluted with CH₂Cl₂, washed with 2% HCl, H₂O, aqueous solution of NaHCO₃, H₂O, 5% aqueous solution of NaHSO₃, H₂O, dried (Na₂SO₄) and concentrated *in vacuo.* The analytical sample was prepared by column chromatography on silica (2% MeOH in CHCl₃). Mp 98-110°C (decomp.) Yield 572 mg, 50%. ¹H NMR (CDCl₃) 8.81 (s, 8H,), 8.45 (d, J=8.2 Hz, 8H), 8.30 (d, J=8.0 Hz, 8H), 8.09 (d, J=6.9 Hz, 4H), 7.89 (d, J=7.3 Hz, 4H), 7.70 (t, J=7.1 Hz, 4H), 7.56 (t, J=7.1 Hz, 4H), 5.98 (s, 4H), 4.56 (t, J=6.5 Hz, 8H), 4.21 (t, J=6.1 Hz, 8H), 1.85-2.20 (m, 16H), 1.60-1.80 (m, 16H). MS (MALDI) 1838 (M⁺+23), 1817 (M⁺+1). Anal. Calcd for C₁₁₂H₉₄N₄O₂₀x4 H₂O: C, 71.18; H, 5.40; N, 2.97. Found: C, 71.27; H, 5.24; N, 3.03.
**N-Acetyl-4-(7-hydroxy-1-heptenyl)-aniline (29)**. A solution of 5.213 g (28.8 mmol) of 6-bromohexanol and 7.55 g (28.8 mmol) of triphenylphosphine in 50 mL of CH₃CN was refluxed for 24 hr. Evaporation of solvent yielded the crude phosphonium salt, which was used directly in the next reaction. The crude phosphonium salt and 4.690 g (28.7 mmol) of 4-acetamidobenzaldehyde were dissolved in a mixture of 150 mL of CH₂Cl₂ and 150 mL of THF. To the cooled solution was added 1.529 g (60.5 mmol) of 95% NaH as a slurry in CH₂Cl₂ (10mL). The reaction mixture was stirred in an ice bath for 1 hr, then at room temperature for 19 hr. The mixture was partitioned between 350 mL CH₂Cl₂ and 500 mL 1N HCl. The aqueous phase was extracted with CH₂Cl₂ (4 x 100 mL). The CH₂Cl₂ extracts were combined, dried with MgSO₄, and evaporated to dryness. Column chromatography on silica gel eluting first with 1% MeOH in CH₂Cl₂ and then with 2% MeOH in CH₂Cl₂ afforded 4.913 g (69% from 6-bromohexanol) of alkene **29** as a mixture of *E* and *Z* isomers: ¹H NMR (250 MHz, CDCl₃, TMS) δ 7.5- 7.4 (m,4H), 7.3-7.1 (m, 4H), 6.4-6.3 (m, 2H), 6.2-6.1 (m, 1H), 5.7-5.6 (m, 2H), 3.65 (t, J = 6.5 Hz, 2H), 3.63 (t, J = 6.5 Hz, 2H), 2.4-2.1 (m, 4H), 2.18 (s, 3H), 2.17 (s, 3H), 1.7-1.3 (m, 12H).
**4-(7-Hydroxyheptyl)-aniline (30).** To a solution of 4.913 g (19.9 mmol) of N-acetyl-4-(7-hydroxy-1-heptenyl)-aniline **29** in 100 mL of 10% MeOH in CH₂Cl₂ in a Parr bottle were added 490 mg of 10% Pd/C. The bottle was placed on a hydrogenation apparatus and shaken for 4 hr at 25 psi of hydrogen. Removal of catalyst by filtration through a celite pad and evaporation of solvent afforded 5.294 g of alkane: ¹H NMR (300 MHz, CDCl₃, TMS) δ 7.80 (s, NH), 7.38 (d, J = 8 Hz, 2H), 7.09 (d, J = 8 Hz, 2H), 3.61 (t, J = 6.6 Hz, 2H), 2.54 (t, J = 7.6 Hz, 2H), 2.12 (s, 3H), 1.6-1.5 (m, 4H), 1.4-1.3 (m, 6H).

A solution of the alkane in 40 mL of MeOH was mixed with 190 mL of 2N HCl. The reaction mixture was refluxed for 23 hr. Then the reaction mixture was added to a cooled mixture of 190 mL 2N NaOH and 200 mL CH₂Cl₂. The aqueous phase was extracted with CH₂Cl₂ (4 x 100 mL). The CH₂Cl₂ extracts were combined, dried with MgSO₄, and evaporated to dryness, to afford 3.579 g of aniline **30** (87% from alkene): ¹H NMR (300 MHz, CDCl₃, TMS) δ 6.95 (d, J = 8.3 Hz, 2H), 6.61 (d, J = 8.3 Hz, 2H), 3.60 (t, J = 6.6 Hz, 2H), 2.48 (t, J = 7.6 Hz, 2H), 1.6-1.5 (m, 4H), 1.4-1.3 (m, 6H).
**N-(9-Acridinyl)-4-(7-hydroxyheptyl)-aniline (31).** To a solution of 636.9 mg (3.07 mmol) of 4-(7-hydroxyheptyl)-aniline **30** and 428 µL (3.07 mmol) of Et₃N in 20 mL of MeOH were added 656.4 mg (3.07 mmol) of 9-chloroacridine. After stirring for 7 hr at room temperature, the solvent was evaporated. Purification by column chromatography on silica gel with 5% MeOH in CH₂Cl₂ gave 1.079 g (91 %) of N-(9-acridinyl)-4-(7-hydroxyheptyl)-aniline **31**: ¹H NMR (300 MHz, CDCl₃, TMS) δ 8.0-7.9 (m, 4H), 7.63 (t, J = 7 Hz, 2H), 7.3-7.2 (m, 2H), 7.07 (d, J = 8.3 Hz, 2H), 6.85 (d, J = 8.3 Hz, 2H), 3.64 (t, J = 6.6 Hz, 2H), 2.57 (t, J = 7.6 Hz, 2H), 1.7-1.5 (m, 4H), 1.4-1.3 (m, 6H).
**N-(9-acridinyl)-4-(7-iodoheptyl)-aniline (32).** To a solution of 604.1 mg (1.57 mmol) of N-(9-acridinyl)-4-(7-hydroxyheptyl)-aniline **31** in 20 mL of pyridine cooled to 0°C was added 200 µL (2.58 mmol) of methanesulfonyl chloride. The reaction mixture was stirred at 0°C for 1 hr 20 min, then partitioned between 180 mL of CH₂Cl₂ and 75 mL of water. The aqueous phase was extracted with CH₂Cl₂ (3 x 30 mL). The CH₂Cl₂ extracts were combined, washed with 40 mL of saturated NaCl solution, dried with MgSO₄, and evaporated to dryness.

The sulfonate was dissolved in 20 mL of acetone. To the solution was added 355.0 mg (2.37 mmol) of NaI. and the mixture was refluxed for 8 hr, then stirred at room temperature for 16hr. The reaction mixture was partitioned between 200 mL of ethyl acetate and 100 mL of water. The organic phase was washed with 5% sodium thiosulfate (3 x 30 mL). All aqueous phases were combined and backextracted with 75 mL of ethyl acetate. Both ethyl acetate phases were combined, dried with MgSO₄, and evaporated to dryness, to afford 600.2 mg (77%) of N-(9-acridinyl)-4-(7-iodoheptyl)-aniline **32:** ¹H NMR (300 MHz, CDCl₃, TMS) δ 8.0-7.9 (m, 4H), 7.66 (t, J = 7 Hz, 2H), 7.3-7.2 (m, 2H), 7.06 (d, J = 8 Hz, 2H), 6.81 (d, J = 8 Hz, 2H), 3.18 (t, J = 7 Hz, 2H), 2.57 (t, J = 7.6 Hz, 2H), 1.9-1.8 (m, 2H), 1.7-1.7 (m, 2H), 1.4-1.3 (m, 6H).
**Quinone-anilinoacridine (33) (SL-11064).** To a solution of 1.554 g (3.14 mmol) of N-(9-acridinyl)-4-(7-iodoheptyl)-aniline **32** in a mixture of 40 mL of CHCl₃ and 2 mL of MeOH was added 1.765 g (6.28 mmol) of silver salt. The reaction mixture was refluxed for 23 hr. The reaction mixture was diluted with CH₂Cl₂, filtered, and evaporated to dryness. Purification and separation of the para- and orthoquinone isomers were accomplished using a series of columns on silica gel using 5% MeOH in CH₂Cl₂, Et₂O, and 10% MeOH in CH₂Cl₂. Isolated 108.9 mg of 33 as a dark orange solid.
**N-Acetyl-4-(7-methanesulfonyl-1-heptenyl)-aniline.** To a cooled solution of 500 mg (2.02 mmol) of N-acetyl-4-(7-hydroxy-1-heptenyl)-aniline **29** and 0.5 mL (6.18 mmol) of pyridine in 10 mL of CH₂Cl₂ was added 240 µL (3.10 mmol) of methane-sulfonyl chloride. The reaction mixture was stirred at room temperature for 22 hr. The reaction mixture was diluted with CH₂Cl₂, washed with 1N HCl (4 x 50 mL), washed with saturated NaCl solution (50 mL), dried with MgSO₄, and evaporated to dryness. Column chromatography on silica gel with 5% MeOH in CH₂Cl₂ afforded 416.1 mg (63%) of mesylate (mixture of *E* and *Z* isomers): ¹H NMR (250 MHz, CDCl₃, TMS) δ 7.47 (d, J = 8Hz), 7.43 (d, J = 8 Hz), 7.29 (d, J = 8 Hz), 7.22 (d, J = 8Hz), 6.4-6.3 (m), 6.2-6.0 (m), 5.7-5.6 (m), 4.23 (t, J = 6.6 Hz), 4.22 (t, J = 6.6 Hz), 2.4-2.3 (m), 2.3-2.1 (m), 2.18 (s), 2.17 (s), 1.9-1.7 (m), 1.6-1.4 (m).
**N-Acetyl-4-(7-iodo-1-heptenyl)-aniline (34).** To a solution of 2.641 g (8.11 mmol) of N-acetyl-4-(7-methanesulfonyl-1-heptenyl)-aniline in 60 mL of acetone was added 1.832 g (12.2 mmol) of NaI. The reaction mixture was refluxed for 19 hr. Then, filtration and evaporation of solvent gave 3.410 g (quant) of iodide **34,** which was used as is in the next reaction.
**Phosphonium iodide (35).** A solution of 3.410 g of N-acetyl-4-(7-iodo-1-heptenyl)-aniline **34** and 2.143 g (8.17 mmol) of triphenylphosphine in 70 mL of CH₃CN was refluxed for **43** hr. Evaporation of solvent and column chromatography on silica gel with 5% MeOH in CH₂Cl₂ yielded 4.781 g (95% from mesylate) of phosphonium iodide **35.**
**1-(3,4-Dimethoxy-1-naphthyl)-8-(4-acetamidophenyl)-1,7-octadiene (36).** To a cooled solution of 3.17 g (5.12 mmol) of phosphonium iodide **35** and 1.093 g (5.05 mmol) of 3,4-dimethoxy-I-naphthaldehyde **18** in 20 mL of THF and 25 mL of CH₂Cl₂ was added 130 mg (5.14 mmol) of 95% NaH. The reaction mixture was stirred at room temperature for 21 hr. The mixture was partitioned between 200 mL 1N HCl and 350 mL CH₂Cl₂. The aqueous phase was extracted with CH₂Cl₂ (6 x 75 mL). The CH₂Cl₂ extracts were combined, dried with MgSO₄, and evaporated to dryness. Column chromatography on silica gel with 1% MeOH in CH₂Cl₂ afforded 1.073 g (49%) of diene **36.**
**1-(3,4-Dimethoxy-1-naphthyl)-8-(4-acetamidophenyl)-octane.** To a solution of 556.3 mg (1.29 mmol) of 1-(3,4-dimethoxy-1-naphthyl)-8-(4-acetamidophenyl)-1,7-octadiene **36** in 20 mL of CH₂Cl₂ in a Parr bottle were added 55.4 mg of 10% Pd/C. The bottle was placed on a hydrogenation apparatus and shaken for 2.5 hr at 32 psi of hydrogen. Removal of catalyst by filtration through a celite pad and evaporation of solvent afforded 554.6 mg (99%) of octane: ¹H NMR (250 MHz, CDCl₃, TMS) δ 8.14 (d, J = 8 Hz, 1H), 7.94 (d, J = 8 Hz, 1H), 7.5-7.4 (m, 1H), 7.4-7.3 (m, 3H), 7.12 (s 1H), 7.11 (d, J = 8.2 Hz, 2H), 3.99 (s, 3H), 3.98 (s, 3H), 3.0-2.9 (m, 214), 2.6-2.5 (m, 2H), 2.16 (s, 3H), 1.8-1.3 (m 12H).
**1-(3,4-Dimethoxy-1-naphthyl)-8-(4-aminophenyl)-octane (37).** A solution of 554.6 mg (1.28 mmol) of 1-(3,4-dimethoxy-1-naphthyl)-8-(4-acetamidophenyl)-octane in 20 mL of MeOH was mixed with 21 mL of 2N HCl. The reaction mixture was refluxed for 23 hr. Then the reaction mixture was partitioned between 75 mL of CH₂Cl₂ and 21 mL of 2N NaOH. The aqueous phase was extracted with CH₂Cl₂ (5 x 40 mL). The CH₂Cl₂ extracts were combined, dried with MgSO₄, and evaporated to dryness. Column chromatography on silica gel with 1% MeOH in CH₂Cl₂ gave 374.6 mg (75%) of aniline **37**: ¹H NMR (250 MHz, CDCl₃, TMS) δ 8.14 (d, J = 8 Hz, 1H), 7.94 (d, J = 8 Hz, 1H), 7.47 (t, J = 8 Hz, 1H), 7.37 (t, J = 8 Hz, 1H), 7.12 (s, 1H), 6.96 (d, J = 8 Hz, 2H), 6.62 (d, J = 8 Hz, 2H), 3.99 (s, 3H), 3.97 (s, 3H), 3.1-3.0 (m, 2H), 2.5-2.4 (m, 2H), 1.8-1.3 (m 12H).
**Naphthylacridine (38).** To a solution of 99 mg (2.53x10⁻⁴ mol) of 1-(3,4-dimethoxy-1-naphthyl)-8-(4-aminophenyl)-octane 37 and 35 mL (2.51x10⁻⁴ mol) of Et₃N in 4 mL of MeOH were added 54 mg (2.53x10⁻⁴ mol) of 9-chloroacridine. The reaction mixture was stirred at room temperature for 20 hr. Evaporation of solvent and column chromatography on silica gel with first 1% MeOH in CH₂Cl₂ and then 3% MeOH in CH₂Cl₂ afforded 118.2 mg (82%) of acridine **38:** ¹H NMR (250 MHz, CDCl₃, TMS) δ 8.14 (d, J = 8 Hz, 1H), 8.0-7.9 (m, 5H), 7.66 (br t, 2H), 7.46 (t, J = 8 Hz, 1H), 7.37 (t, J = 8 Hz, 1H), 7.3-7.2 (m, 2H), 7.12 (s, 1H), 7.06 (d, J = 8.4 Hz, 2H), 6.82 (d, J = 8.4 Hz, 2H), 3.1-3.0 (m, 2H), 2.6-2.5 (m, 2H), 1.8-1.3 (m, 12H).
**Quinone-acridine (39) (SL-11125).** To a solution of 546 mg (9.60x10⁻⁴ mol) of acridine 38 in 15 mL of CH₂Cl₂ cooled to -68°C was added 9.6 mL of 1M BBr₃ in CH₂Cl₂. After 18.5 hr at -10°C, the reaction mixture was cooled to -68°C and 10 mL of Et₂O were added. After stirring at room temperature for 30 min, 20 mL of saturated NaHCO₃ solution were added. The resulting precipitate was collected by filtration and triturated twice with 50 mL of CH₂Cl₂ to give 555.9 mg of quinone **39**: ¹H NMR (250 MHz, DMSO-d₆, TMS) δ 9.11 (s), 8.59 (s), 8.14 (d, J = 9 Hz), 8.0-7.9 (m), 7.82 (d, J = 8 Hz), 7.4-7.2 (m), 6.98 (s), 2.87 (t, J = 7 Hz), 2.65 (t, J = 7 Hz), 1.7-1.5 (m), 1.4-1.3 (m).
**N-(9-acridyl)-mesitylenesulfonamide (41).** To a suspension of 4.00 g (20.6 mmol) of 9-aminoacridine **40** in 350 mL of CHCl₃ was added 2.9 mL (20.8 mmol) of Et₃N and 4.50 g (20.6 mmol) of mesitylenesulfonyl chloride. The reaction mixture was refluxed for 72 hr. Then the reaction mixture was filtered and the solvent was evaporated. The material was purified by column chromatography on silica gel by eluting first with 1% MeOH in CH₂Cl₂ and then with 5% MeOH in CH₂Cl₂ to yield 458.4 mg (6%) of sulfonamide 41 as an orange solid: ¹H NMR (300 MHz, CDCl₃, TMS) δ 9.25 (s, 1H), 8.77 (d, J = 8 Hz, 2H), 7.46 (t, J = 8 Hz, 2H), 7.21 (d, J = 8 Hz, 2H), 7.15 (t, J = 8 Hz, 2H), 7.02 (s, 2H), 2.78 (s, 6H), 2.36 (s, 3H).
**N-(9-acridyl)-N-(5-bromapentyl)-mesitylenesulfonamide (42).** A solution of 450 mg (1.20 mmol) of N-(9-acridyl)-mesitylenesulfonamide in 20 mL of DMF was placed under an atmosphere of argon and cooled to 0°C. To the cooled solution was added 36 mg (1.42 mmol) ofNaH (95%). The reaction mixture was stirred at 0°C for 5 min and at room temperature for 1 hr. Then the reaction mixture was cooled to 0°C, and 1.65 mL (12.1 mmol) of 1,5-dibromopentane were added. The reaction mixture was stirred at 70-80°C for 23 hr. The reaction mixture was cooled, and quenched with 20 mL of water. The mixture was partitioned between CH₂Cl₂ and water. The aqueous phase was washed with CH₂Cl₂ (2 x 20 mL). The CH₂Cl₂ washes were combined with the organic phase, dried with MgSO₄, and evaporated to dryness. The material was purified by column chromatography on silica gel with CH₂Cl₂ to afford 382.2 mg (60%) of bromide **42** as an orange oil: ¹H NMR (300 MHz, CDCl₃, TMS) δ 8.25 (d, J = 9 Hz, 2H), 7.94 (d, J = 9 Hz, 2H), 7.76 (t, J = 8 Hz, 2H), 7.45 (t, J = 8 Hz, 2H), 6.87 (s, 2H), 4.0-3.9 (m, 2H), 3.27 (t, J = 6.5 Hz, 2H), 2.30 (s, 3H), 2.22 (s, 6H), 1.8-1.6 (m, 4H), 1.4-1.3 (m, 2H).
**Mesityl-acridine-quinone (43).** To a solution of 632.6 mg (1.20 mmol) ofN-(9-acridyl)-N-(5-bromopentyl)-mesitylenesulfonamide **42** in 15 mL of benzene was added 338.4 mg (1.20 mmol) of silver salt. The reaction mixture was refluxed for 24 hr. The reaction mixture was diluted with CH₂Cl₂ and filtered to remove insoluble salts. The solvent was removed and the material was purified by column chromatography on silica gel with Et₂O to afford 333.1 mg (45%) of ortho-quinone **43** as an orange glassy solid: ¹H NMR (300 MHz, CDCl₃, TMS) δ 8.24 (d, J = 9 Hz, 2H), 8.11 (d, J = 8 Hz, 1H), 7.95 (d, J = 9 Hz, 2H), 7.8-7.7 (m, 3H), 7.7-7.5 (m, 2H), 7.5-7.4 (m, 2H), 6.86 (s, 2H), 5.85 (s, 1H), 4.1-4.0 (m, 4H), 2.29 (s, 3H), 2.21 (s, 6H), 1.9-1.5 (m, 4H), 1.5-1.4 (m, 2H).
**Acridine-quinone (44) (SL-11059).** Under an atmosphere of argon, 151.4 mg (2.45x10⁻⁴ mol) of mesityl-acridine-quinone **43** was dissolved in 30 mL of 0.1M SmI₂ in THF. Then, 2.2 mL (18.2 mmol) of DMPU were added dropwise. The reaction mixture was refluxed for 24 hr. Filtration to remove a precipitate and evaporation of solvent yielded an orange oil, which was purified by column chromatography on silica gel with 5% MeOH in CH₂Cl₂ to afford 48.7 mg (45%) of acridine-quinone **44** as an orange glassy solid: ¹H NMR (300 MHz, DMSO-d₆, TMS) δ 8.54 (d, J = 8 Hz, 2H), 7.96 (t, J = 7 Hz, 2H), 7.92 (d, J = 7 Hz, 1H), 7.79 (d, J = 8 Hz, 2H), 7.7-7.6 (m, 3H), 7.51 (t, J = 8 Hz, 2H), 6.01 (s, 1H), 4.20 (t, J = 6 Hz, 2H), 4.13 (t, J = 7 Hz, 2H), 2.1-1.9 (m, 4H), 1.7-1.6 (m, 2H).

### Synthesis of quinol phosphates: General Procedure

To a solution of 500 mg (2.05 mmol) of 4-pentyloxy-1,2-naphthoquinone **46** in 10 mL of benzene was added 2.3 mL (25.1 mmol) of dibenzylphosphite. The reaction mixture was refluxed under nitrogen for 2.5 hr, after which the benzene was removed. Column chromatography of the residue on silica gel with 1% MeOH in CH₂Cl₂ afforded 729.3 mg (70%) of aryldibenzylphosphate **47** (mixture of two regioisomers) as an orange oil: *R*_{*f*} = 0.51, 0.66 (1% MeOH in CH₂Cl₂); ¹H NMR (250 MHz, CDCl₃, TMS) major regioisomer δ 8.1 (d), 8.0 (br, s), 7.8 (d), 7.4 (t), 7.3-7.1 (m), 6.50 (s), 5.3-5.0 (AB of ABX, δ_{A} = 5.16, δ_{B} = 5.08, J_{AB} = 11.5 Hz, J_{AX} = 8.3 Hz, J_{BX} = 8.8 Hz), 4.01 (t, J = 6 Hz), 2.0-1.8 (m), 1.6-1.3 (m), 0.96 (t, J = 7 Hz); ¹³C NMR (52 MHz, CDCl₃, TMS) both regioisomers δ 153.4, 144.7, 135.6 (d, J = 6.1 Hz, minor regioisomer), 134.8 (d, J = 5.5 Hz, major regioisomer), 128.7-127.7 (m), 127.2, 123.0, 122.2, 121.4, 119.8, 99.5, 71.0 (q, J = 4.8 Hz), 68.3, 28.8, 22.5.

To a solution of 1.637 g (3.23 mmol) of aryldibenzylphosphate **47** in 40 mL of MeOH was added 150 mg of 10% Pd/C. The reaction mixture was placed under an atmosphere of hydrogen (balloon) and stirred at room temperature for 1 hr. Removal of catalyst by filtration and evaporation of solvent afforded phosphate as a brown oil. The phosphate was dissolved in 6 mL of benzene. Addition of 9 mL of hexane and cooling gave a precipitate. The precipitate was collected by filtration, washed with benzene/hexane = 2:3, and dried, affording 797.3 mg (76%) of arylphosphate **48** as a gray solid; *R*_{*f*} = 0.77 (MeOH); ¹H NMR (250 MHz, acetone-d₆, TMS) δ 8.13 (d, J = 8 Hz, 1H), 7.96 (d, J = 8Hz, 1H), 7.49 (t, J = 7 Hz, I H), 7.32 (t, J = 7 Hz, 1H), 6.59 (s, 1H), 4.13 (t, J = 6Hz, 1 H), 2.0-1.8 (m, 2H), 1.6-1.3 (m, 4H), 0.96 (t, J = 7 Hz, 3H); ¹³C NMR (52 MHz, acetone-d₆, TMS) δ 153.3 (d, J = 1.3 Hz), 145.8 (narrow t), 129.3 (d, J = 3.3 Hz), 127.4, 123.2, 122.2, 121.6, 120.9, 100.0, 68.7, 29.2, 28.7, 22.7, 13.9.
**Ethyl 2'-acetyl-5'-methoxyphenylacetate (50)** Acetyl chloride (21.3 mL, 300 mmol) was added to a mixture of AlCl₃ (26.7 g, 200 mmol) and ethyl 3'-methoxyphenylacetate (**49,** 28.66 g, 147.6 mmol) in CS₂ (200 mL) at 0 °C. The ice bath was removed and the mixture was allowed to warm to 20 °C with HCl gas bubbling out. After stirring at 20 °C for 30 min, the mixture was refluxed for 30 min. Upon cooling down, the mixture was added ice (200 g) and aqueous 2 N HCl (400 mL). The resulting mixture was extracted with ethyl acetate (2 x 200 mL). The extracts were washed with water (2 x 100 mL), dried over MgSO₄ and concentrated in *vacuo.* The residue was crystallized from a mixture of ethyl acetate (20 mL) and hexanes (60 mL) to afford **50** (30.60 g, 88%): ¹H NMR (CDCl₃) δ 7.84 (1 H, d, *J* = 8.6 Hz), 6.86 (1 H, dd, *J =* 8.6, 2.6 Hz), 6.75 (1H, d, *J* = 2.6 Hz), 4.17 (2H, q, *J* = 7.1 Hz), 3.92 (2H, s), 3.86 (3H, s), 2.55 (3H, s), 1.28 (3H, t, *J* = 7.1 Hz); ¹³C NMR (CDCl₃) δ 199.04 (s), 171.44 (s), 162.22 (s), 137.70 (s), 132.97 (d), 129.48 (s), 118.68 (d), 111.84 (d), 60.60 (t), 55.39 (q), 41.17 (t), 28.39 (q), 14.24 (q).
**2-Hydroxy-7-methoxy-1,4-naphthoquinone(51).** Sodium ethoxide (10.40 g, 150 mmol) was added to a suspension of **50** (30. 45 g, 128.90 mmol) in absolute alcohol (200 mL) at 20 °C. After stirring the mixture for 1 h, air was bubbled in for 20 h. The mixture was concentrated *in vacuo*. The residue was dissolved in water (500 mL), and extracted with diethyl ether (200 mL). The ether layer was counter-extracted with water (50 mL). The combined aqueous phase was acidified with concentrated HCl (30 mL). The mixture was filtered to afford **51** (14.42 g, 55%): ¹H NMR (DMSO-d6) δ 11.56 (1H, s, br), 7.89 (1H, d, *J* = 8.5 Hz), 7.42 (1H, d, *J =* 2.8 Hz), 7.36 (1H, dd, *J* = 8.5, 2.8 Hz), 6.10 (1H, s), 3.92 (3H, s); ¹³C NMR (DMSO-d6) δ.184.07 (s), 181.20 (s), 162.92 (s), 159.16 (s), 132.35 (s), 127.82 (d), 125.16 (s), 120.02 (d), 110.85 (s), 109.94 (d), 55.90 (q).
**7- Methoxy-lapachol (52).** A mixture of K₂CO₃ (30 mmol) and **51** (10.21 g, 50 mmol) in HMPA (100 mL) was stirred for 30 min, when it became a suspension. Dimethylallyl bromide (8.7 mL, 75 mmol) and KI (4.15 g, 25 mmol) were added, and stirring was continued for 20 h at 20 °C. The mixture was diluted with ice water (600 mL) and concentrated HCl (30 mL), and extracted with ethyl acetate (2 x 200 mL). Some solid was collected by filtration to afford the first portion of **53** (0.628 g): ¹H NMR (CDCl₃) δ 8.01 (1H, d, *J* = 8.6 Hz), 7.56 (1H, d, *J* = 2.7 Hz), 7.20 (1H, dd, *J* = 8.6, 2.7 Hz), 6.09 (1H, s), 5.49 (1H, t, *J* = 6.8 Hz), 4.57 (2H, d, *J* = 6.8 Hz), 3.94 (3H, s), 1.81 (3H, s), 1.76 (3H, S). The ethyl acetate extracts were pooled, extracted with saturated NaHCO₃ (2 x 150 mL), and the resultant aqueous extracts were acidified with concentrated HCl and filtered to recover **51** (2.10 g, 21%).

The main ethyl acetate extract was concentrated *in vacuo.* The residue was dissolved in a mixture of 1 N NaOH (500 mL) and diethyl ether (300 mL). After separation, the organic layer was extracted with 1 N NaOH (100 mL) and concentrated *in vacuo.* The residue was chromatographed on silica gel (10% ethyl acetate in hexanes) to afford a second portion of **53** (3.43 g, 30% total).

The NaOH extracts were acidified by concentrated HCl (50 mL), and extracted with ethyl acetate (2 x 200 mL). The pooled extracts were dried (MgSO₄), concentrated *in vacuo,* and the residue was purified by chromatography on silica gel (10% ethyl acetate in hexanes) to afford **52** (4.39 g, 32%): ¹H NMR (CDCl₃) δ 8.05 (1H, d, *J* = 8.6 Hz), 7.51 (1H, d, *J* = 2.7 Hz), 7.20 (1H, dd, *J* = 8.6, 2.7 Hz), 7.18 (OH, s), 5.20 (1H, tt, *J* = 6.7, 1.5 Hz), 3.93 (3H, s), 3.29 (2H, d, *J* = 7.2 Hz), 1.79 (3H, s), 1.68 (3H, s); ¹³C NMR (CDCl₃) δ 183.99 (s), 181.85 (s), 163.28 (s), 152.51 (s), 133.71 (s), 131.18 (s), 129.04 (d), 126.23 (s), 123.28 (s), 120.69 (d), 119.82 (d), 109.82 (d), 55.89 (q), 25.77 (q), 22.60 (t), 17.90 (q).
**8-Methoxy-β-lapachone (54)** Concentrated H₂SO₄ (25 mL) was added to compound **52** (2.454 g) at 20 °C. After stirring for 20 min, the mixture was diluted with ice water (500 mL). The resulting red precipitate **54** was collected by filtration, washed with water, and dried *in vacuo*. It was obtained as a red powder (2.36 g, 96%): ¹H NMR (CDCl₃) δ 7.72 (1H, d, *J* = 8.6 Hz), 7.56 (1H, d, *J* = 2.7 Hz), 7.12 (1H, dd, *J =* 8.6, 2.7 Hz), 3.90 (3H, S), 2.55 (2H, t, *J* = 6.7 Hz), 1.84 (2H, t, *J* = 6.7 Hz), 1.46 (6H, S).
**8-Hydroxy-β-lapachone (55)** Boron tribromide (15.0 mL, 1.0 M in CH₂Cl₂) was added to a solution of **54** (1.05 g, mmol) in anhydrous CH₂Cl₂ (40 mL) at 0 °C. After stirring for 15 min, the mixture was allowed to warm to 20 °C and kept stirring for 2 h. Ice water (500 mL) was added, the mixture was extracted with CHCl₃ (3 x 100 mL), the combined extracts were dried, and concentrated *in vacuo.* The residue was treated with concentrated H₂SO₄ (20 mL) at 20 °C. The mixture was diluted with ice water (500 mL) and extracted with CHCl₃ (3 x 100 mL). The combined extracts were reextracted with aqueous 5% NaHSO₃ (3 x 150 mL). The aqueous extracts were acidified with concentrated HCl (100 mL), and extracted with CHCl₃ (3 x 150 mL). The extracts were dried and concentrated to afford **55** (270 mg, 27%): ¹H NMR (CDCl₃) δ 9.81 (OH, s), 7.64 (1H, d, *J* = 8.5 Hz), 7.49 (1H, d, *J* = 2.6 Hz), 7.06 (1H, dd, *J =* 8.5, 2.6 Hz), 2.51 (2H, t, *J* = 6.6 Hz), 1.84 (2H, t, *J* = 6.6 Hz), 1.45 (6H, s); HRMS (*m*/*z*) calcd for C₁₅H₁₄O₄ 258.0892, found 258.0885.

### Preparation of 1,2-Naphthoquinone bisulfite adducts

**General Procedure I.** The quinone was dissolved in 10% NaHSO₃. After standing for several hours at room temperature or with cooling, the quinone-bisulfite adduct precipitated. The quinone-bisulfite was collected by filtration and dried. The quinone-bisulfite was stablized with addition of 300 % its weight of sodium bisulfite.

**General Procedure II.** The quinone is dissolved in 10% NaHSO₃ in a volume of solution such that there is no more than 300% weight excess of NaHSO₃ (relative to quinone-bisulfite). When the quinone-bisulfite did not precipitate, it was recovered from the solution by evaporation of the water in vacuo. This procedure gives a quinone-bisulfite adduct with a 300% weight excess NaHSO₃.

### Synthesis of morpholino-Ser-Lys-Leu-Gln-β-Ala-β-Lapachone (Scheme 13) Boc-Gln-β-Ala-β-Lapachone

To a solution of 1.000 g (2.437 mmol) of β-Ala-β-Lapachone-TFA salt (SL-11006) and 600.3 mg (2.437 mmol) of Boc-Gln in 10 mL of DMF was added 395.3 mg (2.925 mmol) of 1-hydroxybenzotriazole: The mixture was cooled in an ice bath. Then 270 µL (2.456 mmol) of N-methylmorpholine were added, followed by 553.0 mg (2.680 mmol) of DCC. The reaction mixture was stirred in the ice bath for 30 min and at room temperature for 6.5 hr. The reaction mixture was then diluted with CH₂Cl₂ and filtered. The filtrate was washed with saturated NaHCO₃ (50 mL), with 5% citric acid (3 x 50 mL), with saturated NaHCO₃ (2 x 50 mL), with saturated NaCl (50 mL), dried with MgSO₄, and evaporated to dryness. Purification by column chromatography on silica gel with 5% MeOH in CH₂Cl₂ afforded 692.7 mg (51%) of peptide as an orange glassy solid: *R*_{*f*} = 0.11 (5% MeOH in CH₂Cl₂); ¹H NMR (250 MHz, acetone-d₆, TMS) δ 8.00 (dd, J = 7.6, 1.3 Hz, 1H), 7.9-7.7 (m, 2H), 7.64 (td, J = 7.6, 1.3 Hz, 1H), 7.5-7.4 (br d, NH), 6.9 (br s, NH), 6.2 (br s, NH), 5.2-5.1 (m, 1H), 4.1-4.0 (m, 1H), 3.5-3.4 (m, 2H), 2.7-2.5, (m, 4H), 2.3-2.2 (m, 2H), 2.0-1.8 (m, 2H), 1.53 (s, 3H), 1.51 (s, 3H), 1.39 (s, 9H); ¹³C NMR (52 MHz, acetone-d₆, TMS) δ 179.8, 178.8, 175.0, 172.5, 171.6, 160.8, 156.2, 111.1, 135.6, 133.0, 131.6, 131.2, 128.7, 124.8, 80.8, 80.3, 79.2, 70.2, 54.8, 35.6, 34.7, 32.1, 28.4, 24.8, 23.2, 23.1.

### Gln-β-Ala-β-Lapachone

To a solution of 681.9 mg (1.223 mmol) of Boc-Gln-β-Ala-β-Lapachone in 10 mL ofCH₂Cl₂ was added 10 mL of TFA. The reaction mixture was stirred at room temperature for 25-30 min. The solvent was removed *in vacuo.* Column chromatography on silica gel with 10-20% MeOH in CH₂Cl₂ afforded 578.5 mg (83%) of the TFA salt as an orange glassy solid: *R*_{*f*} = 0.55 (BuOH/H₂O/AcOH = 5:3:2), 0.05 (10% MeOH in CH₂Cl₂), 0.24 (5% MeOH in CH₂Cl₂).

### Boc-Leu-Gln-β-Ala-β-Lapachone

To a solution of 650.2 mg (1.138 mmol) of Gln-β-Ala-β-Lapachone-TFA salt and 263.0 mg (1.138 mmol) of Boc-Leu in 4.6 mL of DMF was added 184.5 mg (1.365 mmol) of 1-hydroxybenzotriazole. The mixture was cooled in an ice bath. Then 130 µL (1.182 mmol) of N-methylmorpholine were added, followed by 258.4 mg (1.252 mmol) of DCC. The reaction mixture was stirred in the ice bath for 30 min and at room temperature for 6.5 hr. The reaction mixture was then diluted with CH₂Cl₂ and filtered. The filtrate was washed with saturated NaHCO₃ (30 mL), with 5% citric acid (4 x 30 mL), with saturated NaHCO₃ (3 x 30 mL), with saturated NaCl (30 mL), dried with MgSO₄, and evaporated to dryness. Purification by column chromatography on silica gel with 5% MeOH in CH₂Cl₂ afforded 396.9 mg (51%) of peptide as a yellow-orange glassy solid: *R*_{*f*} = 0.1 (5% MeOH in CH₂Cl₂), 0.45 (10% MeOH in CH₂Cl₂), 0.81 (20% MeOH in CH₂Cl₂), 0.78 (BuOH/H₂O/AcOH = 5:3:2); ¹H NMR (250 MHz, acetone-d₆, TMS) δ 8.00 (d, J = 7.5 Hz, 1H), 7.9-7.7 (m, 2H), 7.64 (t, J = 7.5 Hz, 1H), 7.5 (br d, NH), 6.9 (br s, NH), 6.3 (br s, NH), 5.2-5.1 (m, 1H), 4.4-4.2 (m, 1H), 4.1-4.0 (m, 1H), 3.6-3.3 (m, 2H), 2.7-2.5 (m, 4H), 2.3-2.2 (m, 2H), 2.0-1.8 (m, 2H), 1.8-1.7 (m, 1H), 1.6-1.5 (m, 2H), 1.53 (s, 3H), 1.51 (s, 3H), 1.39 (s, 9H), 1.0-0.9 (m, 6H); ¹³C NMR (52 MHz, acetone-d₆, TMS) δ 179.9, 179.0, 175.2, 173.4, 172.0, 171.5, 160.9, 156.8, 135.7, 133.1, 131.6, 131.2, 128.8, 124.9, 111.2, 80.9, 80.4, 79.5, 70.3, 54.5, 53.5, 41.7, 35.8, 34.8, 32.1, 28.5, 27.8, 25.4, 24.9, 23.4, 23.2, 21.9.

### Leu-Gln-β-Ala-β-Lapachone

To a solution of 317.0 mg (4.725x10⁻⁴ mol) of Boc-Leu-Gln-β-Ala-β-Lapachone in 4 mL of CH₂Cl₂ was added 4 mL of TFA. The reaction mixture was stirred at room temperature for 25-30 min. The solvent was removed *in vacuo.* Column chromatography on silica gel with 20% MeOH in CH₂Cl₂ afforded 277.3 mg (86%) of the TFA salt as an orange glassy solid: *R*_{*f*} = 0.17 (10% MeOH in CH₂Cl₂), 0.39 (20% MeOH in CH₂Cl₂), 0.74 (BuOH/H₂O/AcOH = 5:3:2).

### Nα-Boc-Lys(Nε-Cbz)-Leu-Gln-β-Ala-β-Lapachone

To a solution of 277.3 mg (4.050x10⁻⁴ mol) of Leu-Gln-β-Ala-β-Lapachone-TFA salt and 168.0 mg (4.049x10⁻⁴ mol) of Nα-Boc-Lys(Nε-Cbz) in 1.6 mL of DMF was added 65.7 mg (4.862x10⁻⁴ mol) of 1-hydroxybenzotriazole. The mixture was cooled in an ice bath. Then 50 µL (4.548x10⁻⁴ mol) of N-methylmorpholine were added, followed by 91.9 mg (4.454x10⁻⁴ mol) of DCC. The reaction mixture was stirred in the ice bath for 30 min and at room temperature for 6.5 hr. The reaction mixture was then diluted with 2 mL of CHCl₃ and filtered. The filtrate was washed with saturated NaHCO₃ (20 mL), with 5% citric acid (4 x 20 mL), with saturated NaHCO₃ (3 x 20 mL), with saturated NaCl (2 x 20 mL), dried with MgSO₄, and evaporated to dryness. Purification by column chromatography on silica gel with 10% MeOH in CH₂Cl₂ afforded 167.5 mg (42%) of peptide as an orange glassy solid: *R*_{*f*} = 0.08 (5% MeOH in CH₂Cl₂), 0.44 (10% MeOH in CH₂Cl₂); ¹H NMR (250 MHz, DMSO-d₆, TMS) δ 8.0-7.7 (m, 6H, quinone-H5, H6, H7, H8, & NH's), 7.7-7.6 (m, NH), 7.5-7.4 (m, 2H, Cl-Cbz), 7.4-7.3 (m, 2H, Cl-Cbz), 7.20 (br s, NH), 6.73 (br s, NH), 6.90 (br d, J = 7.9 Hz, NH), 5.07 (s, 3H), 4.3-4.2 (m, 1H), 4.2-4.1 (m, 1H), 3.9-3.8 (m, 1H), 3.3-3.2 (m, 2H), 3.0-2.9 (m, 2H), 2.8-2.7 (m, 2H), 2.6-2.4 (m, 2H), 2.1-2.0 (m, 2H), 1.8-1.3 (m, 11H), 1.43 (s, 3H), 1.39 (s, 3H), 1.36 (s, 9H), 0.85 (d, J = 6.5 Hz, 3H), 0.81 (d, J = 6.6 Hz, 3H); ¹³C NMR (52 MHz, DMSO-d₆, TMS) δ 178.6, 177.8, 173.5, 173.4, 172.0, 171.7, 171.0, 170.5, 162.2, 155.7, 134.9, 134.5, 132.2, 131.4, 130.9, 129.9, 129.5, 129.2, 127.9, 127.2, 123.7, 79.7, 79.3, 78.0, 68.9, 62.4, 54.2, 52.0, 50.8, 40.7, 35.7, 33.5, 31.2, 30.7, 29.0, 28.1, 27.8, 24.1, 23.9, 23.0, 22.8, 22.7. 22.1, 21.4.

### Lys(Nε-Cbz)-Leu-Gln-β-Ala-β-Lapachone

To a suspension of 203.1 mg (2.099x10⁻⁴ mol) of Boc-Lys(Nε-Cbz)-Leu-Gln-β-Ala-β-Lapachone in 2 mL of CHCl₃ was added 1.7 mL of TFA (material dissolved). The reaction mixture was stirred at room temperature for 20-25 min. The solvent was removed *in vacuo.* Column chromatography on silica gel with 20% MeOH in CH₂Cl₂ afforded 202.0 mg (98%) of the TFA salt as an orange glassy solid: *R*_{*f*} = 0.10 (10% MeOH in CH₂Cl₂), 0.40 (20% MeOH in CH₂Cl₂).

### Morpholino-Ser(OBn)-Lys(Nε-Cbz)-Leu-Gln-β-Ala-β-Lapachone

To a solution of 194.8 mg (1.985x10⁻⁴ mol) of Lys(Nε-Cbz)-Leu-Gln-β-Ala-β-Lapachone-TFA salt and 61.2 mg (1.985x10⁻⁴ mol) of morpholino-Ser(OBn) in 1.0 mL of DMF was added 32.2 mg (2.383x10⁻⁴ mol) of 1-hydroxybenzotriazole. The mixture was cooled in an ice bath. Then 23 µL (2.092x10⁻⁴ mol) of N-methylmorpholine were added, followed by 45. I mg (2.186x10⁻⁴ mol) of DCC. The reaction mixture was stirred in the ice bath for 35 min and at room temperature for 6 hr. The reaction mixture was then diluted with 2 mL of CH₂Cl₂ and filtered. The filtrate was washed with 5% citric acid (3 x 20 mL), with saturated NaHCO₃ (3 x 20 mL), with saturated NaCl (20 mL), dried with MgSO₄, and evaporated to dryness. Purification by column chromatography on silica gel with 10% MeOH in CH₂Cl₂ afforded 83.3 mg (36%) of peptide as an orange glassy solid: *R*_{*f*} = 0.05 (5% MeOH in CH₂Cl₂), 0.41 (10% MeOH in CH₂Cl₂); ¹H NMR (250 MHz, acetone-d₆, TMS) δ 8.0-7.7 (m, 7H, quinone-H5, H6, H7, H8, NH's), 7.7-7.6 (m, NH), 7.5-7.2 (m, 10H, Cl-Cbz, OBn, NH), 6.75 (br s, NH), 6.60 (br d, J = 7.1 Hz, NH), 5.07 (s, 3H), 4.49 (s, 2H), 4.4-4.3 (m, 1H), 4.3-4.0 (m, 3H), 3.7-3.6 (m, 2H), 3.6-3.5 (m, 4H), 3.3-3.2 (m, 6H), 3.0-2.9 (m, 2H), 2.8-2.7 (m,2H), 2.5-2.4 (m, 2H), 2.1-2.0 (m, 2H), 1.8-1.3 (m, 11H), 1.43 (s, 3H), 1.38 (s, 3H), 0.82 (d, J = 6.0 Hz, 3H), 0.78 (d, J = 6.1 Hz, 3H).

### Morpholino-Ser-Lys-Leu-Gln-β-Ala-β-Lapachone (SL-11147)

To a solution of 78.3 mg (6.763x10⁻⁵ mol) of morpholino-Ser(OBn)-Lys(Nε-Cbz)-Leu-Gln-β-Ala-β-Lapachone in 1.5 mL of MeOH/CH₂Cl₂ = 1:9 was added 30.6 mg 10% Pd/C. Then 0.5 mL of MeOH and one drop of HCl were added. The reaction mixture was placed under an atmosphere of H₂ (balloon) and stirred at room temperature for 16 hr. Removal of catalyst by filtration and evaporation of solvent afforded 64.5 mg of crude quinone-tetrapeptide. The material was purified by prep HPLC to yield 14.4 mg (24%): *R*_{*f*} = 0.04 (20% MeOH in CH₂Cl₂).

### N-Fmoc-Ser(OBn) t-butyl ester

Isobutylene was condensed into a 500 mL pressure bottle until the volume was between 30 and 40 mL. A solution of 3.02 g (7.23 mmol) of N-Fmoc-Ser(OBn) in 20 mL of THF was added, followed by 2 mL of concentrated H₂SO₄. The bottle was securely stoppered and shaken at room temperature for 24 hr. The reaction mixture was poured into an ice-cold mixture of 150 mL of ethyl acetate and 150 mL of saturated NaHCO₃. The organic phase was washed with water (3 x 50 mL) and dried with MgSO₄. The solvent was removed, and column chromatography on silica gel with CH₂Cl₂ afforded 2.453 g (72%) of t-butyl ester as a colorless oil: ¹H NMR (250 MHz, acetone-d₆, TMS) δ 7.85 (d, J = 7.5 Hz, 2H), 7.74 (d, J = 7.3 Hz, 2H), 7.5-7.3 (m, 9H), 6.71 (br d, J = 8.6 Hz, NH), 4.55 (ABq, δ_{A} = 4.57, δ_{B} = 4.52, J_{AB} = 12 Hz, 2H), 4.4-4.2 (m, 4H), 3.9-3.7 (AB of ABX, δ_{A} = 3.89, δ_{B} = 3.75, J_{AB} = 9.5 Hz, J_{AX} = 4.6 Hz, J_{BX} = 3.6 Hz, 2H); ¹³C NMR (52 MHz, acetone-d₆, TMS) δ 170.0, 156.8, 145.0, 144.9, 142.0, 129.0, 128.4, 128.3, 128.2, 127.8, 126.1, 120.7, 81.9, 73.6, 70.9, 67.3, 55.9, 47.9, 28.1.

### Ser(OBn) t-butyl ester

To a solution of 3.049 g (6.44 mmol) of N-Fmoc-Ser(OBn) t-butyl ester in 50 mL of CH₂CL₂ was added 3 mL of piperidine. The reaction mixture was stirred at room temperature for 2.3 hr. Removal of solvent and column chromatography on silica gel with 5% MeOH in CH₂Cl₂ yielded 1.306 g (81 %) of Ser(OBn) t-butyl ester as a colorless oil: *R*_{*f*} = 0.12 (2% MeOH in CH₂Cl₂); ¹H NMR (250 MHz, acetone-d₆, TMS) δ 7.4-7.2 (m, 5H), 4.53 (Abq, δ_{A} = 4.55, δ_{B} = 4.52, J_{AB} = 12 Hz, 2H), 3.7-3.6 (m, AB of ABX, δ_{A} = 3.68, δ_{B} = 3.61, J_{AB} = 12 Hz, J_{AX} = 4.9 Hz, J_{BX} = 4.4 Hz, 2H), 3.5-3.4 (m, X of ABX, δ_{X} = 3.45, 1H), 1.43 (s, 9H) ; ¹³C NMR (52 MHz, acetone-d₆, TMS) δ 173.9, 139.5, 128.9, 128.2, 128.1, 80.7, 73.8, 73.5, 56.2, 28.1.

### Morpholino-Ser(OBn) t-butyl ester

To a solution of 140.6 mg (5.59x10⁻⁴ mol) of Ser(OBn) t-butyl ester in 4 mL of pyridine was added 66 µL (5.66x10⁻⁴ mol) of 4-morpholinecarbonyl chloride. After stirring for 1 hr, the reaction mixture was partitioned between 75 mL of CH₂Cl₂ and 60 mL of water. The organic phase was washed with saturated NaHCO₃ (50 mL), with 1N HCl (2 x 50 mL), with saturated NaCl (50 mL), dried with MgSO₄, and evaporated to dryness. The crude amide was purified by column chromatography on silica gel with ethyl acetate to yield 80.9 mg (40%) of amide as a light orange oil: *R*_{*f*} = 0.58 (ethyl acetate), 0.60 (5% MeOH in CH₂Cl₂); ¹H NMR (250 MHz, acetone-d₆, TMS) δ 7.4-7.2 (m, 5H), 5.8 (br d, NH), 4.53 (Abq, δ_{A} = 4.55, δ_{B} = 4.52, J_{AB} = 12 Hz, 2H), 4.5-4.4 (m, X of ABX, δ_{X} = 4.47, 1H), 3.9-3.6 (m, AB of ABX, δ_{A} = 3.86, δ_{B} = 3.69, J_{AB} = 9.4 Hz, J_{AX} = 4.4 Hz, J_{BX} = 3.7 Hz, 2H), 3.63-3.58 (m, 4H), 3.4-3.3 (m, 4H), 1.44 (s, 9H) ; ¹³C NMR (52 MHz, acetone-d₆, TMS) δ 170.9, 157.9, 139.2, 129.0, 128.3, 128.2, 81.5, 73.5, 71.3, 67.0, 55.5, 44.9, 28.1.

### Morpholino-Ser(OBn)

A solution of 80 mg (2.195x10⁻⁴ mol) of morpholino-Ser(OBn) t-butyl ester in a mixture of 1.5 mL of CH₂Cl₂ and 1.5 mL of TFA was stirred at room temperature for 30 min. The solvent was removed *in vacuo* and the remaining TFA was removed by repeated evaporation with acetone. The residue was triturated with Et₂O. The material was then filtered, washed with Et₂O, washed with 0.5 mL acetone, washed again with Et₂O, and dried to yield 41.8 mg (62%) of amino acid as an off-white solid: *R*_{*f*} = 0.72 (BuOH/H₂O/AcOH = 5:3:2); ¹H NMR (250 MHz, acetone-d₆, TMS) δ 7.4-7.3 (m, 5H), 6.0-5.9 (br d, NH), 4.6-4.5 (m, 3H, OCH₂Ph & X of ABX), 3.95-3.75 (m, AB of ABX, δ_{A} = 3.90, δ_{B} = 3.73, J_{AB} = 9.6 Hz, J_{AX} = 4.9 Hz, J_{BX} = 3.9 Hz, 2H), 3.6-3.5 (m, 4H), 3.4-3.3 (m, 4H); ¹³C NMR (52 MHz, DMSO-d₆, TMS) d 172.4, 157.2, 138.2, 128.2, 127.4, 127.4, 72.0, 69.5, 65.9, 53.8, 43.9.

### Synthesis of Morpholino-Ser-Lys-Leu-Gln-Leu-β-Lapachone (Scheme 14)

### Boc-Leu-β-Lapachone

A solution of 2.820 g (12.20 mmol) of Boc-Leu and 1.976 g (12.19 mmol) of 1,1-carbonyldiimidazole in 33 mL of DMF was stirred at room temperature for 20 min. To the solution was added 2.100 g (8.130 mmol) of 3-hydroxy-β-lapachone followed by 1.6 mL (10.70 mmol) of DBU. After stirring at room temperature for 5 hr, the reaction mixture was partitioned between 200 mL of water and 200 mL of CHCl₃. The aqueous phase was washed with CHCl₃ (4 x 50 mL). The CHCl₃ extracts were combined, dried with MgSO₄, and evaporated to dryness. Column chromatography on silica gel with 2% MeOH in CH₂Cl₂ afforded 2.038 g (53%) of quinone as an orange glassy solid (and mixture of two diastereomers): *R*_{*f*} = 0.45 (5% MeOH in CH₂Cl₂); ¹H NMR (250 MHz, acetone-d₆, TMS) δ 8.1-8.0 (m, 1H), 8.0-7.9 (m, 1H), 7.9-7.8 (m, 1H), 7.7-7.6 (m, 1H), 6.34 (br d, NH), 5.2-5.1 (m, 1H), 4.2-4.1 (m, 1H), 2.9-2.8 (m, 1H), 2.7-2.5 (m, 1H), 1.8-1.6 (m, 3H), 1.56 (s, 1.5H), 1.53 (s, 3H), 1.52 (s, 1.5H), 1.34 (s, 4.5H), 1.33 (s, 4.5H), 0.91 (d, J = 7.0 Hz, 1.5H), 0.88 (d, J = 6.7 Hz, 1.5H), 0.84 (d, J = 6.3 Hz, 1.5H), 0.82 (d, J = 6.1 Hz, 1.5H).

### Leu-β-Lapachone

To a solution of 2.017 mg (4.277 mmol) of Boc-Leu-β-Lapachone in 20 mL of CH₂Cl₂ was added 20 mL of TFA. The reaction mixture was stirred at room temperature for 30 min. The solvent was removed *in vacuo.* Column chromatography on silica gel with 20% MeOH in CH₂Cl₂ afforded 2.507 g (quant.) of the TFA salt as an orange glassy solid: *R*_{*f*} = 0.52 (10% MeOH in CH₂Cl₂), 0.82 (20% MeOH in CH₂Cl₂); ¹H NMR (250 MHz, DMSO-d₆, TMS) δ 8.6-8.5, (br s, NH), 8.0-7.9 (m, 1H), 7.9-7.8 (m, 2H), 7.7-7.6 (m, 1H), 5.3-5.2 (m, 1H), 4.1-4.0 (m, 1H), 2.8-2.5 (m, 2H), 1.8-1.5 (m, 3H), 1.52 (s, 1.5H), 1.49 (s, 1.5H), 1.43 (s, 3H), 0.83 (d, J = 6.0 Hz, 3H), 0.66 (br t, 3H); ¹³C NMR (52 MHz, DMSO-d₆, TMS) δ 178.7, 177.8, 169.2, 169.1, 160.0, 159.7, 135.1, 135.1, 131.5, 131.4, 131.1, 131.0, 129.8, 129.8, 127.9, 123.9, 123.8, 109.6, 109.3, 79.4, 79.1, 71.1, 70.9, 50.6, 50.4, 39.0, 24.0, 23.9, 22.9, 22.3, 22.1, 22.0, 21.8, 21.7, 21.1.

### Boc-Gln-Leu-β-Lapachone

To a solution of 2.235 g (3.895 mmol) of Leu-β-Lapachone-TFA salt and 959.1 mg (3.894 mmol) of Boc-Gln in 15.6 mL of DMF was added 631.4 mg (4.673 mmol) of 1-hydroxybenzotriazole. The mixture was cooled in an ice bath. Then 760 µL (6.912 mmol) of N-methylmorpholine were added, followed by 883.9 mg (4.284 mmol) of DCC. The reaction mixture was stirred in the ice bath for 30 min and at room temperature for 5.8 hr. The reaction mixture was then diluted with 8 mL of CH₂Cl₂ and filtered. The filtrate was washed with 5% citric acid (3 x 50 mL), with saturated NaHCO₃ (3 x 50 mL), with saturated NaCl (50 mL), dried with MgSO₄, and evaporated to dryness. Purification by column chromatography on silica gel with 5% MeOH in CH₂Cl₂ afforded 1.555 g (66%) of peptide as an orange glassy solid: *R*_{*f*} = 0.19 (5% MeOH in CH₂Cl₂), 0.09 (5% MeOH in CHCl₃), 0.37 (10% MeOH in CHCl₃); ¹H NMR (250 MHz, DMSO-d₆, TMS) δ 8.24 (br d, J = 7 Hz, NH), 8.17 (br d, J = 7 Hz, NH), 8.0-7.9 (m, 1H), 7.8-7.7 (m, 2H), 7.7-7.6 (m, 1H), 7.22 (br s, NH), 6.83 (br d, J = 8 Hz, NH), 6.76 (br s, NH), 5.1-5.0 (m, 1H), 4.3-4.1 (m, 1H), 3.9-3.8 (m, 1H), 2.8-2.6 (m, 1H), 2.6-2.4 (m, 1H), 2.1-2.0 (m, 2H), 1.8-1.4 (m, 5H), 1.47 (s, 1.5H), 1.43 (s, 1.5H), 1.42 (s, 1.5H), 1.40 (s, 1.5H), 1.36 (s, 9H), 0.86 (d, J = 6.3 Hz, 1.5H), 0.79 (d, J = 6.2 Hz, 1.5H), 0.73 (br t, 3H); ¹³C NMR (52 MHz, DMSO-d₆, TMS) δ 178.7, 177.8, 177.7, 173.7, 172.0, 171.7, 171.5, 159.9, 159.7, 155.1, 135.1, 135.0, 131.5, 131.4, 131.0, 130.9, 129.8, 129.7, 127.9, 127.8, 123.8, 109.8, 109.6, 79.5, 79.3, 77.9, 69.6, 69.4, 53.7, 53.6, 50.5, 50.4, 31.4, 28.1, 27.6, 27.4, 24.2, 24.1, 24.0, 22.6, 22.5, 22.1, 21.9, 21.6, 21.2.

### Gln-Leu-β-Lapachone

To a solution of 1.519 g (2.533 mmol) of Boc-Gln-Leu-β-Lapachone in 12 mL of CH₂Cl₂ was added 11 mL of TFA. The reaction mixture was stirred at room temperature for 30 min. The solvent was removed *in vacuo*. Column chromatography on silica gel with 20% MeOH in CH₂Cl₂ afforded 1.976 mg (quant) of the TFA salt as an orange glassy solid; ¹H NMR (250 MHz, DMSO-d₆, TMS) δ 8.97 (br d, J = 6.5 Hz, NH), 8.90 (br d, J = 7.0 Hz, NH), 8.30 (br s, NH), 8.0-7.9 (m, 1H), 7.9-7.8 (m, 2H), 7.7-7.6 (m, 1H), 7.45 (br s, NH), 6.98 (br s, NH), 5.2-5.1 (m, 1H), 4.3-4.2 (m, I H), 3.9-3.8 (m, I H), 2.8-2.7 (m, 1H), 2.5-2.4 (m, 1H), 2.2-2.1 (m, 2H), 2.0-1.8 (m, 2H), 1.7-1.5 (m, 3H), 1.49 (s, 1.5H), 1.44 (s, 1.5H), 1.42 (s, 1.5H), 1.41 (s, 1.5H), 0.87 (d, J = 6.3 Hz, 1.5H), 0.81 (d, J = 6.3 Hz, 1.5H), 0.75 (d, J = 5.8 Hz, 1.5H), 0.73 (d, J = 5.8 Hz, 1.5H); ¹³C NMR (52 MHz, DMSO-d₆, TMS) δ 178.7, 177.8, 177.8, 173.5, 171.3, 171.1, 168.7, 168.7, 159.9, 159.8, 135.1, 131.5, 131.4, 131.1, 131.0, 129.9, 129.8, 128.0, 123.8, 109.7, 109.5, 79.5, 79.3, 69.9, 69.8, 51.7, 51.6, 50.8, 50.8, 30.3, 26.8, 24.2, 24.1, 22.7, 22.5. 22.2, 22.0, 21.9, 21.6, 21.2.

### Boc-Leu-Gln-Leu-β-Lapachone

To a solution of 1.949 g (max 2.533 mmol) of Gln-Leu-β-Lapachone-TFA salt and 585.7 mg (2.533 mmol) of Boc-Leu in 10 mL ofDMF was added 410.6 mg (3.038 mmol) of 1-hydroxybenzotriazole. The mixture was cooled in an ice bath. Then 685 µL (6.230 mmol) of N-methylmorpholine were added, followed by 574.7 mg (2.785 mmol) of DCC. The reaction mixture was stirred in the ice bath for 30 min and at room temperature for 5.5 hr. The reaction mixture was then diluted with CHCl₃ and filtered. The filtrate was washed with 5% citric acid (5 x 50 mL), with saturated NaHCO₃ (4 x 70 mL), with saturated NaCl (70 mL), dried with MgSO₄, and evaporated to dryness. Purification by column chromatography on silica gel with 5% MeOH in CHCl₃ afforded 1.221 g (68%, from Boc-Gln-Leu-β-Lapachone) of peptide as an orange glassy solid: *R*_{*f*} = 0.09 (5% MeOH in CHCl₃), 0.29 (7% MeOH in CHCl₃); ¹H NMR (250 MHz, DMSO-d₆, TMS) δ 8.36 (br d, NH), 8.30 (br d, NH), 8.0-7.9 (m, 1H), 7.9-7.7 (m, 2H), 7.7-7.6 (m, 1H), 7.19 (br s, NH), 6.90 (br s, NH), 6.75 (br d, NH), 5.1-5.0 (m, 1H), 4.3-4.1 (m, 2H), 4.0-3.9 (m, 1H), 2.8-2.7 (m, 1H), 2.5-2.4 (m, 1H), 2.1-2.0 (m, 2H), 1.8-1.4 (m, 8H), 1.47 (s, 1.5H), 1.43 (s, 1.5H), 1.41 (s, 1.5H), 1.40 (s, 1.5H), 1.37 (s, 4.5H) 1.35 (s, 4.5H), 0.9-0.8 (m, 7.5H), 0.78 (d, J = 6.2 Hz, 1.5H), 0.73 (d, J = 5.5 Hz, 1.5H), 0.71 (d, J = 5.3 Hz, 1.5H); ¹³C NMR (52 MHz, DMSO-d₆, TMS) δ 178.7, 177.8, 177.7, 173.6, 173.6, 172.3, 171.5, 171.4, 171.3, 159.9, 159.7, 155.2, 135.0, 131.5, 131.4, 131.0, 130.9, 129.8, 129.8, 127.9, 127.9, 123.8, 109.7, 109.6, 79.5, 79.3, 78.0, 69.6, 69.5, 52.8, 51.4, 50.5, 50.5, 40.7, 31.2, 28.1, 24.2, 24.1, 22.9, 22.6, 22.5, 22.1, 22.0, 21.9, 21.6, 21.4, 21.2.

### Leu-Gln-Leu-β-Lapachone

To a solution of 1.196 g (1.678 mmol) of Boc-Leu-Gln-Leu-β-Lapachone in 8 mL of CH₂Cl₂ was added 8 mL of TFA. The reaction mixture was stirred at room temperature for 30 min. The solvent was removed in *vacuo.* Column chromatography on silica gel with 20% MeOH in CHCl₃ afforded 1.430 g (quant) of the TFA salt as an orange glassy solid: *R*_{*f*} = 0.04 (10% MeOH in CHCl₃), 0.10 (15% MeOH in CHCl₃), 0.19 (20% MeOH in CHCl₃). ; ¹H NMR (250 MHz, DMSO-d₆, TMS) δ 8.46 (br d, J = 6.6 Hz, NH), 8.41 (br d, J = 7.2 Hz, NH), 8.0-7.9 (m, 1H), 7.9-7.8 (m, 2H), 7.7-7.6 (m, 1H), 7.26 (br s, NH), 6.77 (br s, NH), 5.1-5.0 (m, 1H), 4.3-4.1 (m, 2H), 3.5-3.4 (m, 1H), 2.8-2.7 (m, 1H), 2.5-2.4 (m, 1H), 2.1-2.0 (m, 2H), 1.9-1.4 (m, 8H), 1.47 (s, 1.5H), 1.43 (s, 1.5H), 1.41 (s, 1.5H), 1.40 (s, 1.5H), 0.9-0.8 (m, 7.5H), 0.78 (d, J = 6.1 Hz, 1.5H), 0.74 (d, J = 5.9 Hz, 1.5H), 0.72 (d, J = 5.5 Hz, 1.5H); ¹³C NMR (52 MHz, DMSO-d₆, TMS) δ 178.7, 177.8, 177.8, 173.6, 171.6, 171.4, 171.2, 159.9, 159.8, 135.1, 131.5, 131.4, 131.1, 131.0, 129.9, 129.8, 127.9, 123.9, 109.8, 109.6, 79.6, 79.3, 69.6, 69.5, 51.9-51.6, 51.6, 50.5, 42.3-41.8, 31.2, 28.2, 28.0, 24.2, 24.1, 23.7, 22.8, 22.7, 22.6, 22.1, 21.9, 21.8, 21.6, 21.3, 21.2.

### Nα-Boc-Lys(Nε-Cl-Cbz)-Leu-Gln-Leu-β-Lapachone

To a solution of 1.400 g (max 1.643 mmol) of Leu-Gln-Leu-β-Lapachone-TFA salt and 681.6 mg (1.643 mmol) of Nα-Boc-Lys(Nε-Cl-Cbz) in 6.6 mL of DMF was added 266.3 mg (1.971 mmol) of 1-hydroxybenzotriazole. The mixture was cooled in an ice bath. Then 380 µL (3.456 mmol) of N-methylmorpholine were added, followed by 372.9 mg (1.807 mmol) of DCC. The reaction mixture was stirred in the ice bath for 30 min and at room temperature for 5.5 hr. The reaction mixture was then diluted with CHCl₃ and filtered. The filtrate was washed with 5% citric acid (4 x 50 mL), with saturated NaHCO₃ (4 x 50 mL), with saturated NaCl (65 mL), dried with MgSO₄, and evaporated to dryness. Purification by column chromatography on silica gel with 5% MeOH in CHCl₃ afforded 897.4 mg (54%) of peptide as an orange glassy solid: *R*_{*f*} = 0.10 (5% MeOH in CHCl₃); ¹H NMR (250 MHz, DMSO-d₆, TMS) δ 8.31 (br d, J = 7 Hz, NH). 8.25 (br d, J = 7 Hz, NH), 8.0-7.9 (m, 2H (1 quinone-H + 1 NH)), 7.8-7.7 (m, 3H (2 quinone-H + 1 NH)), 7.7-7.6 (m, 1 H (quinone-H)), 7.5-7.4 (m, 2H), 7.4-7.3 (m, 3H (2 Cl-Ph-H + 1 NH)), 7.19 (br s, NH), 6.90 (br d, J = 8 Hz, NH), 6.77 (br s, NH), 5.1-5.0 (m, 4H), 4.3-4.1 (m, 3H), 3.9-3.8 (m, 1H), 3.0-2.9 (m, 2H), 2.8-2.7 (m, 1H), 2.5-2.4 (m, 1H), 2.1-2.0 (m, 2H), 1.9-1.4 (m, 14H), 1.47 (s, 1.5H), 1.42 (s, 1.5H), 1.41 (s, 1.5H), 1.40 (s, 1.5H), 1.37 (s, 9H), 0.9-0.8 (m, 7.5H), 0.77 (d, J = 6.2 Hz, 1.5H), 0.73 (d, J = 5.7 Hz, 1.5H), 0.70 (d, J = 5.6 Hz, 1.5H); ¹³C NMR (52 MHz, DMSO-d₆, TMS) δ 178.7, 177.8, 177.7, 173.6, 171.8, 171.6, 171.4, 171.3, 159.9, 159.7, 155.7, 155.3, 135.0, 134.5, 132.2, 131.5, 131.4, 131.0, 130.9, 129.8, 129.8, 129.5, 129.1, 127.9, 127.8, 127.2, 123.8, 109.7, 109.6, 79.5, 79.3, 78.0, 69.6, 69.5, 62.4, 54.3, 51.6, 50.7, 50.5, 50.4, 41.0, 40.1, 31.3, 29.0, 28.1, 27.9, 27.7, 24.2, 24.1, 24.0, 23.9, 23.0, 22.7, 22.6, 22.5, 22.1, 22.0, 21.9, 21.6, 21.5, 21.2.

### Lys(Nε-Cl-Cbz)-Leu-Gln-Leu-β-Lapachone

To a solution of 1.196 g (1.678 mmol) of Boc-Lys(Nε-Cl-Cbz)-Leu-Gln-Leu-β-Lapachone in 6 mL of CH₂Cl₂ was added 5 mL of TFA. The reaction mixture was stirred at room temperature for 30 min. The solvent was removed *in vacuo.* Column chromatography on silica gel with 15% MeOH in CHCl₃ afforded 568.9 mg (65%) of the TFA salt as an orange glassy solid: *R*_{*f*} = 0.09 (10% MeOH in CHCl₃), 0.23 (15% MeOH in CHCl₃), 0.38 (20% MeOH in CHCl₃). ; ¹H NMR (250 MHz, DMSO-d₆, TMS) δ 8.28 (br d, J = 7 Hz, NH), 8.23 (br d, J = 7 Hz, NH), 8.1-8.0 (m, NH), 8.0-7.9 (m, 2H (1 quinone-H + 1 NH)), 7.8-7.7 (m, 2H), 7.7-7.6 (m, 1H), 7.5-7.4 (m, 2H), 7.4-7.3 (m, 3H (2 Cl-Ph-H + 1NH)), 7.23 (br s, NH), 6.78 (br s, NH), 5.1-5.0 (m, 4H), 4.3-4.1 (m, 4H), 3.0-2.9 (m, 2H), 2.8-2.7 (m, 1H), 2.5-2.4 (m, 1H), 2.1-2.0 (m, 2H), 1.9-1.4 (m, 14H), 1.47 (s, 1.5H), 1.42 (s, 1.5H), 1.41 (s, 1.5H), 1.39 (s, 1.5H), 0.9-0.8 (m, 7.5H), 0.77 (d, J = 6.2 Hz, 1.5H), 0.73 (d, J = 5.8 Hz, 1.5H), 0.71 (d, J = 5.6 Hz, 1.5H); ¹³C NMR (52 MHz, DMSO-d₆, TMS) δ 178.7, 177.8, 177.7, 173.7, 171.8, 171.6, 171.4, 171.3, 159.9, 159.7, 155.7, 135.0, 134.6, 132.2, 131.5, 131.4, 131.0, 130.9, 129.9, 129.8, 129.5, 129.2, 127.9, 127.8, 127.2, 123.8, 109.7, 109.6, 79.5, 79.3, 69.6, 69.4, 62.4, 54.4, 51.7, 50.6, 50.5, 50.4, 41.1, 31.2, 29.2, 27.6, 27.5, 24.2, 24.2, 24.1, 23.0, 22.6, 22.5, 22.4, 22.0, 21.9, 21.6, 21.2.

### Morpholino-Ser(OBn)-Lys(Nε-Cl-Cbz)-Leu-Gln-Leu-β-Lapachone

To a solution of 544.9 mg (5.323x10⁻⁴ mol) of Lys(Nε-Cl-Cbz)-Leu-Gln-Leu-β-Lapachone-TFA salt and 164.2 mg 5.325x10⁻⁴ mol) of morpholino-Ser(OBn) in 2.15 mL of DMF was added 86.2 mg (6.379x10⁻⁴ mol) of 1-hydroxybenzotriazole. The mixture was cooled in an ice bath. Then 59 µL (5.366x10⁻⁴ mol) of N-methylmorpholine were added, followed by 120.7 mg (5.850x10⁻⁴ mol) of DCC. The reaction mixture was stirred in the ice bath for 30 min and at room temperature for 5.5 hr. The reaction mixture was then diluted with CHCl₃ and filtered. The filtrate was washed with 5% citric acid (4 x 30 mL), with saturated NaHCO₃ (4 x 30 mL), with saturated NaCl (30 mL), dried with MgSO₄, and evaporated to dryness. Purification by column chromatography on silica gel with 7% MeOH in CHCl₃ afforded 515.8 mg (81%) of peptide as an orange glassy solid: *R*_{*f*} = 0.17 (7% MeOH in CHCl₃), 0.36 (10% MeOH in CHCl₃); ¹H NMR (250 MHz, DMSO-d₆, TMS) δ 8.22 (br d, J = 7 Hz, NH), 8.18 (br d, J = 7 Hz, NH), 8.0-7.9 (m, 2H (1 quinone-H + 1 NH)), 7.9-7.7 (m, 3H (2 quinone-H + I NH)), 7.7-7.6 (m, 1H), 7.5-7.4 (m, 2H), 7.4-7.2 (m, 8H (2 Cl-Ph-H + 5 Ph-H + 1 NH)), 7.20 (br s, NH), 6.78 (br s, NH), 6.60 (br d, J = 7 Hz, NH), 5.1-5.0 (m, 4H), 4.50 (s, 2H), 4.4-4.3 (m, 1H), 4.3-4.1 (m, 4H), 3.7-3.6 (m, 2H), 3.6-3.5 (m, 4H), 3.3-3.2 (m, 4H), 3.0-2.9 (m, 2H), 2.8-2.6 (m, 1H), 2.5-2.4 (m, 1H), 2.1-2.0 (m, 2H), 1.9-1.4 (m, 14H), 1.46 (s, 1.5H), 1.42 (s, 1.5 H), 1.41 (s, 1.5H), 1.39 (s, 1.5H), 0.9-0.7 (m, 9H), 0.72 (d, J = 5.4 Hz, 1.5H), 0.70 (d, J = 5.3 Hz, 1.5H); ¹³C NMR (52 MHz, DMSO-d₆, TMS) δ 178.7, 177.8, 177.7, 173.6, 171.6, 171.5, 171.4, 171.3, 171.3, 170.8, 170.8, 159.9, 159.7, 157.3, 155.7, 138.2, 135.0, 134.5, 132.2, 131.5, 131.4, 131.0, 130.9, 129.9, 129.8, 129.5, 129.1, 128.1, 127.9, 127.8, 127.4, 127.3, 127.2, 123.8, 109.8, 109.6, 79.5, 79.3, 71.9, 69.6, 69.5, 65.8, 62.4, 54.6, 52.7, 51.7, 51.0, 50.5, 50.4, 43.9, 31.3, 31.3, 29.0, 27.8, 27.7, 24.2, 24.2, 24.1, 24.0, 22.9, 22.5, 22.5, 22.0, 21.8, 21.6, 21.4, 21.2.

### Morpholino-Ser-Lys-Leu-Gln-Leu-β-Lapachone (SL-11154)

To a solution of 486.8 mg (4.057x10⁻⁴ mol) of morpholino-Ser(OBn)-Lys(Nε-Cl-Cbz)-Leu-Gln-Leu-β-Lapachone in 9 mL of MeOH/CHCl₃ = 1:9 was added 180.5 mg 10% Pd/C. Then two drops of HCl were added. The reaction mixture was placed under an atmosphere of H₂ (balloon) and stirred at room temperature for 15.5 hr. Removal of catalyst by filtration and evaporation of solvent afforded a light brown solid. The material was dissolved in 12 mL of MeOH/CHCl₃ = 1:9, and stirred at room temperature for I hr while bubbling air through the solution. Evaporation of solvent afforded an orange glassy solid. Column chromatography on silica gel with 20-30% MeOH in CHCl₃ yielded 52.8 mg (14%) of material as an orange solid. The material was further purified by prep HPLC: *R*_{*f*} = 0.06 (20% MeOH in CHCl₃).

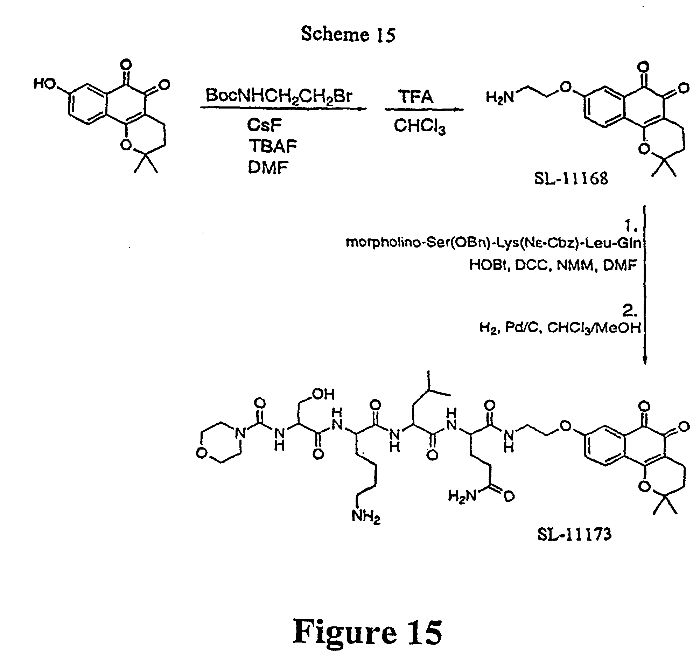

### Synthesis Of Morpholine-Ser-Lys-Leu-Gln-NHCH₂CH₂O-β-Lapachone (SI-11173) (see Fig. 15)

### 8-(N-Boc-(2-Aminoethoxy))-β-Lapachone

To a solution of 507.1 mg (2.263 mmol) of N-boc-2-bromethylamine and 562.3 mg (2.177 mmol) of 8-hydroxy-β-Lapachone in 18 mL of DMF was added 727 mg (4.786 mmol) of CsF, followed by 2.2 mL of a solution of 1M TBAF in THF. The reaction mixture was stirred under N₂ at room temperature for 48 hr. Then the reaction mixture was partitioned between 100 mL of CHCl₃ and 75 mL of water plus 10 mL of 5% citric acid. The aqueous phase was extracted with CHCl₃ (5 x 40 mL). The CHCl₃ extracts were combined, dried with MgSO₄, and evaporated. Column chromotography on silica gel with 5% MeOH in CHCl₃ afforded 305.8 mg (35%) of quinone as a red-orange glassy solid; R_{*f*} = 0.49(5% MeOH in CHCl₃); ¹H NMR (250 MHz, DMSO-d₆, TMS) δ 7.68 (d, J=8.6 Hz, 1H), 7.35 (d, J=2.7 Hz, 1H), 7.28 (dd, J=8.6, 2.7 Hz, 1H), 7.05 (br t, NH), 4.08 (t, J=5.6 Hz, 2H), 3.4-3.3 (m, 2H), 2.37 (t, J=6.5 Hz, 2H), 1.81 (t, J=6.5 Hz, 2H), 1.41 (s, 6H), 1.39 (s, 9H), ¹³C NMR (52 MHz, DMSO-d₆, TMS) δ 179.0, 177.8, 161.3, 160.3, 131.4, 125.6, 124.7, 120.5, 113.3, 110.3, 78.9, 77.8, 67.0, 30.8, 28.1, 26.2, 15.7.

### 8-(2-Aminoethoxy)-β-Lapachone (SL-11168)

To a solution of 219.8 mg (5.474x10⁻⁴ mol) of 8-(N-Boc-(2aminoethyoxy))-β-lapachone in 6 mL of CHCl₃ was added 6 mL of TFA. The reaction mixture was stirred at room temperature for 20 min. The solvent was removed *in vacuo*. Column chromatography on silica gel with 20% MeOH in CHCl₃ afforded 210.7 mg (93%) of quinone (as the trifluoroacetate salt) as a red glassy solid: R_{*f*}= 0.13 (10% MeOH in CHCl₃); ¹H NMR (250 MHz, DMSO-d₆, TMS) δ 8.1-8.0 (v br s, NH), 7.74 (d, J=8.6 Hz, 1H), 7.45 (d, J=2.6 Hz, 1H), 7.34 (dd, J=8.6, 2.6 Hz, 1H), 4.4-4.2 (m, 2H), 3.3-3.2 (m, 2H), 2.38 (t, 6.5 Hz, 2H), 1.82 (t, J=6.5 Hz, 2H), 1.42 (s, 6H).

### Morpholino-Ser(OBn)-Lys(Nε-Cbz)-Leu-Gln-NHCH₂CH₂O-β-Lapachone

To a solution of 210.7 mg (5.072x10⁻⁴ mol) of NH₂CH₂CH₂O-β-lapachone-TFA salt and 411.9 mg (5.072x10⁴ mol) of morpholino-Ser(OBn)-Lys(Nε-Cbz)-Leu-Gln in 2.25 mL of DMF was added 82.4 mg (6.098x10⁻⁴ mol) of 1-hydroxybenzotriazale. The mixture was cooled in an ice bath. Then 56 µL (5.093x10⁻⁴ mol) of N-methylmorpholine were added, followed by 115.1 mg (5.578x10⁻⁴ mol) of DCC. The reaction mixture was stirred in the ice bath for 45 min and at room temperature for 5 hr. The reaction mixture was then filtered and the filtrate diluted with CHCl₃. The filtrate was washed with 5% citric acid (4 x 30 mL), with saturated NaHCO₃ (3 x 40 mL), with saturated NaCl (40 mL), dried with MgSO₄, and evaporated to dryness. Purification by column chromatography on silica gel with 5% MeOH in CHCl₃ afforded 139.6 mg (25%) of peptide as a red-orange glassy solid: R_{f}= 0.07 (5% MeOH in CHCl₃); 0.33 (10% MeOH in CHCl₃); ¹H NMR (250 MHz, DMSO-d₆, TMS) δ 8.00 (br d, J=6 Hz, NH), 7.85 (br d, J=8 Hz, NH), 7.82 (br d, J=7 Hz, NH), 7.68 (d, J=8.6 Hz, 1H (quinone)), 7.4-7.2 (m, 12H (2 quinone + 10 Ph)), 7.2-7.1 (m, NH), 6.75 (br s, NH), 6.60 (br d, J=7 Hz, NH), 4.99 (s, 2H), 4.48 (s, 2H), 4.4-4.3 (m, 1H), 4.3-4.0 (m, 5H), 3.7-3.6 (m, 2H), 3.6-3.4 (m, 6H), 3.35-3.25 (m, 4H), 3.0-2.9 (m, 2H), 2.4-2.3 (m, 2H), 2.1-2.0 (m, 2H), 1.9-1.4 (m, 13H), 1.40 (s, 6H), 0.81 (d, J=6.4 Hz, 3H), 0.77 (d, J=6.3 Hz, 3H).

### Morpholino-Ser-Lys-Leu-Gln-NHCH₂CH₂O-β-Lapachone

To a solution of 133.1 mg (1.215x10⁻⁴ mol) of morpholino-Ser(OBn)-Lys(Nε-Cbz)-Leu-Gln-NHCH₂CH₂O-β-lapachone in 45 mL MeOH plus 5 mL CHCl₃ was added 57.9 mg of 10% Pd/C. Then two drops of HCl were added. The reaction mixture was placed under an atmosphere of H₂ (balloon) and stirred at room temperature for 23 hr. Removal of catalyst by filtration and evaporation of solvent afforded a reddish-brown solid. The material was dissolved in 20 mL of MeOH and stirred at room temperature for 21 hr while bubbling air through the solution. Evaporation of solvent afforded 107.0 mg of a dark red glassy solid. The material was purified by prep HPLC to yield 55.1 mg (52%).

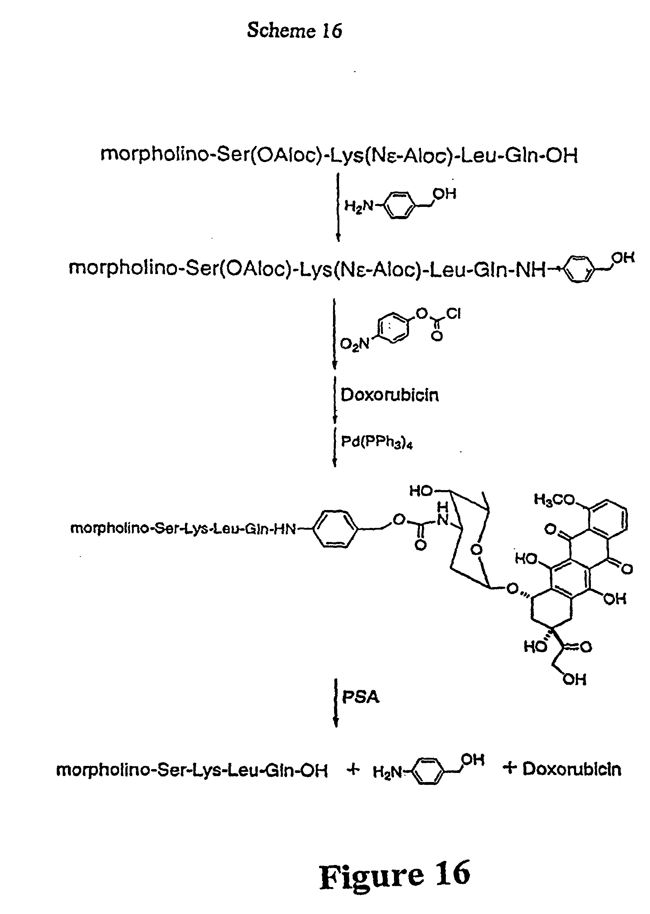

### Synthesis of Morpholino-Ser-Lys-Leu-Gln-PABC-DOX (see Fig. 16)

Morpholino-Ser(OAloc) was prepared from Ser(OtBu)-OtBu. Reaction of Ser(OtBu)-OtBu with 4-morpholinecarbonyl chloride in pyridine yielded morpholino-Ser(OtBu)-OtBu. Morpholino-Ser(OtBu)-OtBu was hydrolyzed with TFA to yield morpholino-Ser. Esterification of morpholino-Ser with isobutylene in the presence of a catalytic amount of H₂SO₄ afforded morpholino-Ser-OtBu. Reaction of morpholino-Ser-OtBu with allyl 1-benzotriazolyl carbonate yielded morpholino-Ser(OAloc)-OtBu. Morpholino-Ser(OAloc)-OtBu was hydrolyzed with TFA in CHCl₃ (1:1) to yield morpholino-Ser(OAloc).

Preparation of the tetrapeptide was accomplished using standard procedures. Fmoc-Leu was coupled to Gln-OtBu with DCC in the presence of 1-hydroxybenzotriazole (HOBt) to give Fmoc-Leu-Gln-OtBu. Removal of the Fmoc group from Fmoc-Leu-Gln-OtBu with piperidine in CH₂Cl₂/DMF produced Leu-Gln-OtBu. Fmoc-Lys(Nε-Aloc) was coupled to Leu-Gln-OtBu with DCC in the presence of HOBt to give Fmoc-Lys(Nε-Aloc)-Leu-Gln-OtBu. Removal of the MFmoc group from Fmoc-Lys(Ne-Aloc)-Leu-Gln-OtBu with piperidine in DMF produced Lys(Nε-Aloc)-Leu-Gln-OtBu. Morpholino-Ser(OAloc) was coupled to Lys(Ne-Aloc)-Leu-Gln-OtBu with DCC in the presence of HOBt to give morpholino-Ser(OAloc)-Lys(Nε-Aloc)-Leu-Gln-OtBu. Hydrolysis of morpholino-Ser(OAloc)-Lys(Ne-Aloc)-Leu-Gln-OtBu with TFA in CHCl₃ (1:1) would give the tetrapeptide morpholino-Ser(OAloc)-Lys(Nε-Aloc)-Leu-Gln-OH. The tetrapeptide is condensed with PABC-DOX as described elsewhere. De Groot et al. (1999) *J. Med. Chem*. 42:5277-83. The amino acid side chains are deprotected as described. De Groot et al. (1999) *J. Med. Chem.* 42:5277-83. Morpholino-Ser-Lys-Leu-Gln-PABC-DOX has been used as a substrate of the enzyme PSA as shown in Figure 16.

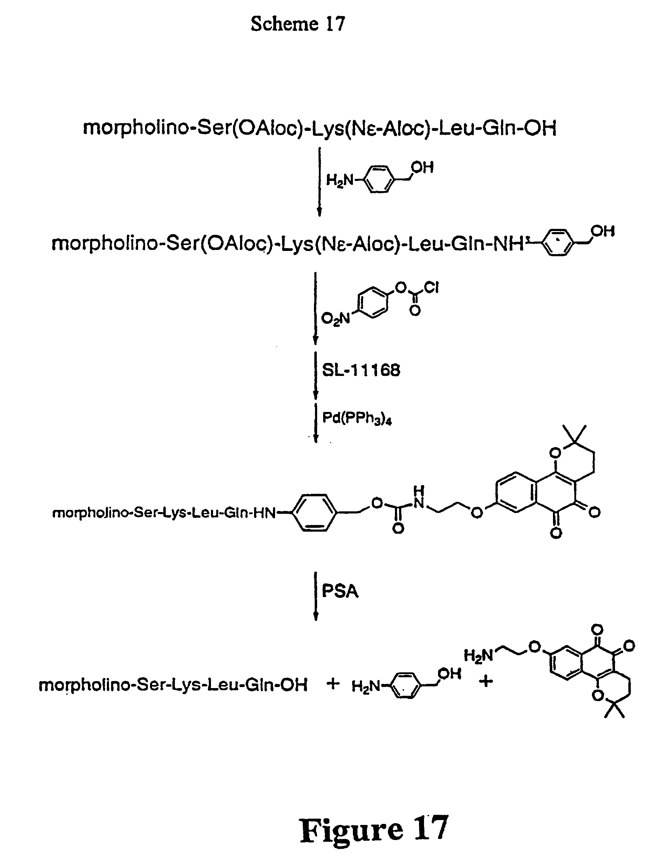

### Synthesis of Morpholino-Ser-Lys-Leu-Gln-PABC-NHCH₂CH₂O-β-Lapachone (see Fig. 17)

Morpholino-Ser(OAloc) was prepared from SER(OtBu)-OtBu. Reaction of Ser(OtBu)-OtBu with 4-morpholineacarbonyl chloride in pyridine yielded morpholino-Ser(OtBu)-OtBu. Morpholino-Ser(OtBu)-OtBu was hydrolyzed with TFA to yield morpholino-Ser. Esterification of morpholino-Ser with isobutylene in the presence of a catalytic amount of H₂SO₄ afforded morpholino-Ser-OtBu. Reaction of morpholino-Ser-OtBu with allyl 1-benzotriazolyl carbonate yielded morpholino-Ser(OAloc)-OtBu. Morpholino-Ser(OAloc)-OtBu was hydrolyzed with TFA in CHCl₃ (1:1) to yield morpholino-Ser(OAloc).

Preparation of the tetrapeptide was accomplished using standard procedures. Fmoc-Leu was coupled to Gln-OtBu with DCC in the presence of 1-hydroxybenzotriazole (HOBt) to give Fmoc-Leu-Gln-OtBu. Removal of the Fmoc group from Finoc-Leu-Gln-OtBu with piperidine in CH₂Cl₂/DMF produced Leu-Gln-OtBu. Fmoc-Lys(Nε-Aloc) was coupled to Leu-Gln-OtBu with DCC in the presence of HOBt to give Fmoc-Lys(Nε-Aloc)-Leu-Gln-OtBu. Removal of the Fmoc group from Fmoc-Lys(Nε-Aloc)-Leu-Gln-OtBu with piperidine in DMF produced Lys(Nε-Aloc)-Leu-Gln-OtBu. Morpholino-Ser(OAloc) was coupled to Lys(Nε-Aloc)-Leu-Gln-OtBu with DCC in the presence of HOBt to give morpholino-Ser(OAloc)-Lys(Nε-Aloc)-Leu-Gln-OtBu. Hydrolysis of morpholino-Ser(OAloc)-Lys(Nε-Aloc)-Leu-Gln-OtBu with TFA in CHCl₃ (1:1) would give the tetrapeptide morpholino-Ser(OAloc)-Lys(Nε-Aloc)-Leu-Gln-OH. The tetrapeptide is condensed with PABC-NHCH₂CH₂O-β-lapachone in an analogous manner as the condensation of the tetrapeptide with doxorubicin, described in De Groot et al. (1999) *J. Med. Chem.* 42:5277-83; the amino acid side chains are deprotected using the procedure described in that reference. Morpholino-Ser-Lys-Leu-Gln-PABC-NHCH₂CH₂O-β-lapachone is used as a substrate of the enzyme PSA as shown in Figure 17.

### EXAMPLE 2

### CELL CULTURE AND DRUG TESTING PROTOCOL

**Cell Culture:** The human lung adenocarcinoma cell line, A549, and human prostatic cancer cell line, DUPRO, were a gift from Dr. M. Eileen Dolan, University of Chicago, Department of Medicine. A549 was grown in Ham's F-12K medium (Fisher Scientific, Itasca, IL) supplemented with 10% fetal bovine serum and 2 mM L-glutamine. DUPRO was grown in RPMI-1640 supplemented with 10% fetal bovine serum. The human colon carcinoma cell line, HT29, and the human breast carcinoma cell line, MCF7, were obtained from the American Type Culture Collection, Rockville, MD. HT29 cells were grown in McCoy's 5A medium (Gibco, BRL, Gaithersburg, MD) supplemented with 10% fetal bovine serum. MCF7 cells were grown in Richter's Improved Modified Eagle's medium supplemented with 10% fetal bovine serum and 2.2 g/L sodium bicarbonate. The human prostate adenocarcinoma cell lines, LNCAP, PC-3 and DU145, were gifts from Dr. George Wilding, University of Wisconsin Comprehensive Cancer Center and the Department of Medicine, and were grown in Dulbecco's Modified Eagle's medium supplemented with a 5% fetal bovine serum. The malignant glioma cell line, U251 MG NCI was obtained from the brain tumor tissue bank at the University of California, San Francisco Department of Neurosurgery, and was grown in Dulbecco's Modified Eagle's medium supplemented wth 10% fetal bovine serum. DUPRO, A549 and MCF7 cells were grown in 100 units/mL penicillin and 100 µg/mL streptomycin. HT29 and U251MG NCI cells were grown in 50 µg/mL gentamycin. LNCAP, PC-3 and DU145 cells were maintained in 1% antibiotic antimycotic solution (Sigma, St. Louis, MO). All cell cultures were maintained at 37°C in 5% CO₂/95% humidified air.

**MTT assay.** Exponentially growing monolayer cells were plated in 96 well plates at a density of 500 cells/well and allowed to grow for 24 h. Serially diluted drug solutions were added such that the final drug concentrations in the treatment media were between 0 and 35 µM. Cells were incubated with drug at either 4 hr or 72 hr. After 4 hr and 72 hr treatment, drugs were removed, fresh media (without) drug (100 uL) was added and cells were incubated for 6 days. After six days, 25 µL of a Dulbecco's phosphate-buffered saline solution containing 5 mg/mL of MTT (Thiazolyl blue) (Sigma) was added to each well and incubated for 4h at 37°C. Then 100 µL of lysis buffer (20% sodium dodecyl sulfate, 50% N,N-dimethylformamide and 0.8% acetic acid, pH 4.7) was added to each well and incubated for an additional 22 h. A microplate reader (E max, Molecular Devices, Sunnyvale, CA) set at 570 nm was used to determine the optical density. Results were plotted as a ratio of the optical density in drug treated wells to the optical density in wells treated with vehicle alone. Plotting and estimation of ID₅₀ values were accomplished with manufacturer supplied software.

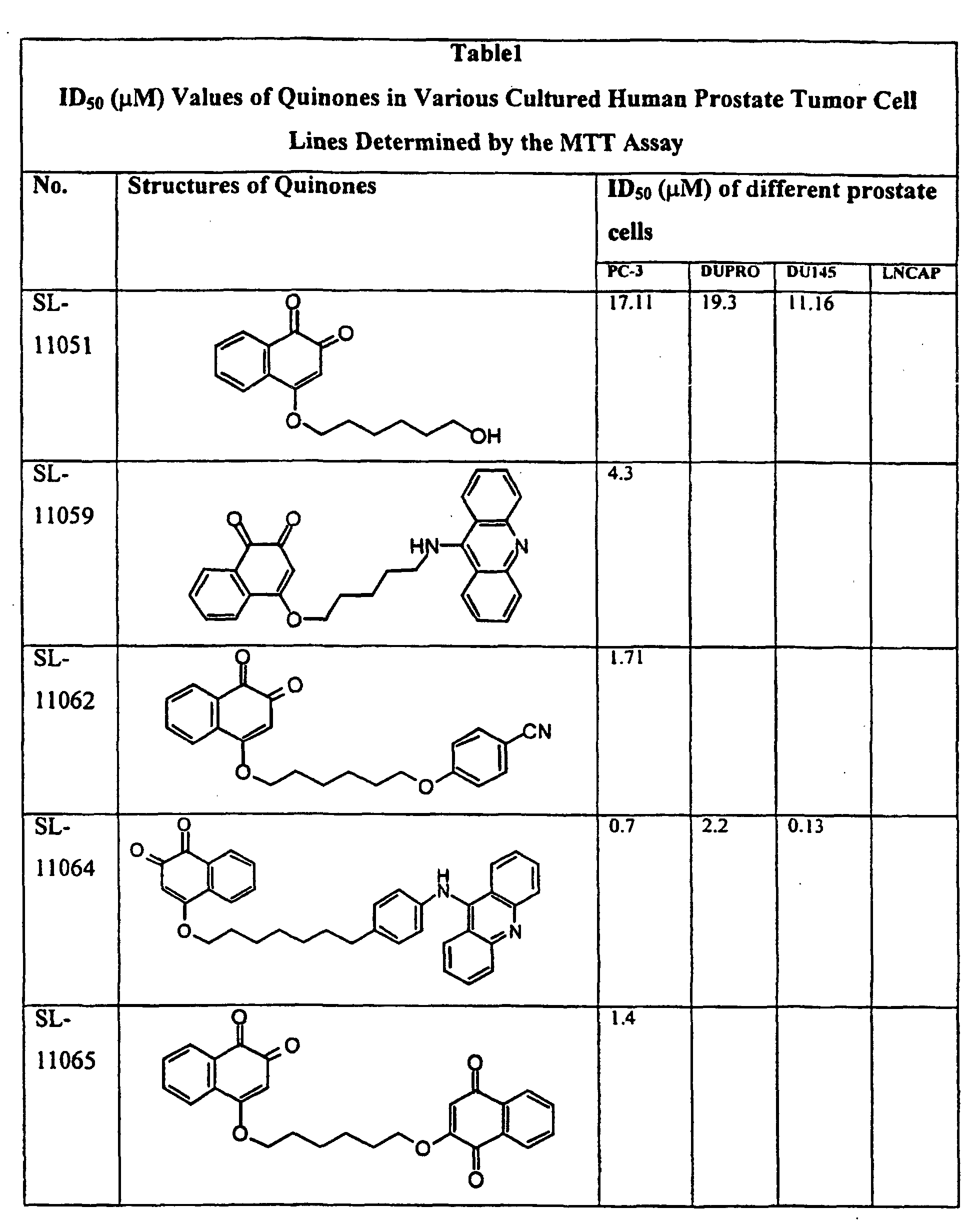

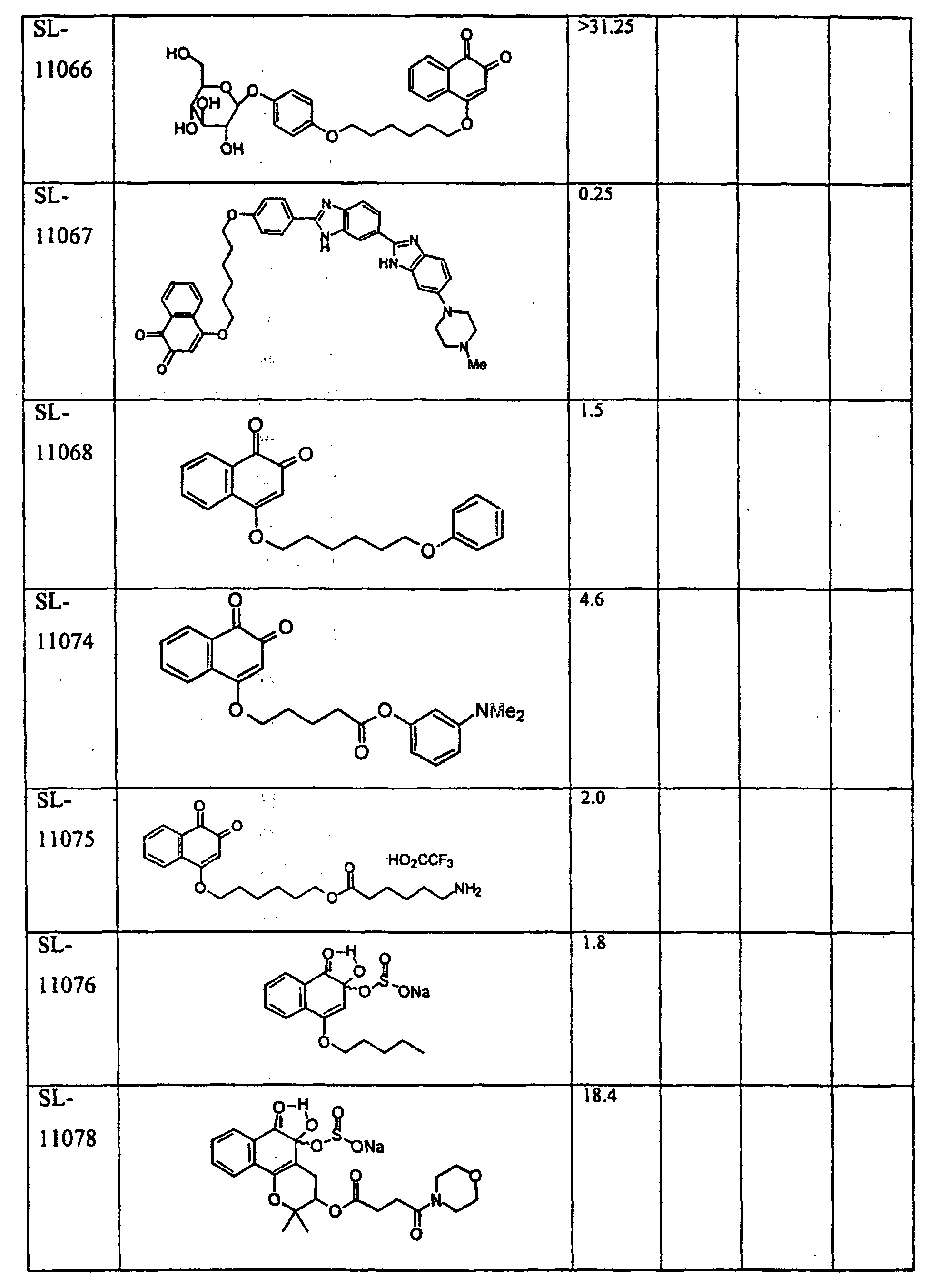

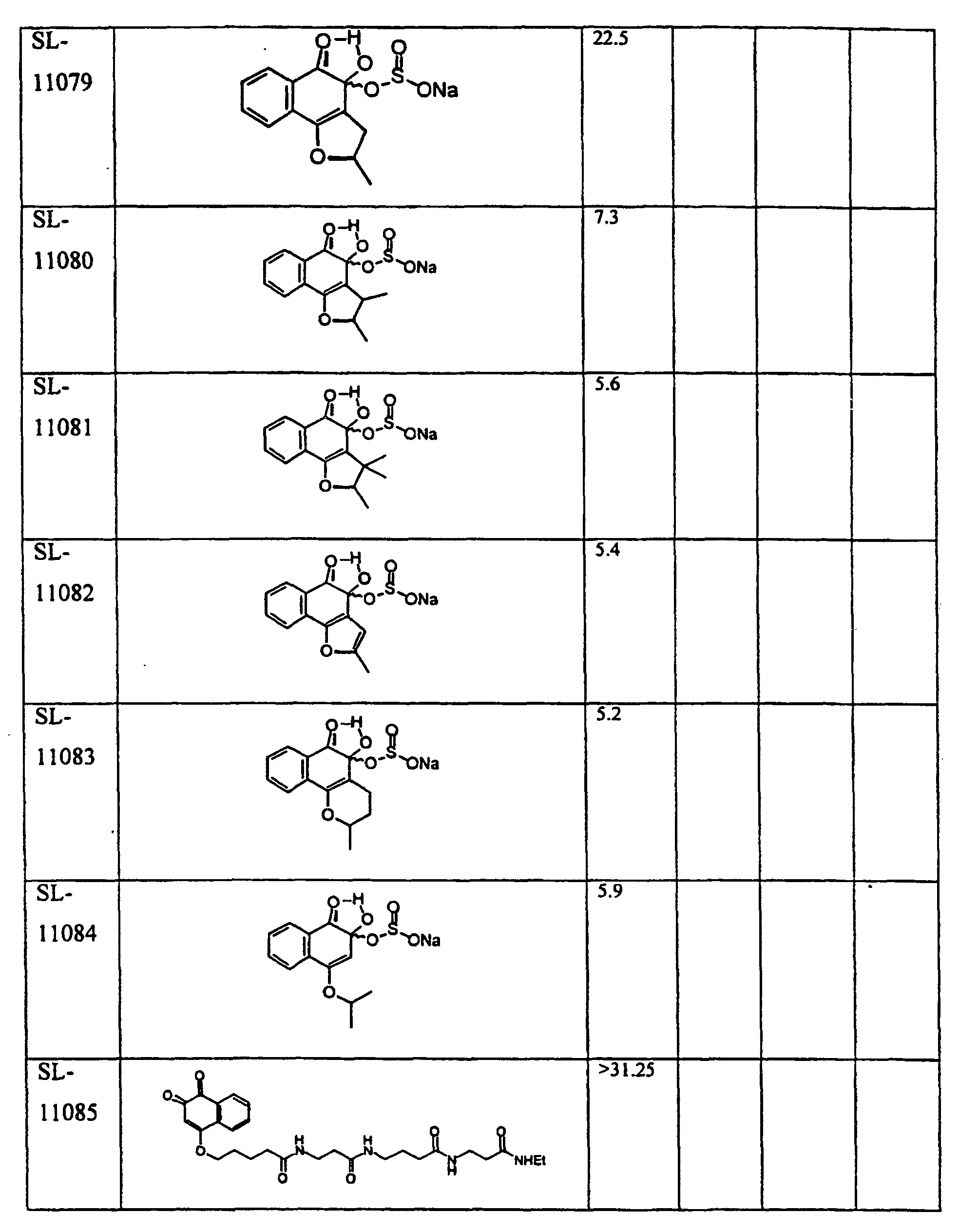

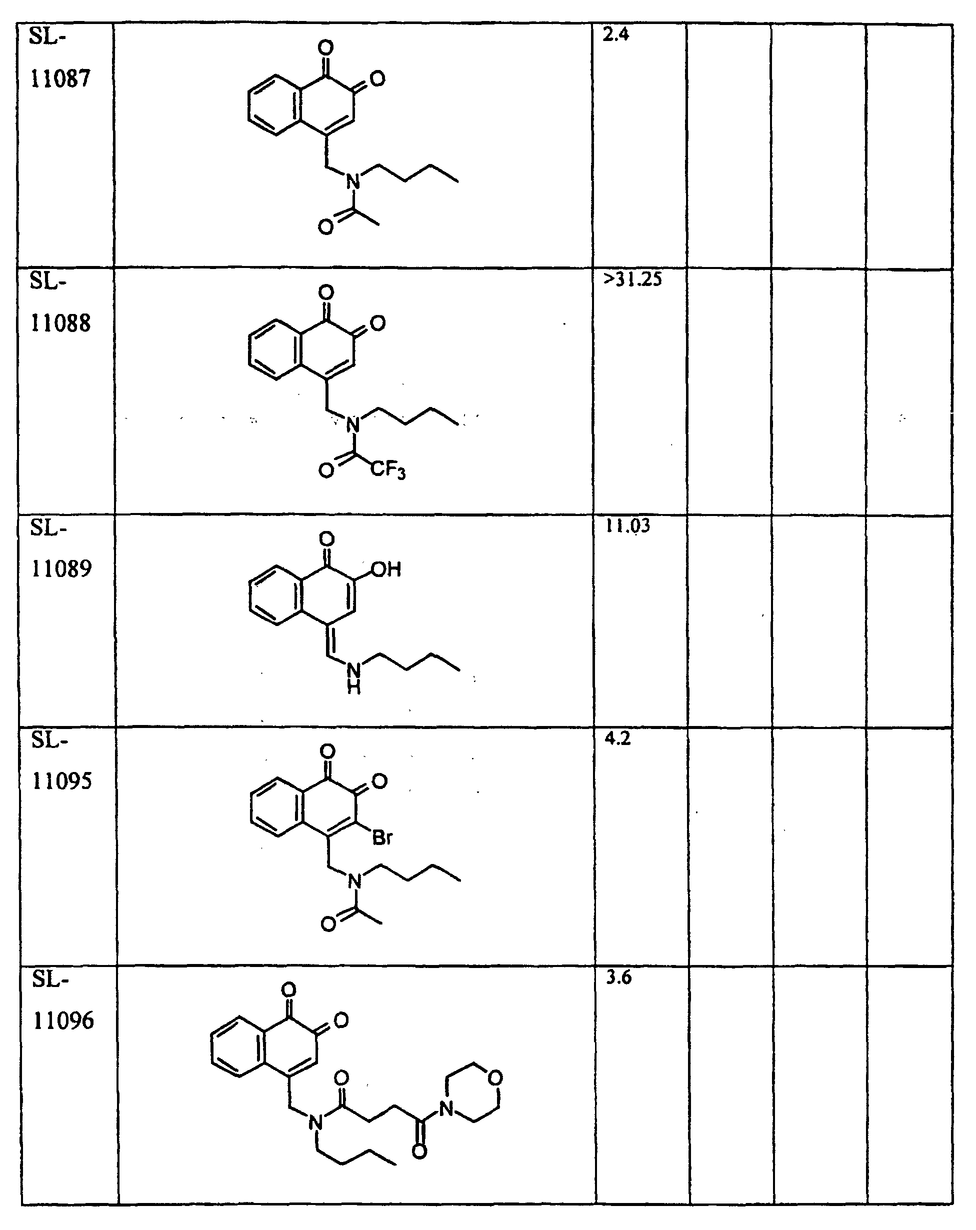

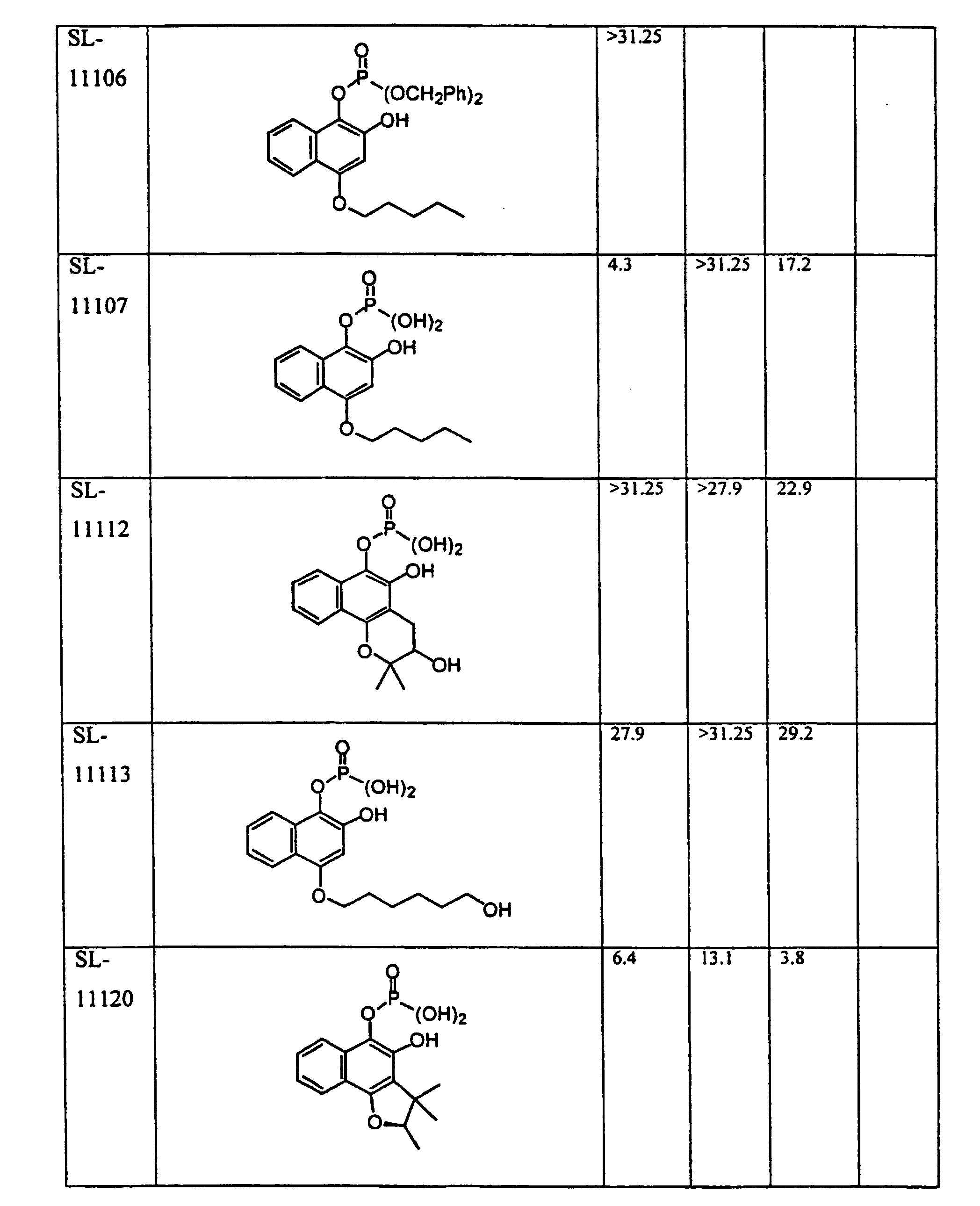

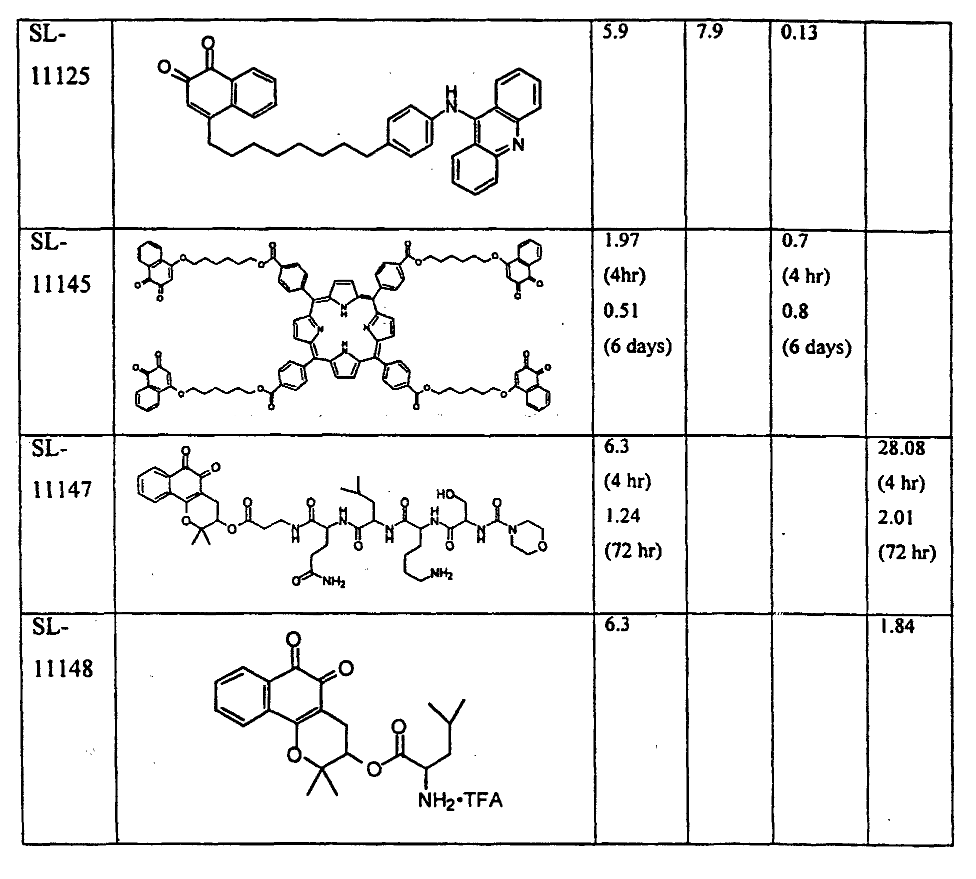

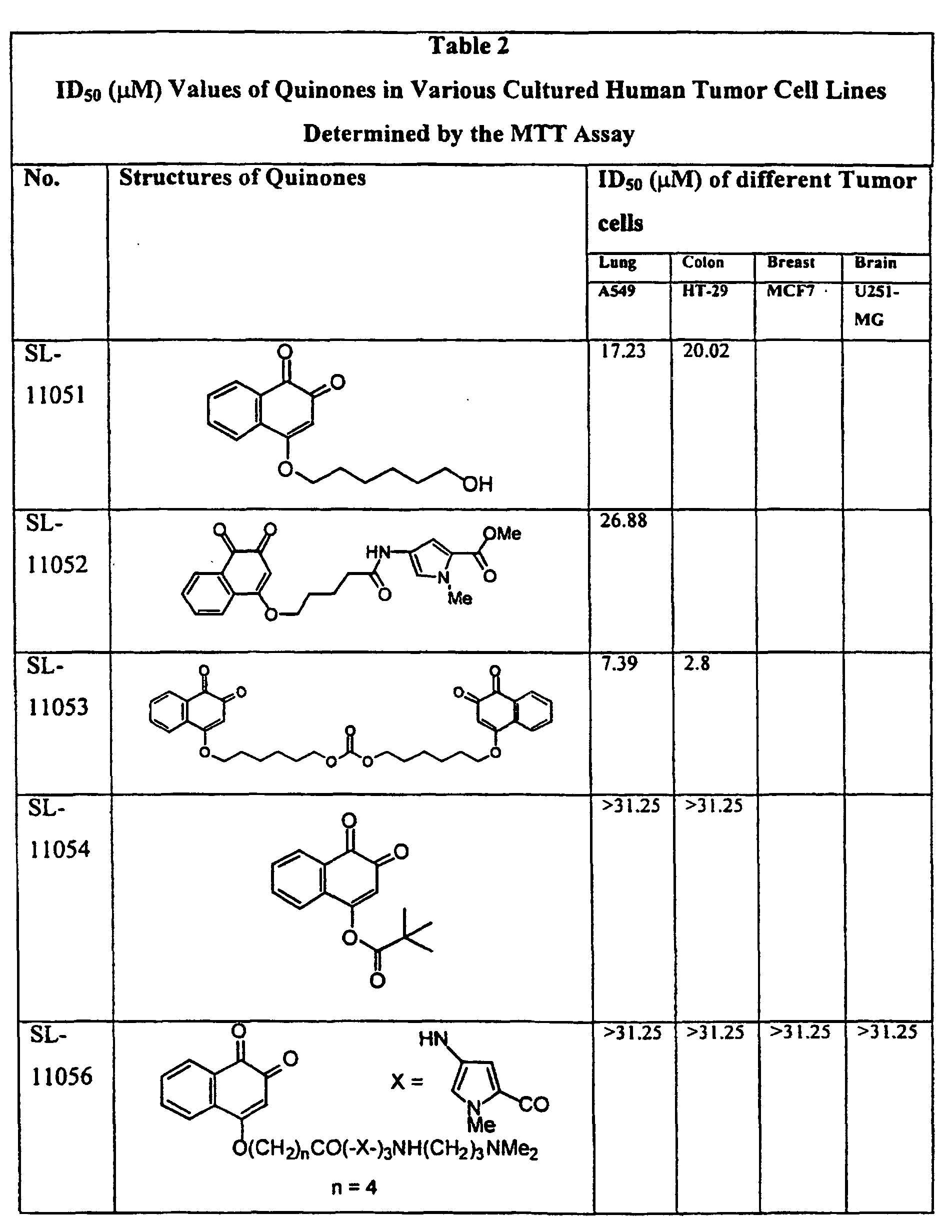

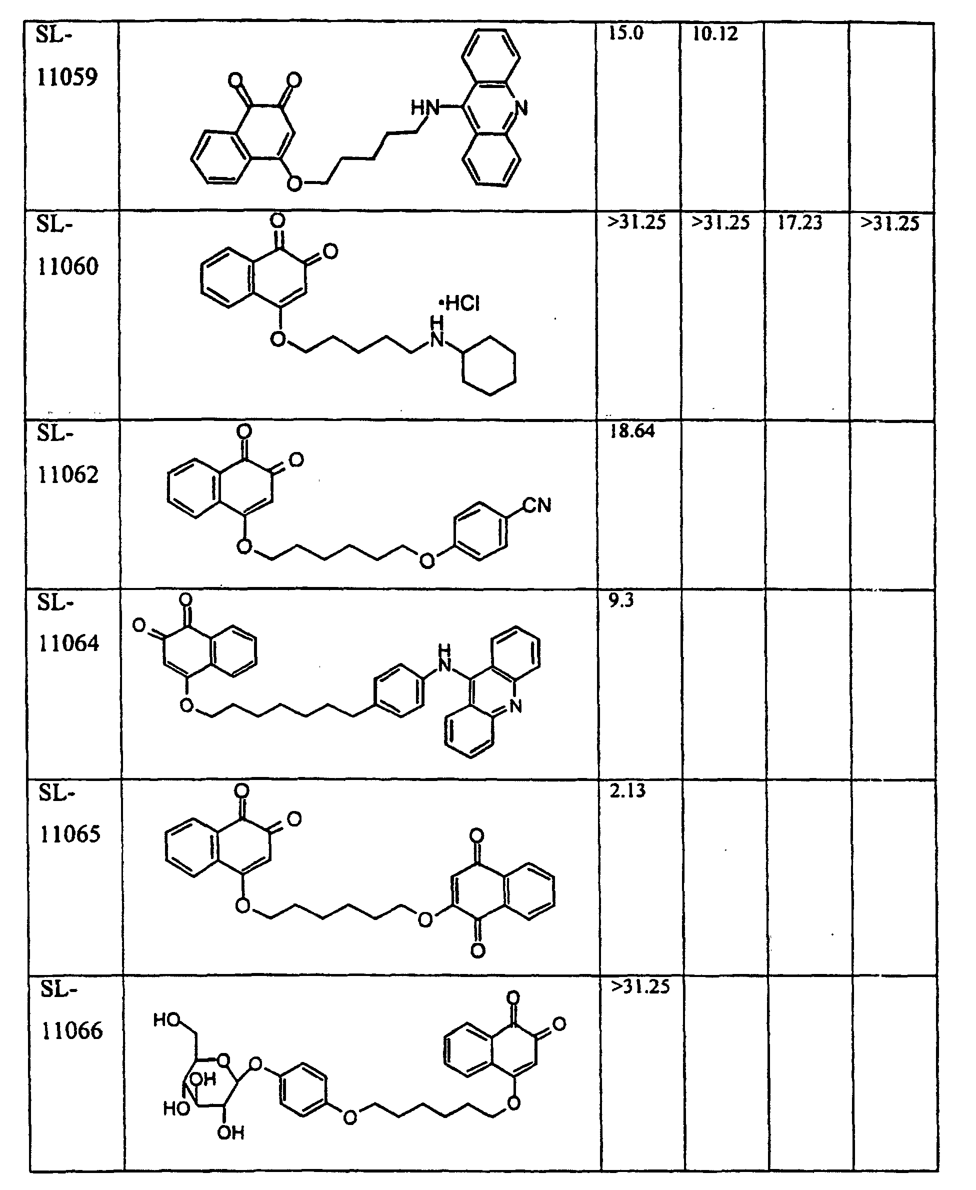

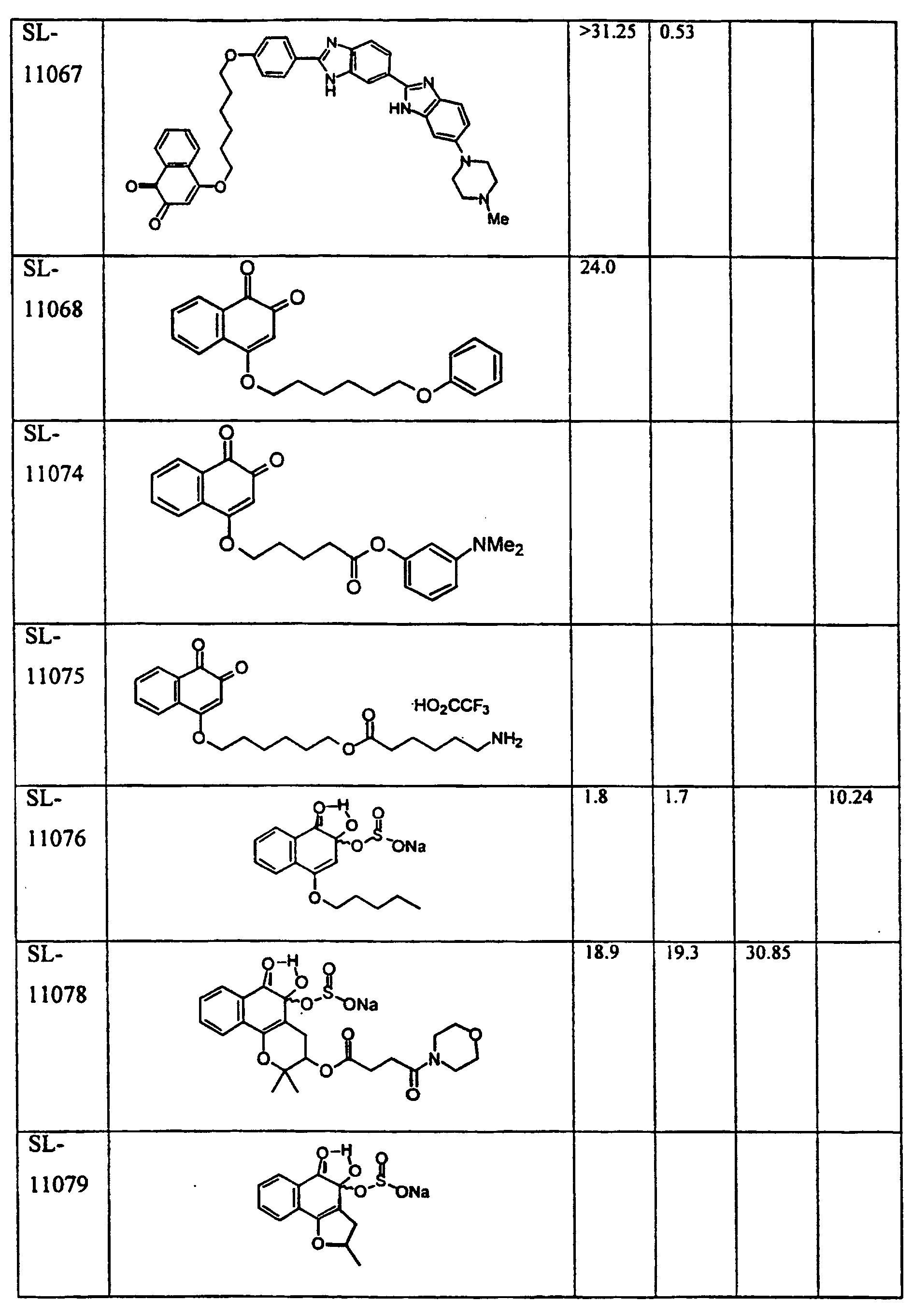

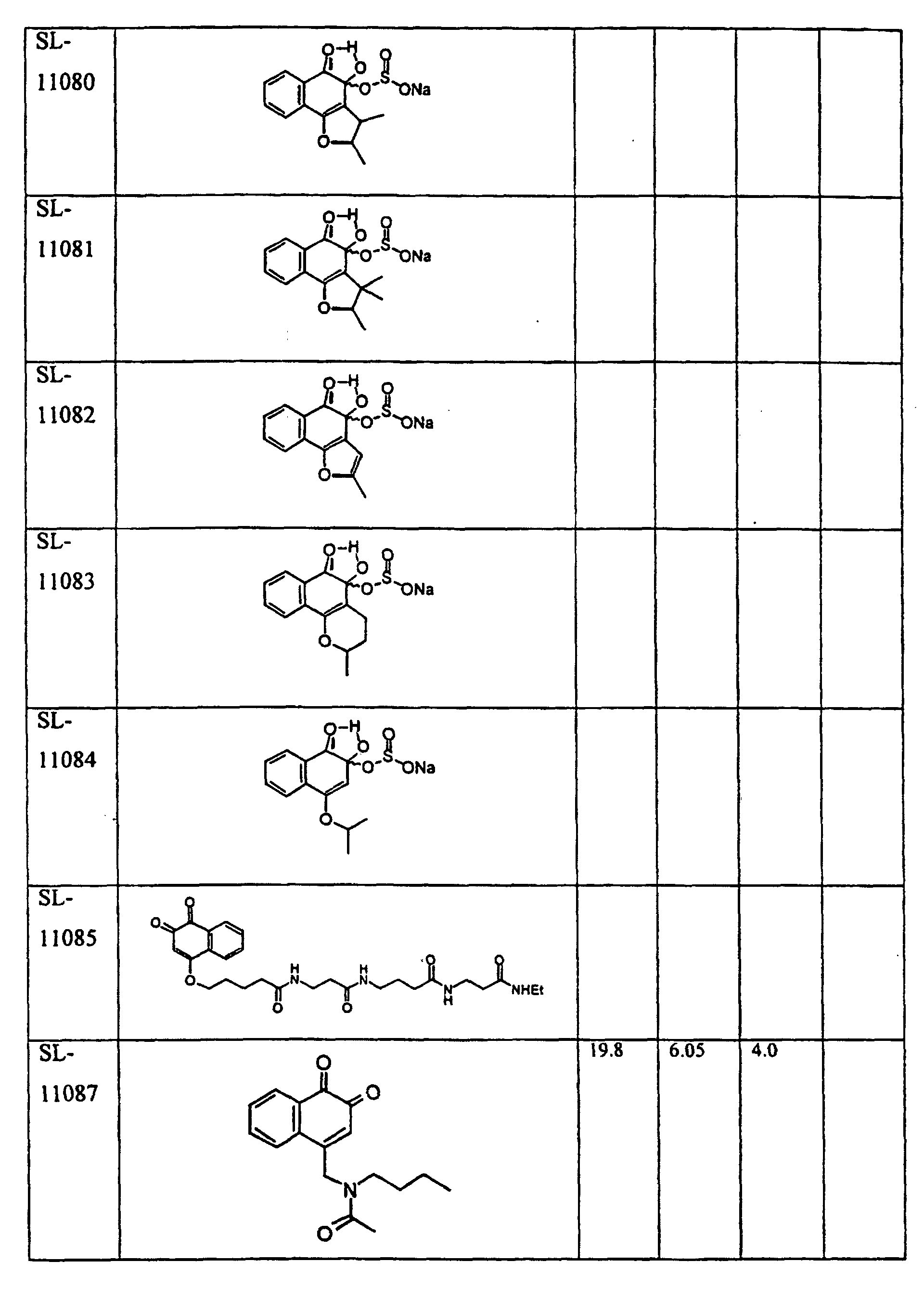

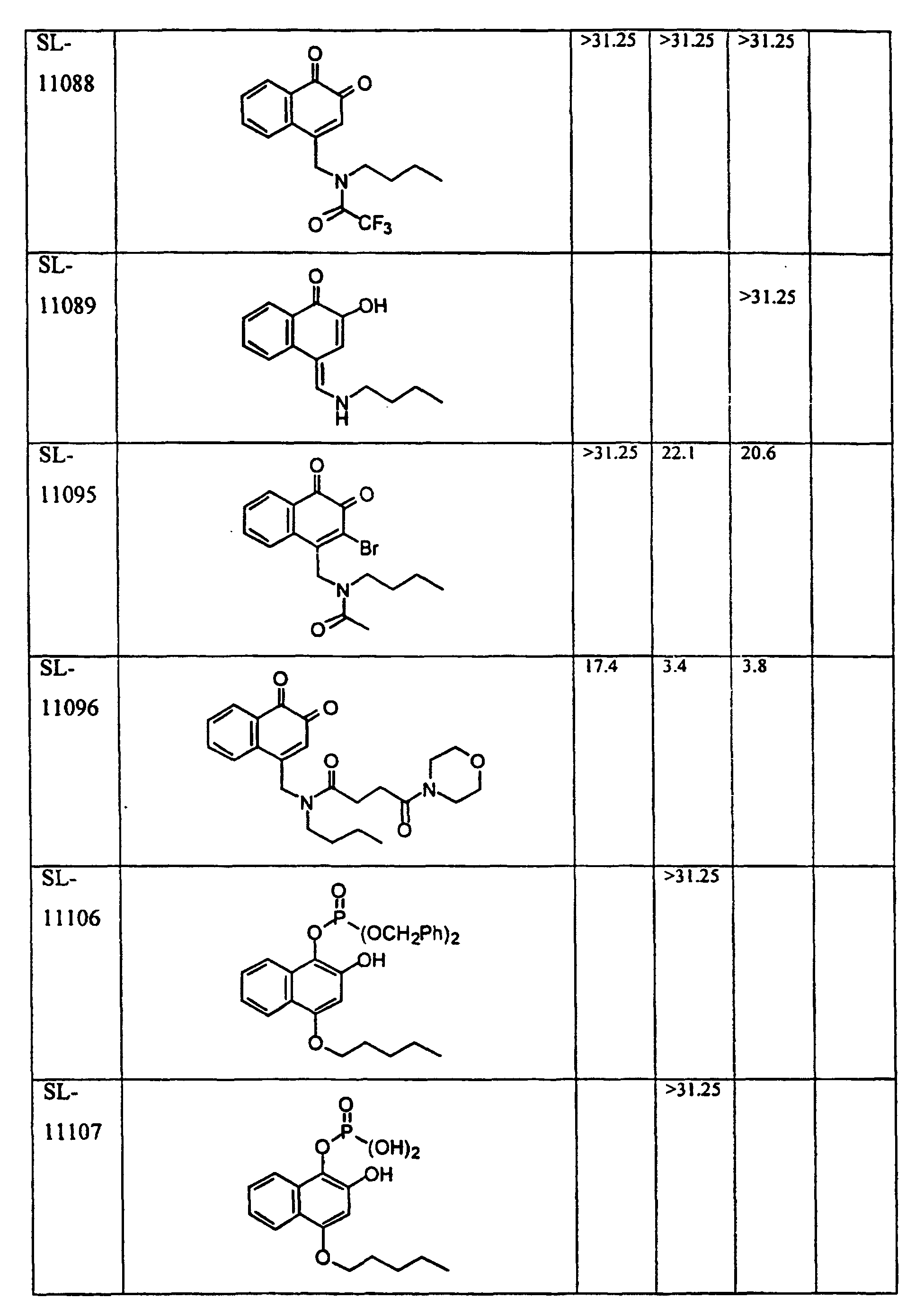

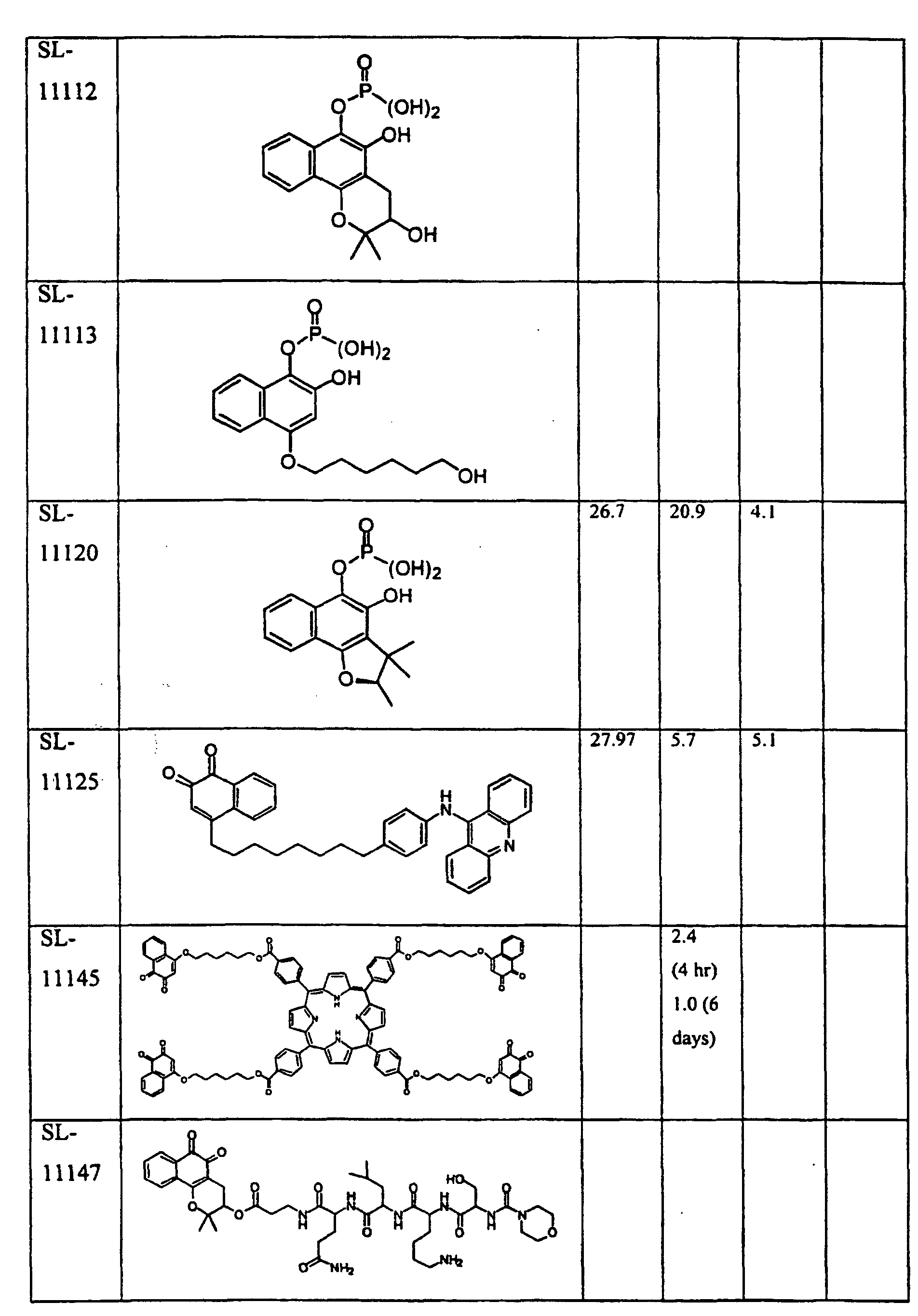

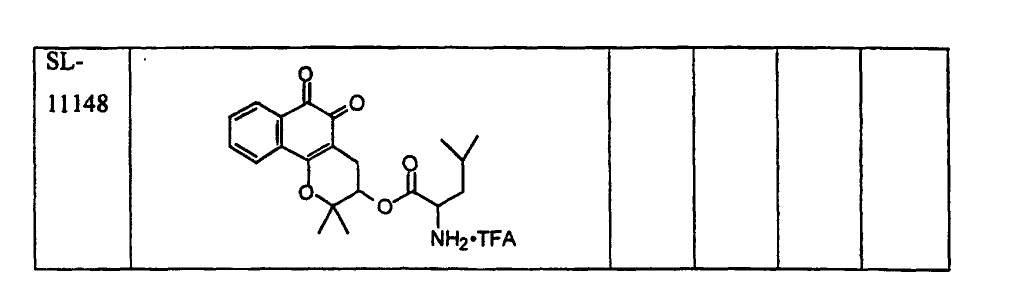

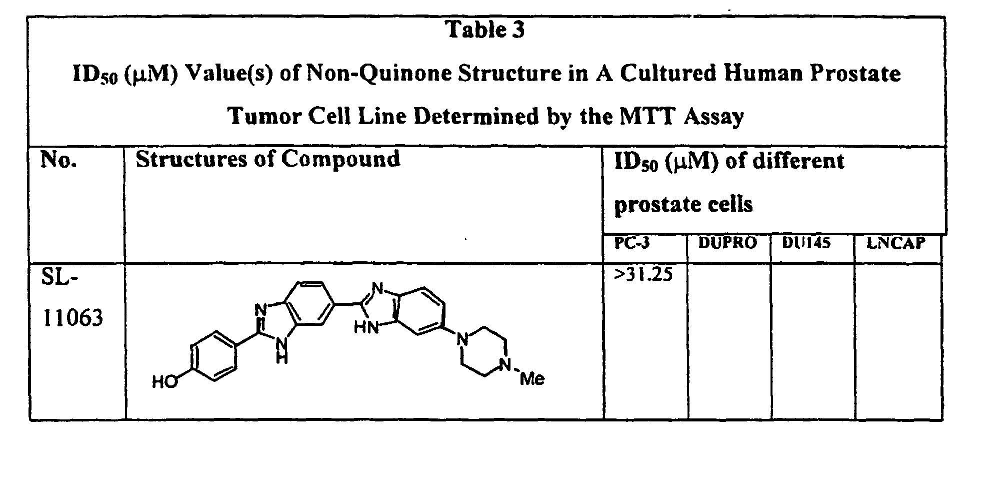

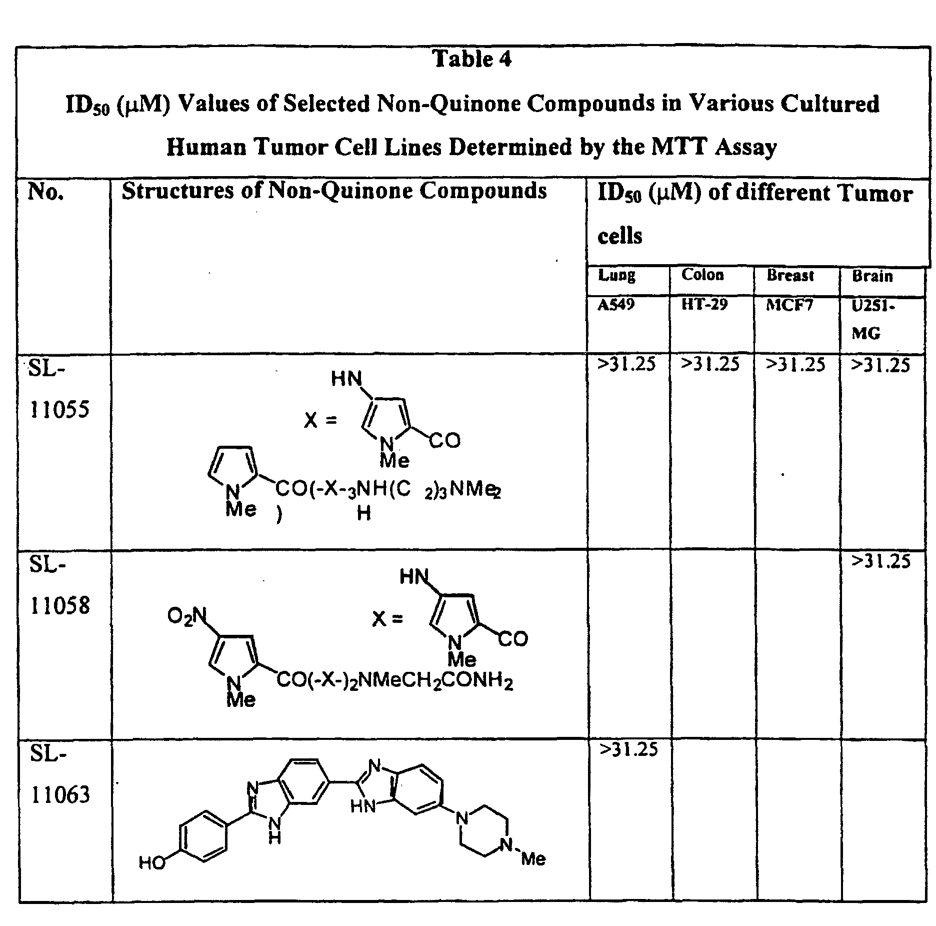

Table 5 lists additional quinones and quinone derivatives which are useful in the invention, either as therapeutics or, in the case of quinones which are not already covalently linked to or derivatized with peptides, as therapeutics in conjunction with peptides.

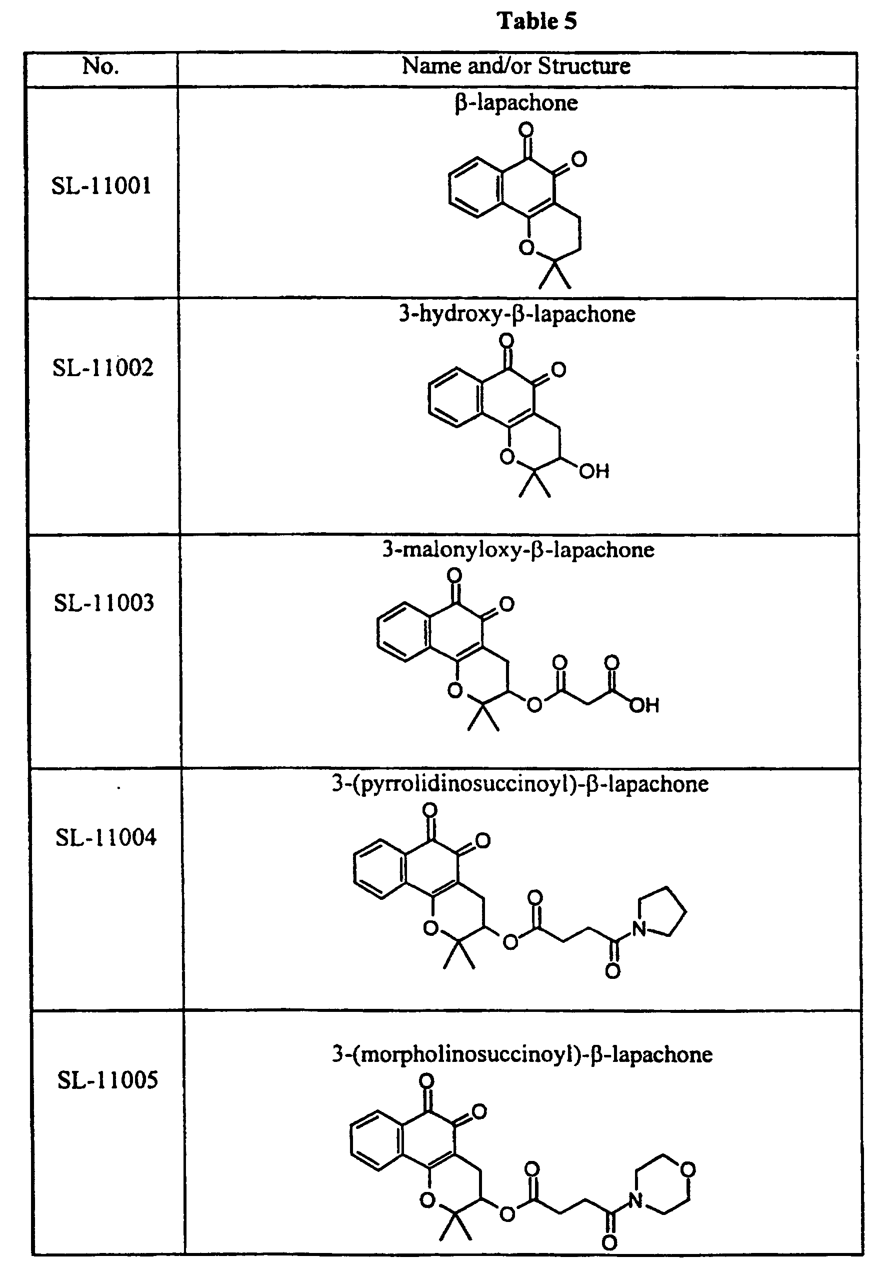

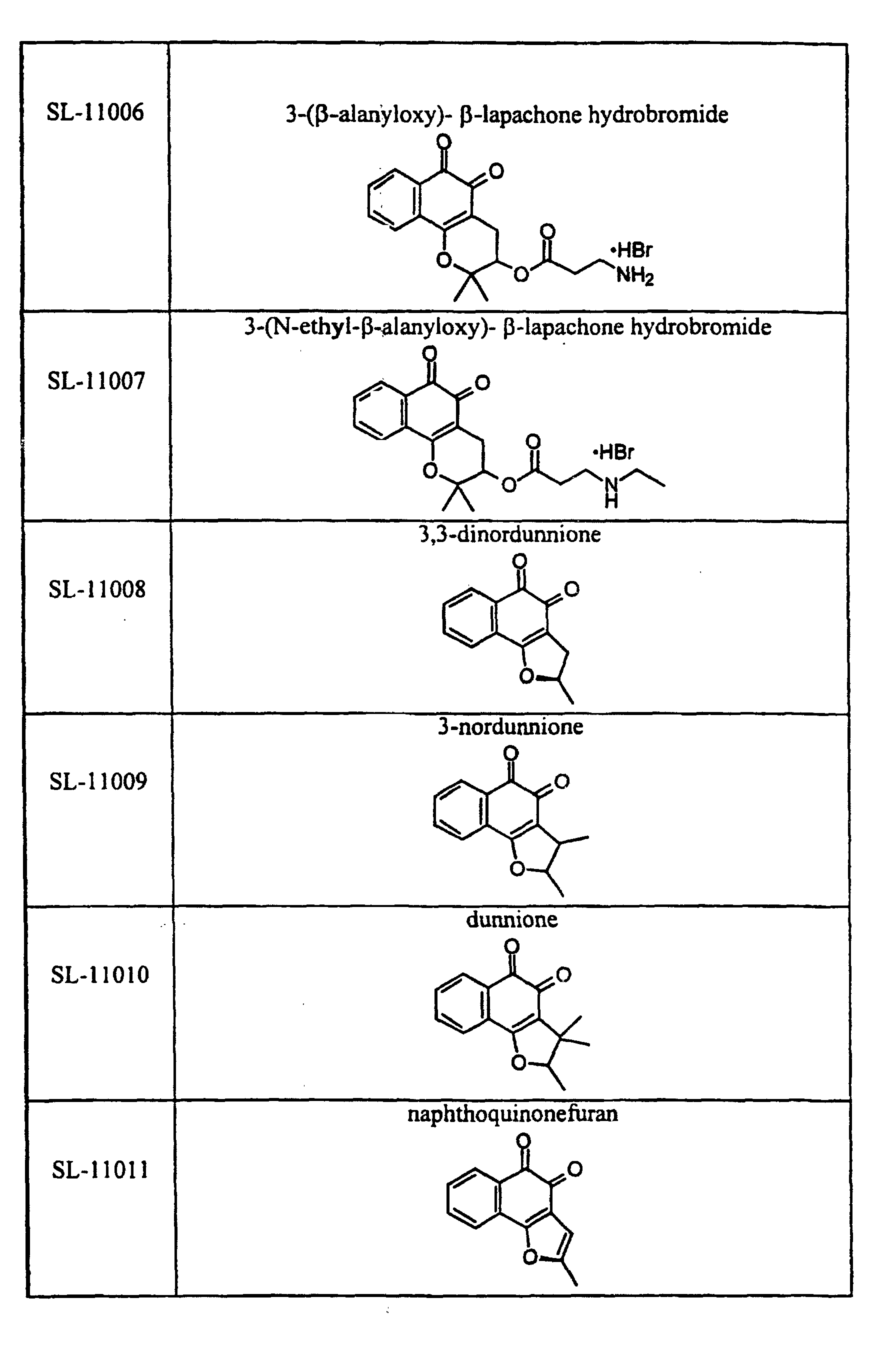

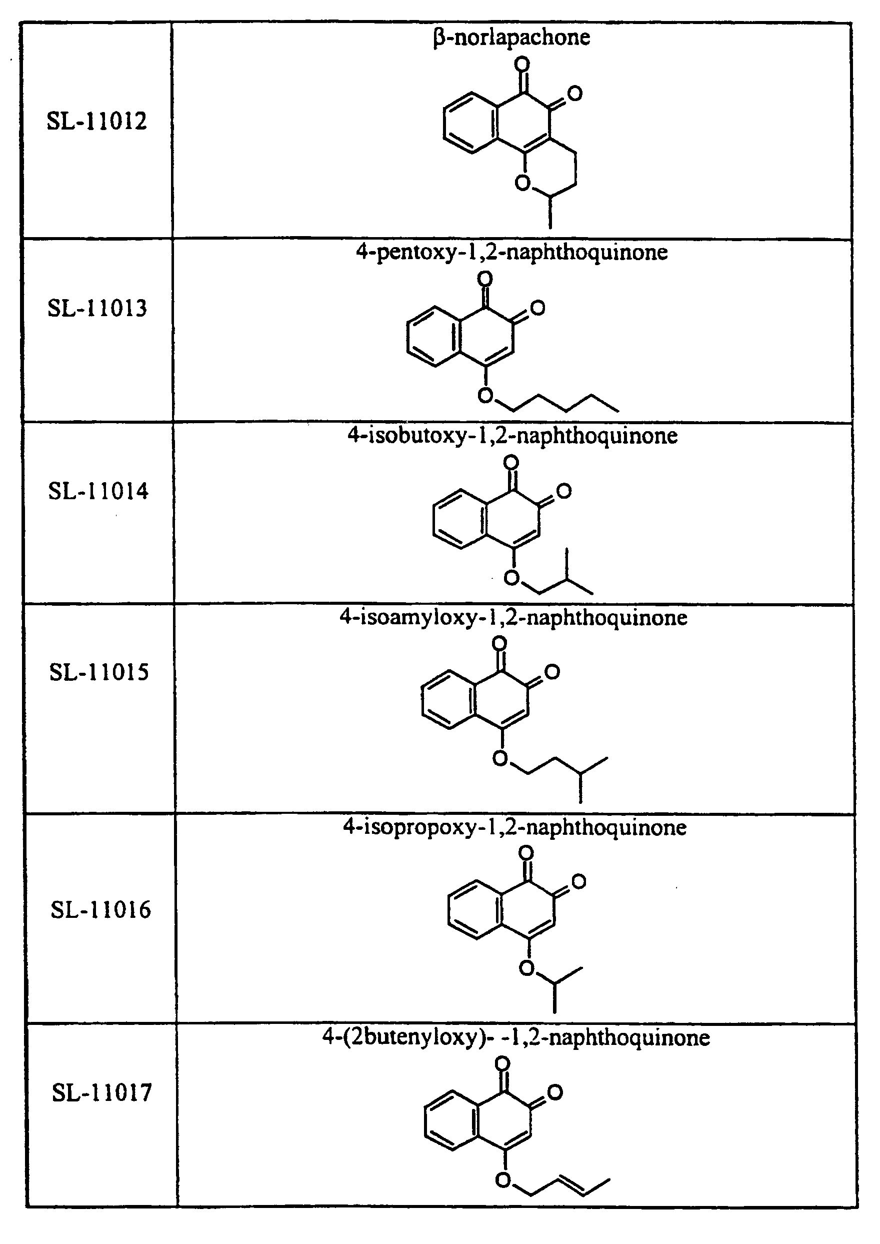

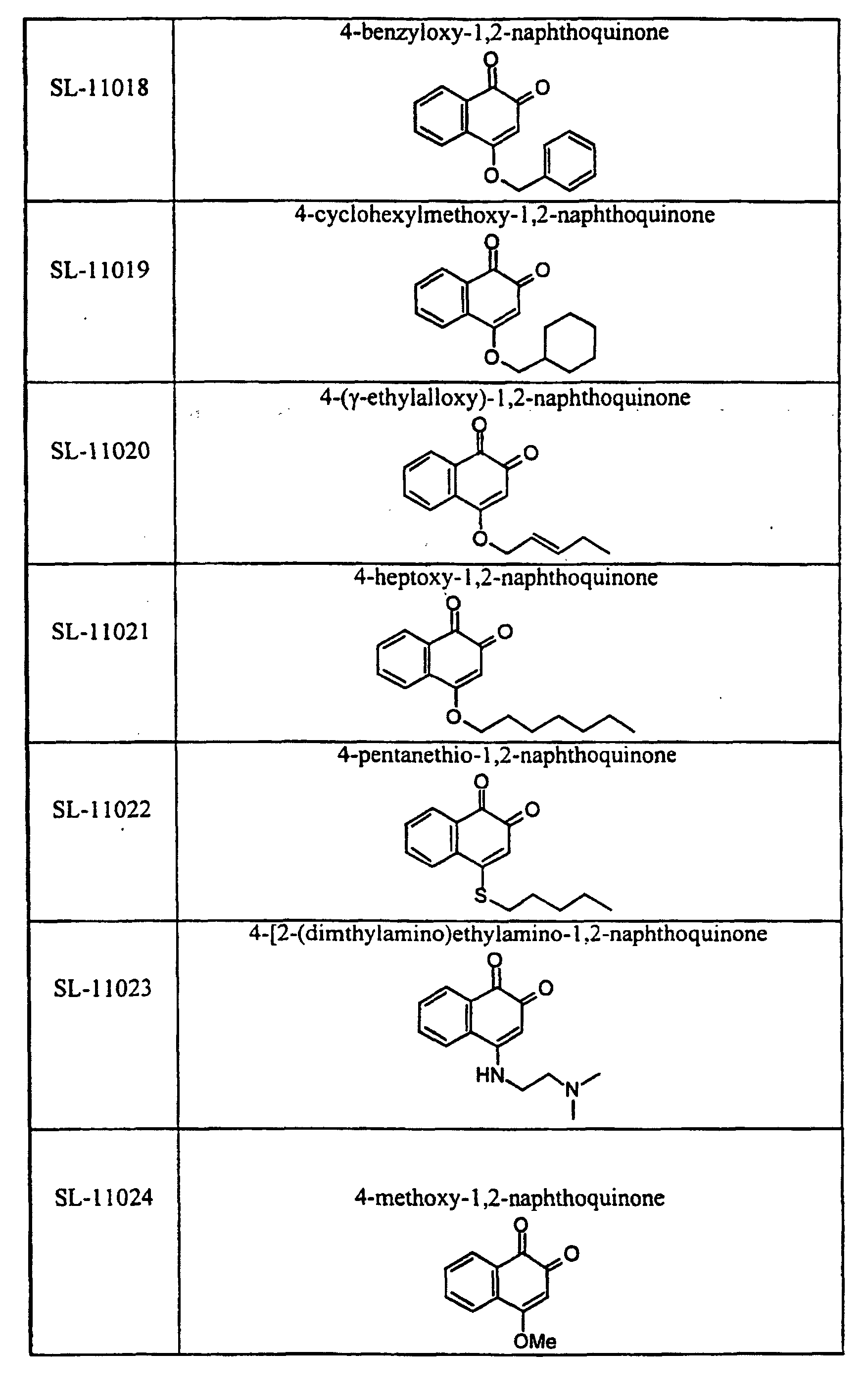

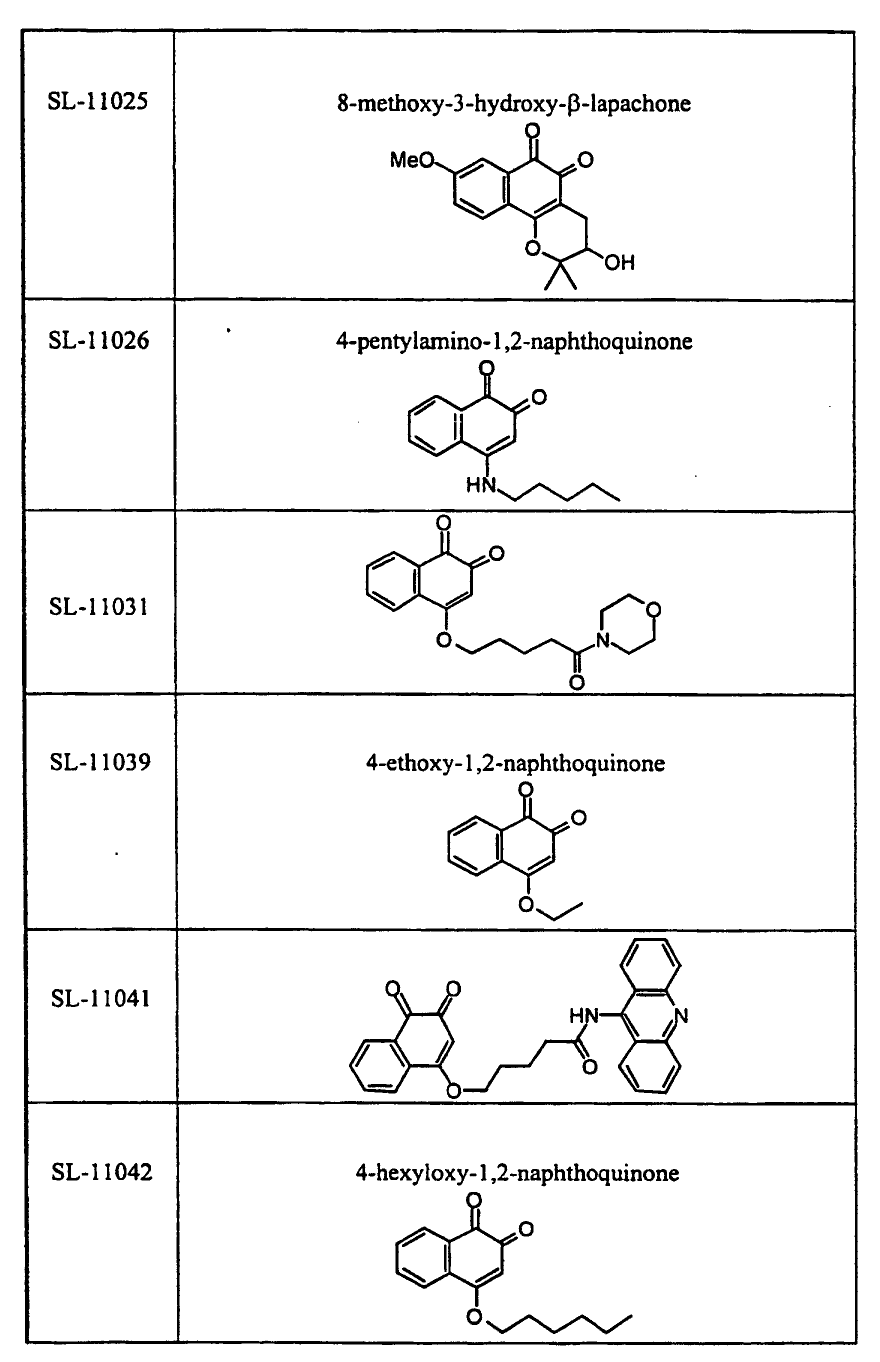

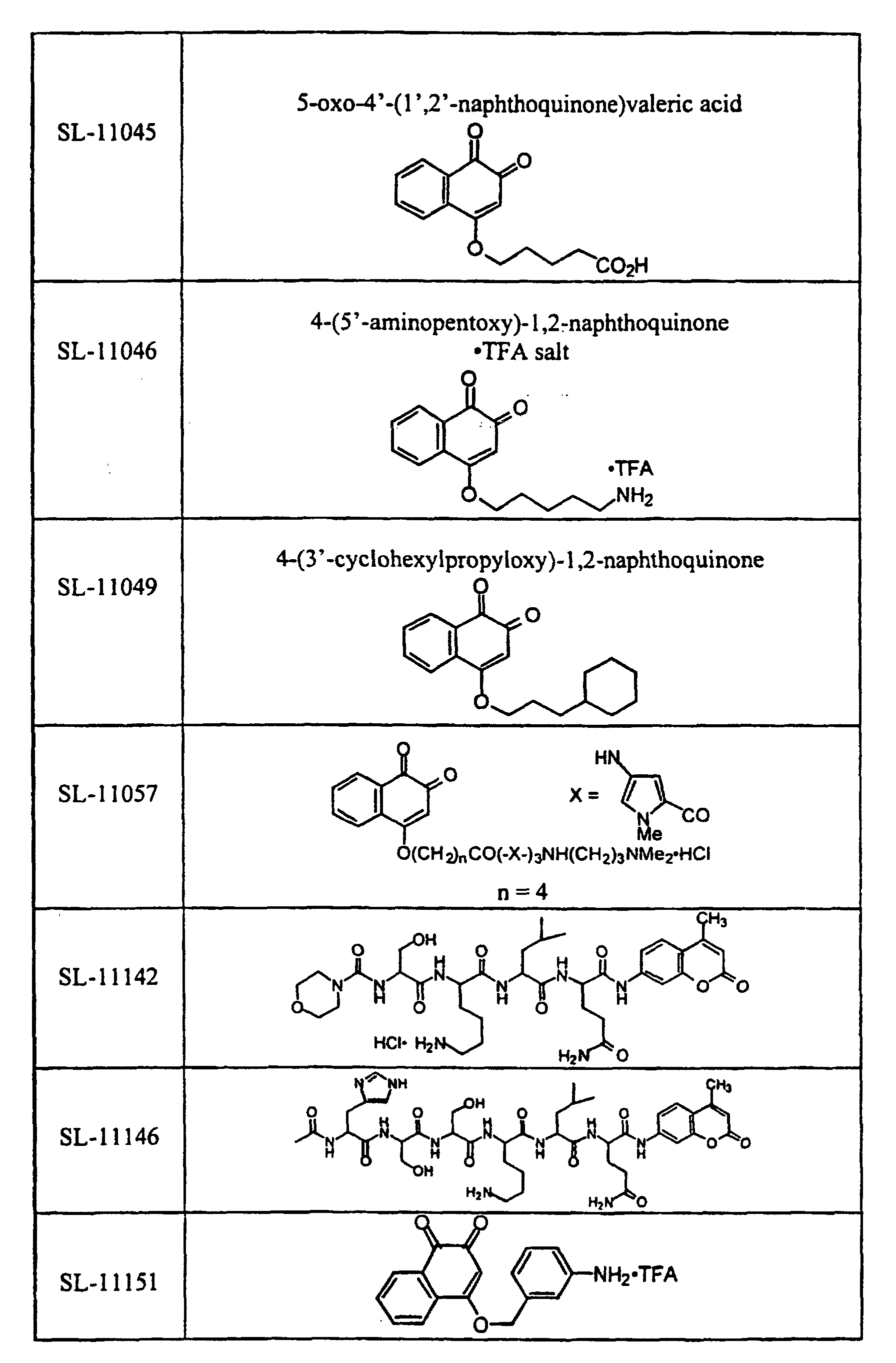

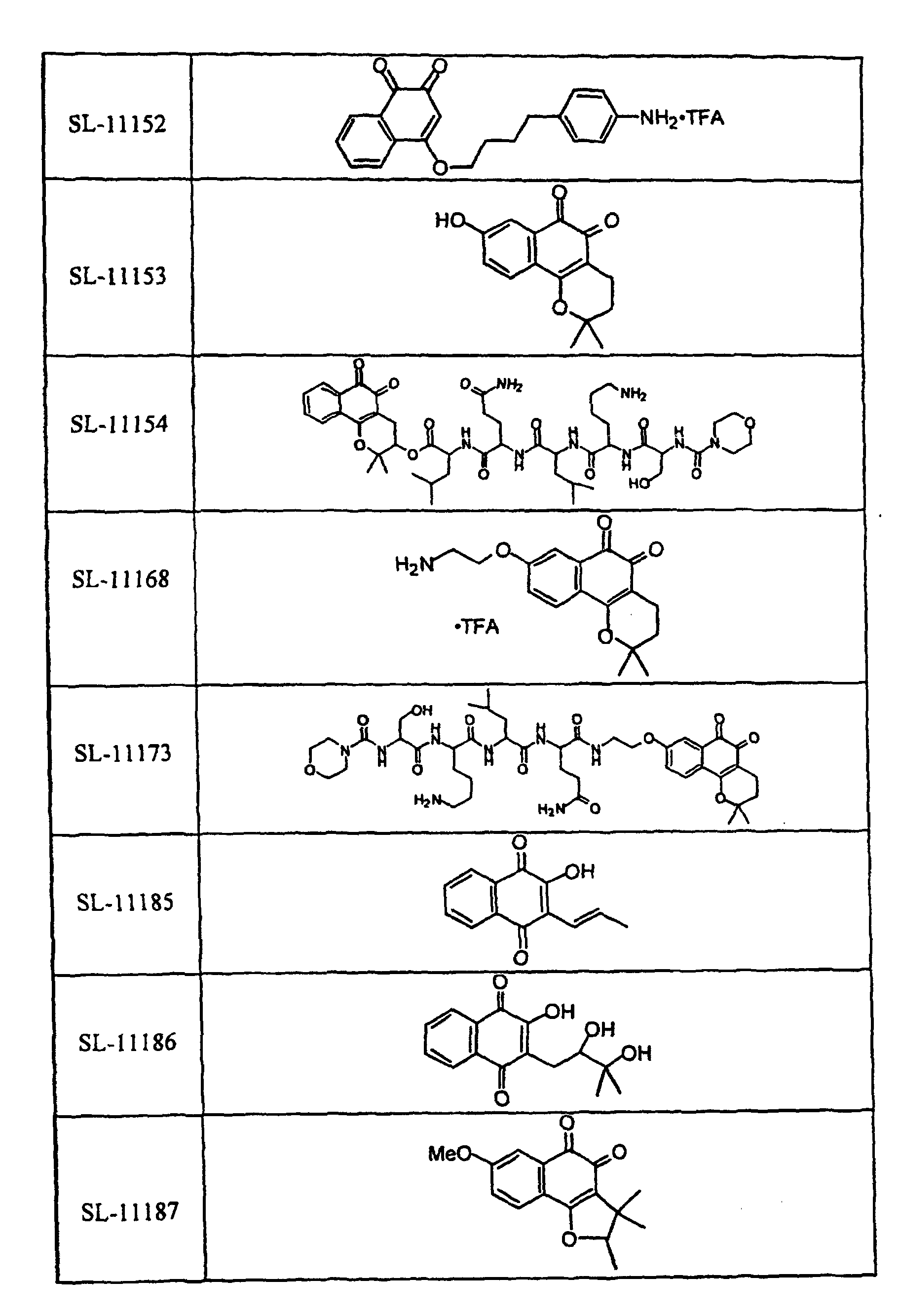

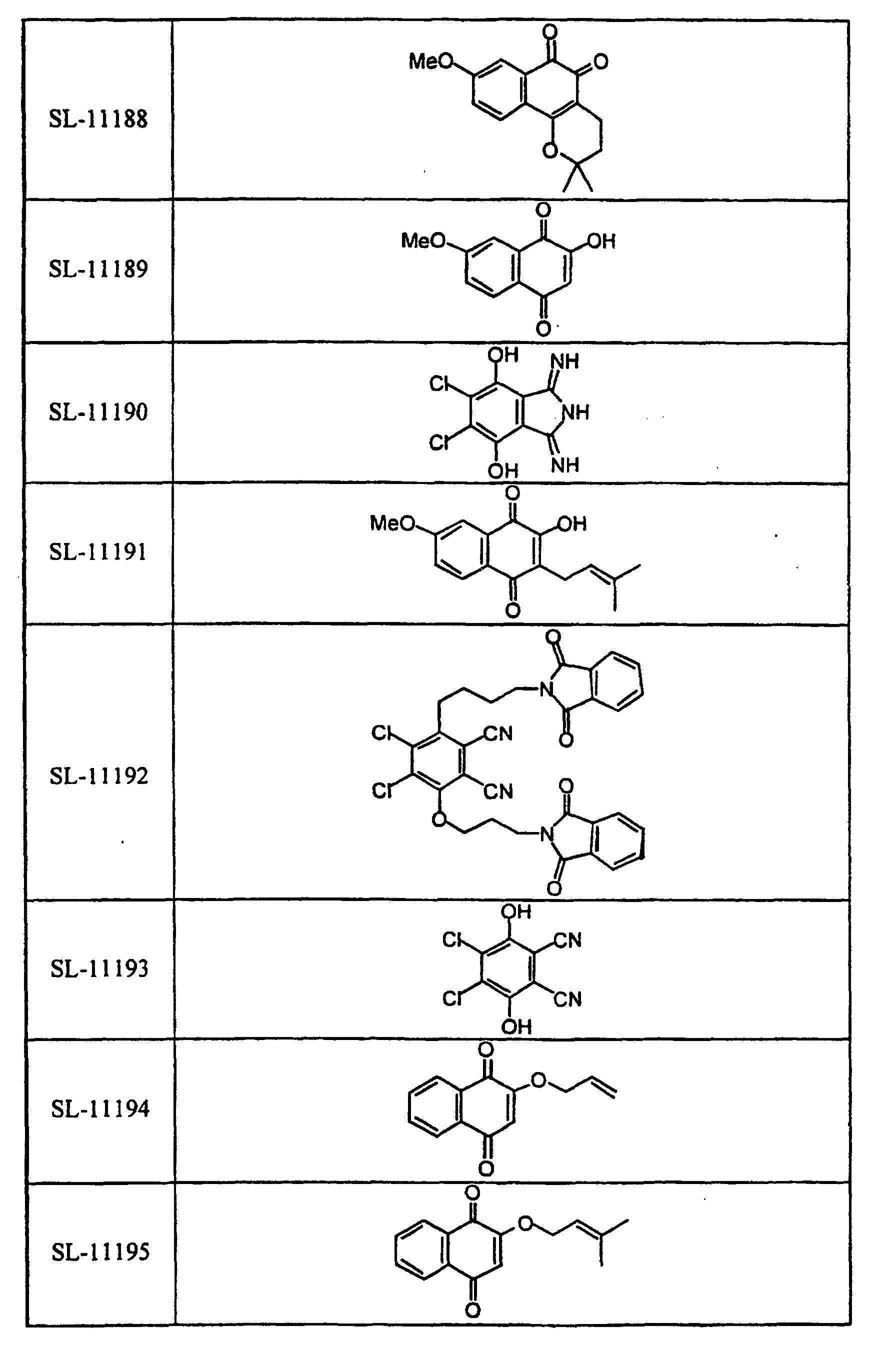

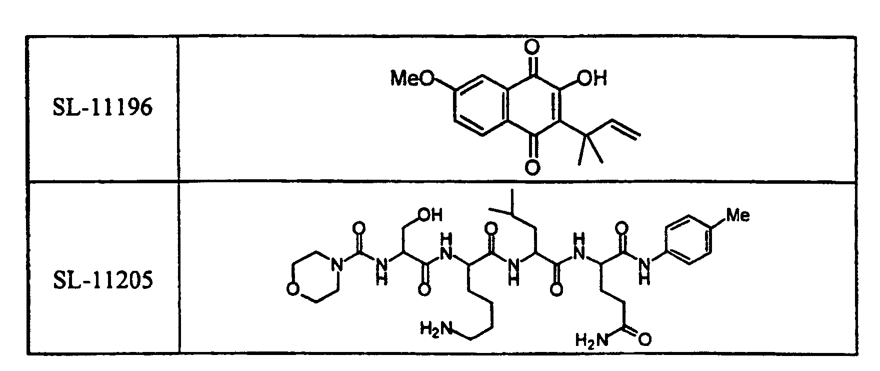

## Claims

1. A compound of the formula 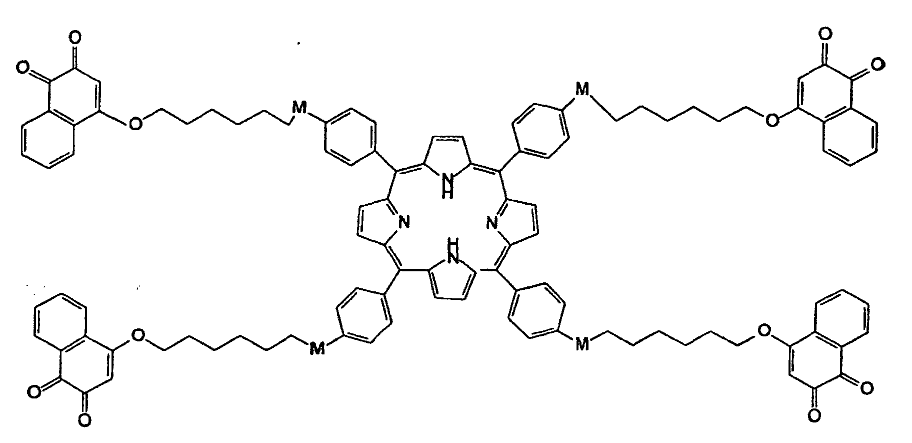 wherein M is selected from the group consisting of -O-, -C(=O)-O-, -O-(C=O)-, -C(=O)-N-, and -N-(C=O)-.

2. A pharmaceutical composition comprising a compound according to claim 1 and a pharmaceutically acceptable carrier.

3. A pharmaceutical composition according to claim 2 for the treatment of cancer.

4. A pharmaceutical composition according to claim 3 wherein the cancer affects cells of the bladder, blood, brain, breast, colon, digestive tract, lung, ovaries, pancreas, prostate gland or skin.

5. A pharmaceutical composition according to claim 2 for the treatment of Alzheimer's disease, epilepsy, multiple sclerosis, problems associated with tissue grafts and organ transplants, psoriasis, restenosis, stomach ulcers or tissue overgrowth after surgery.

6. A pharmaceutical composition according to claim 2 for the treatment of infection or infestation of parasites, bacteria, fungi or insects.

## Patentansprüche

1. Verbindung der Formel 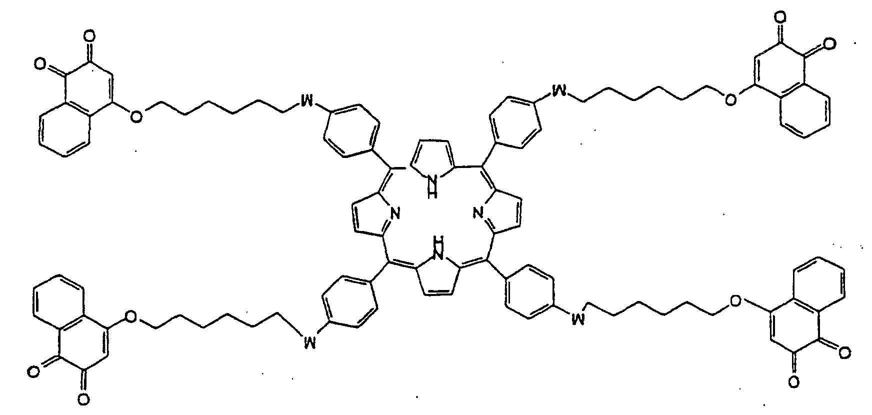 worin M ausgewählt ist aus der Gruppe, bestehend aus -O-, -C(=O)-O, -O-(C=O)-, -C(=O)-N und -N-(C=O)-.

2. Pharmazeutische Zusammensetzung, umfassend eine Verbindung nach Anspruch 1 und einen pharmazeutisch geeigneten Träger.

3. Pharmazeutische Zusammensetzung nach Anspruch 2 für die Behandlung von Krebs.

4. Pharmazeutische Zusammensetzung nach Anspruch 3, wobei der Krebs Zellen der Blase, Blut, Hirn, Brust, Dickdarm, Verdauungstrakt, Lunge, Eierstöcke, Bauchspeicheldrüse, Vorsteherdrüse oder Haut betrifft.

5. Pharmazeutische Zusammensetzung nach Anspruch 2 für die Behandlung von Alzheimer-Krankheit, Epilepsie, multiper Sklerose, mit Gewebe- und Organtransplantaten verbundenen Probleme, Schuppenflechte, Restenose, Magengeschwüren oder Gewebewucherung nach chirurgischen Eingriffen.

6. Pharmazeutische Zusammensetzung nach Anspruch 2 für die Behandlung von Infektion oder Befall durch Parasiten, Bakterien, Pilze oder Insekten.

## Revendications

1. Composé de formule 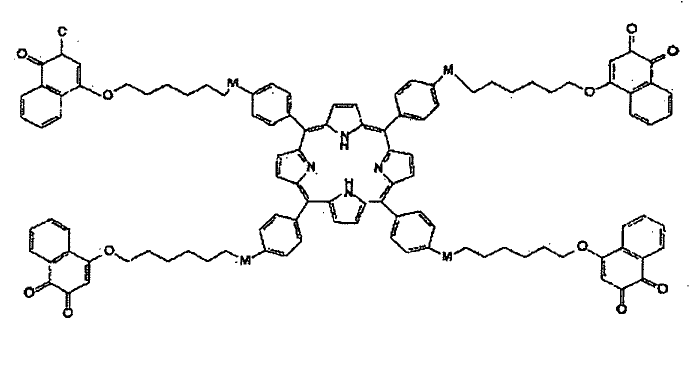 dans laquelle M est choisi dans le groupe constitué de -O-, -C(=O)-O-, -O-(C=O)-, -C(=O)-N-, et -N-(C=O)-.

2. Composition pharmaceutique comprenant un composé selon la revendication 1 et un véhicule pharmaceutiquement acceptable.

3. Composition pharmaceutique selon la revendication 2 pour le traitement d'un cancer.

4. Composition pharmaceutique selon la revendication 3 dans laquelle le cancer affecte les cellules de la vessie, du sang, du cerveau, du sein, du côlon, du tractus digestif, du poumon, des ovaires, du pancréas, de la prostate ou de la peau.

5. Composition pharmaceutique selon la revendication 2 pour le traitement de la maladie d'Alzheimer, l'épilepsie, la sclérose en plaques, des problèmes associés aux greffes de tissu et transplantations d'organe, du psoriasis, de la resténose, d'ulcères de l'estomac ou d'excroissance tissulaire après une opération chirurgicale.

6. Composition pharmaceutique selon la revendication 2 pour le traitement d'une infection ou d'une infestation de parasites, bactéries, champignons ou insectes.
